Europäisches Patentamt

European Patent Office

Office européen des brevets

(19)

(11) **EP 1 334 031 B1**

(12) **EUROPEAN PATENT SPECIFICATION**

(45) Date of publication and mention
of the grant of the patent:
**29.12.2004 Bulletin 2004/53**

(51) Int Cl.$^7$: **B65B 55/08**, B65B 55/16

(21) Application number: **01980884.9**

(22) Date of filing: **26.10.2001**

(86) International application number:
**PCT/IL2001/000995**

(87) International publication number:
**WO 2002/038447 (16.05.2002 Gazette 2002/20)**

(54) **DISINFECTION THROUGH PACKAGING**

DESINFEKTION DURCH VERPACKUNG

DESINFECTION AU TRAVERS D'UN EMBALLAGE

(84) Designated Contracting States:
**AT BE CH CY DE DK ES FI FR GB GR IE IT LI LU
MC NL PT SE TR**

(30) Priority: **26.10.2000 IL 13928700**

(43) Date of publication of application:
**13.08.2003 Bulletin 2003/33**

(73) Proprietor: **Atlantium Lasers Limited
Nicosia (CY)**

(72) Inventors:
• **TRIBELSKY, Zamir
90805 Mevaseret Tzion (IL)**
• **ENDE, Michael
90855 Moshav Shoeva (IL)**

(74) Representative: **Söllner, Udo, Dipl.-Ing. et al
Reinhardt Söllner Ganahl,
Hausen 5b
85551 Kirchheim (DE)**

(56) References cited:
**EP-A- 1 033 138       WO-A-02/12127
US-A- 3 817 703       US-A- 5 069 017
US-A- 5 744 094       US-A- 5 925 885
US-A- 6 013 918**

• **PATENT ABSTRACTS OF JAPAN vol. 017, no.
510 (M-1480), 14 September 1993 (1993-09-14) -&
JP 05 132043 A (IWASAKI ELECTRIC CO LTD),
28 May 1993 (1993-05-28)**
• **PATENT ABSTRACTS OF JAPAN vol. 013, no.
143 (M-811), 7 April 1989 (1989-04-07) -& JP 63
307027 A (HIROSHI UENO), 14 December 1988
(1988-12-14)**
• **PATENT ABSTRACTS OF JAPAN vol. 016, no.
248 (M-1261), 5 June 1992 (1992-06-05) -& JP 04
057726 A (TOPPAN PRINTING CO LTD), 25
February 1992 (1992-02-25)**

**Description**

FIELD OF THE INVENTION

The present invention relates to a method and a system for disinfection and sterilization through packaging (DTP) containing solids, liquids, gases or a combination thereof without damaging said packaging, or their content using high peak power pulsed radiation unit having a high intensity source of light according to claims 1 and 11. The present invention is especially beneficial for disinfecting and sterilizing water-based packaged products, and for granting biosecurity to modern agro-food production lines, biomedical preparation processes such as used for the production of vaccination , and for the production through packaging of opto-pro-biotic or OPB compounds for the enhancement of the human immune system (acquired immune system, or MHC type I, II, III). The methodology of the present invention is especially beneficial for agro-food production, for biomedical fields, and for bottled water or flavored water and beverages. More specifically, the present invention facilitate the disinfection or photo-treatment of liquids and gases after they have been filled into the packaging, and the packaging is closed already, or the packaging will be closed prior or after treatment. The evolutionary step of the method according to the present invention facilitate final polishing, or processing (by light) for increasing the safety, bio-security, and chemical purity of bottled water or packaged liquids or gases or combination. The methodology of the present invention eliminates the need to transfer the liquid or gas, or solids, or combination through a plurality of stages, or through cumbersome distribution systems requiring substantial attention to bio-compatibility, and biological, and chemical cleanliness and purity of origin of components therein (in the packaging). Further more, the methodology of the present invention facilitates the provision of a single step for purification, disinfection, sterilization, oxygenation, dissociation, or reduction, elimination, or equalization of unwanted species of biological, or chemical origin without the need to open the package and treat the content separately. More specifically, the present invention discloses a novel methodology for photo-treatment through packaging without causing molecular migration or damaging to said packaging or their content therein. The competitive advantages of the methodology of the present invention, as well as instruments, or devices using the method, is the capability to ensure purity, freshness, integrity, and naturally of single and multi-components compounds, liquids, gases or solids or any combination thereof by transferring light through packaging for photo-treatment through packaging enhancing the quality levels through out the life of processed products by the method of the present invention for disinfection and sterilization, oxidation, and reduction, dissociation and photo-treatment through packaging DTP (disinfection Through Packaging). Especially beneficial for bottling industries and for the biomedical filed.

BACKGROUND OF THE INVENTION

In industry, especially in food production, sterilization of the end product is a must.

Conventional chemical disinfectants inflict strict limitations on producers and end users as there is always the need to remove the harmful chemical from the water, or agro-food product being packaged. Furthermore, chemical treatment methodologies are being phased out by regulatory legislation limiting the concentration of disinfectant in products DBPs. Public awareness has already focused on threatening volumes of toxic compounds created using chemical disinfectants, and the residual toxic by-products created when chemical residues gets mixed with widely available materials in the environment. Use of electromagnetic radiation and radiation chemistry techniques for sterilization, is documented. These methods use ionizing radiation which can be absorbed by the packaging materials (for example PET does not transmit below 312nm), altering the composition of the packaging and causing molecular migration which induces sensory effects. Furthermore, Gamma rays, X-Rays, Y-Rays and electron beam technologies have failed to provide safe working environments, often producing unstable molecules and compromising the safety of the operators. Furthermore, machines producing ionizing radiation have being limited by regulatory affairs and are cumbersome, requiring high periodical maintenance and replacements. While ionizing radiation is able to treat some forms of packaging (such as glass bottles, for instance,) their use is being limited as spatial control and manipulation of these lower wavelengths is considerably more difficult then stirring UVA pulsed laser beams).

Temperature and heating treatment currently in use through aseptic filling machines and other agro-food production lines is prohibitively expensive, requiring high periodical maintenance and replacements. Currently used heat treatments are performed on the liquid (water for example) before it is packaged, in order to prevent damage to temperature-sensitive polymers used in packaging (PET for example). Increasing the temperature of water-based products to be packaged can result in changes to its taste, flavoring and the integrity of the components can be compromised. The wholesomeness, freshness, the flavor and quality parameters of products treated by high temperature deteriorates rapidly. This decreases the quality while increasing the overhead. No known thermal treatment can offer a solution to water based products after packaging in PET packaging materials, as the PET molecules migrate into the packaged products (as results of the heat generated) and may cause dangerous sensory effects which threaten the health of the consumers. Sterilization using heat substantially increases the production time and expenditure, often leading to machinery and devices which are cumbersome, large in dimensions, and require specialized engineering support and operation.

The principles of disinfection and sterilization techniques which utilize photo-treatment and oxidation processes that involve ultraviolet light, are well known and documented. Use of an effective Wavelength Range (EWR) of 220nm to about 360nm for sterilization, using a variety of conventional continuous wave (CW) type light sources has being documented. Should pathogens be present within a product, these wavelengths are considered to inactivate their DNA and RNA, thus preventing the replication of microbes. This ultraviolet (UV) technology is preferred compared to other disinfection technologies, due to its non-residual, non-chemical nature. However such UV technology only provide a partial solution to the familiar problem of hygiene and biosecurity: this conventional UV technology can only provide disinfection capabilities when a predetermined volume of liquid and gas has to be disinfected, or when the surface area is of particular parameters, having a specific surface curvature. Conventional ultraviolet technologies have failed to provide the ability to disinfect through packaging without inflicting damage or molecular migration within the product or within the packaging, which may cause sensory effects and health threats for the consumers and the producers. An effective disinfection technique which can penetrate through packaging has not being found till now. This is due to the strict limitations imposed by the polychromatic nature of current conventional UV mercury based lamp light sources, the most common being Continuous Wave (CW) / Pulsed Wave (PW) low pressure, medium pressure, or LPHO types of mercury based lamps, which are used, for example, for the disinfection of potable and drinking waters.

Currently used sterilization and disinfection methodologies are hindered by strict limitations such as polychromatic emission, continuos wave operation, non directive optical distribution of the light, and radial, and often wasteful, optical performance of currently used light sources. Further more, low geometrical utilization and lack of appropriate beam management systems have reduced coupling efficiencies, causing alarming head-loss. These limitations reduce the efficiency and increase the costs associated with these conventional sterilization techniques (i.e. mercury based lamps) thus further reducing their use and marketability.

U.S. Patent number 5,925,885 (hereinafter referred to also as D1) relates to the use of polychromatic UV light generated by flash lamps in the wavelength spectrum of between 180nm and 300nm, for sterilization of packages and their content. According the D1 invention (see D1 column 7 lines 27-28 ) one or more reflectors are required inside a sterilization chamber for achieving appropriate light diffusion throughout the product.

As mentioned above, D1 suggests and claims the use of UV light in the wavelength spectrum of between 180nm and 300nm. Although this is the most preferred wavelength range in terms of deactivation of micro-organisms, this range is very ineffective for penetrating through the packaging of the very commonly used PET bottles. Although these bottles are almost transparent to visible light, they absorb nearly 90% of UV light in the wavelength claimed by D1. This means that nearly 90% of the energy that reaches the package (i.e. after loosing the aforementioned loss of energy due to the use of external optics) will be converted to heat at the package envelope causing damage and molecule migration, while only 10% will reach the content intended to be disinfected. Therefore, D1 invention fails to comply with the disinfecting requirements of bottled water industry and the like. It has to be emphasized that as discussed later in the specification of this patent, the absorption of UV light by the PET polymer dramatically decreases at wavelengths near 355nm, however, this wavelength is out of the range which could be generated by flash lamps. Furthermore, flash lamps can not generate the 355nm in its purity as all flash lamps generate polychromatic light (i.e. light output having a plurality of wavelengths of light including IR, VIS, UV such that molecular migration will occur as heat/IR is generated and absorbed by the polymer packaging)

The 355nm wavelength could only be generated using solid state lasers.

U.S. Patent number 3,817,703 (hereinafter referred to also as D2) suggest (see claim 8) to determine the light wavelength to which the material is transparent, and to subject the material to short bursts of high energy density laser light rays from a plurality of light sources disposed about the material to direct light rays at the material from different angles.

It can be clearly seen all along the text of the D2 invention how many optical arrangements are made in order to assure full exposure of the package and its content to the laser beam, ensuring a sufficient amount of its light energy is distributrd throughout packaging and content.

More specifically and in contradistinction to existing technologies in the field of medical preparation, the ability of the methodology of the present invention to process in the final packaging the content or volume facilitate safer, rapid, and economical aseptic production processes, especially beneficial for the bottling industries, biomedical fields, and for the production of immunifying and optoprobiotic compounds embedded in drinking water, mineral water, flavored water, spring water, treated water, air for breathing, and carbonated, oxygenated or enhanced or supplemented beverages or any combination thereof. Especially beneficial for the agro-food industries, bottling and water industries and for progress in immunological production processes eliminating the need to use expensive and often reactive species and noxious chemicals.

DEFINITIONS

Optronic in the context of the present invention means optical and electronic manipulation of the light sources according to the present invention, and wherein said optic or electronic manipulation are linked or synchronized or operating autonomously for smooth efficient operation of devices using method of the present invention.

Biodosimetric curve means in the context of the present invention a predetermined dose of the rendering harmless of specific microbiological (and chemical) compounds or species which otherwise will be noxious and can threaten health or human, animal or plants.

Chemodosimetric curve means in the context of the present invention a predetermined dose of energy for the rendering harmless of specific chemical (and biological) compounds or species which otherwise will be noxious and can threaten health or human, animal or plants.

Thermal Dynamic curve means in the context of the present invention a threshold which above the molecular structure of the polymer, compounds or resin becomes unstable such that molecular migration may occur (i.e. from packaging to content therein) , or visible or sensory damage may pursue upon consumption.

Recurrent means in he context of the present invention reaching its original settings, or dosimetric value, or sequence start, or beginning of exposure time, or time and energy intervals, or energy intensity, or machine control command protocol, thus performance is repeated back to the original level or starting point.

Non recurrent means in he context of the present invention means that performance of devices using the method of the present invention does not reach its original point, level or time and intensity starting point.

Cyclic means in the context of the present invention reaching its original starting point, beginning, or dose or energy density or intensity or sequence start, or time interval, or machine control command protocol cyclically progressing with spectral distribution over predetermined space over a predetermined period of time, thus performance reaching its starting point.

Continuum means in he context of the present invention a continuos flow of strings, events, performances, or machine command protocol, or operation, intensity or activity which does not stop continuously.

Optronic means for the context of the present invention a process or interface wherein optical signals control electrical signals for the objective purpose of the production of electronic or optical or electro-optical output or combinations at a given range and spatial characteristics. Such characteristics as triggering lasers and supplement light sources in a continues string of electro-optic modulation or parameter changes received on a network via machine control protocols for the sake of adjusting or enhancing the interoperability or interconnectivity for maximizing efficiencies or system performance.

Deflecting, refracting, reflecting, refractive index, and spatial uniformity and characteristics of light means in the context of the present invention optical processing and beam management of light produced by radiation units according to the methodology of the present invention having a high intensity source of light, such optical processing and/or beam management caould be applied on CW or PW, or Quasi CW beams generated by a plurality of light sources operating in synchronicity or according to preplanned operation.

In the context of the present invention, spectral ranges of interest in Photochemistry mean:

| Range Name | Wavelength |
|---|---|
| Near Infrared | 700-10000 |
| Visible | 400 - 700 |
| Ultraviolet | |
| UVA | 315-400 |
| UVB | 280-315 |
| UVC | 100-280 |

Little photochemistry occurs in the Near Infrared. Except for some photosynthetic bacteria. Which are capable of storing solar energy at wavelengths out to 980 nm. The Visible range is completely active for photosynthesis in green plants and algae. Also many dyes can undergo photochemical transformations themselves or sensitize reactions in other molecules. Most studies in photochemistry involve the Ultraviolet range. The UVC range is extremely dangerous since it is absorbed by proteins, RNA and DNA and can lead to cell mutations and/or cell death. The UVC range is sometimes called the germicidal range, since it is very effective in inactivating bacteria and viruses. The Vacuum Ultraviolet range is absorbed by almost all substances (including water and air).

In the context of the present invention Coherent and incoherent light means Light sources used in photochemistry can either be coherent (all emitted photons are in phase with each other as they propagate) or incoherent (all emitted photons have random phases). All lasers emit coherent radiation and usually at one wavelength. The dispersion is very small so that a laser beam remains at or near its original diameter as it propagates; the light emitted by all other light sources is almost always incoherent. Most of these sources are either "hot element" sources (e.g., the incandescent light bulb) or "plasma" sources (e.g., a fluorescent light tube).

The appropriate term for UV disinfection is "UV fluence rate" because a Microorganism can receive UV power from any direction, especially when there is more than one UV lamp in the vicinity. In general usage, the irradiance or fluence rate may be expressed as MW cm^(-2). The irradiance is often incorrectly termed "light intensity" see the proper definition of "radiant intensity" above.

Light dose or fluence means: The light dose or fluence (symbol H. units J m~2) is the total radiant energy of all wavelengths passing from all directions through an infinitesimally small sphere of cross-sectional area dA, divided by dA It is given by the average fluence rate times the exposure tune in seconds. The term UV dose is often used in UV disinfection literature. It represents the UV exposure of a given organism in the germicidal range.

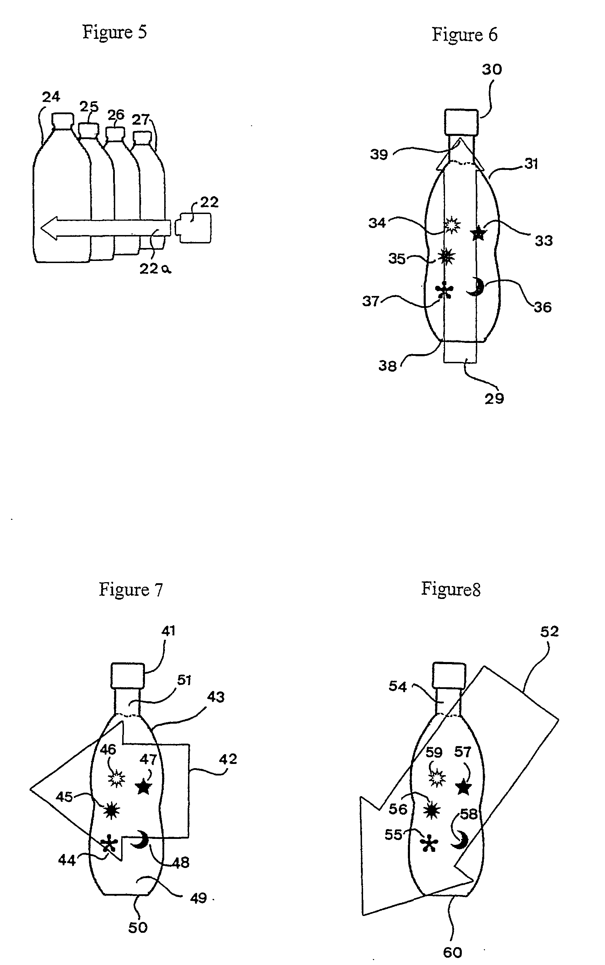

When atoms are raised to an excited state, they emit only in very narrow lines with virtually no emission between the lines. The low-pressure mercury lamp is a very common lamp of this type. For example, The emission lines of a mercury lamp are only sharp when the pressure of the gas is low (<10 tore). If the pressure is increased, the lamp can carry much more power, but the emission lines broaden. For the same length of lamp (about 120cm), a medium pressure lamp (pressure about 1000 tore) can carry up to 30,000W. These lamps are very common in commercial Systems utilizing ultraviolet light. Figure 5 shows a comparison of the emission of low pressure and medium pressure lamps in the ultraviolet region.

Certain radiation units and associated light sources, (lasers) and lamps emit at longer wavelengths, This is the basis of the very popular fluorescent lamp.

Excimer lamps means: Excimer lamps are unique in that they emit in a narrow band of wavelengths. An excimer is an atomic dimmer that is stable only in the excited state and dissociates on decaying to the ground state. Table 4 gives the wavelengths of sonic of the common excimer lamps. Examples: Emission wavelengths (or some common excimer lamps:

| Excimer | Wavelength (nm) | Excimer | Wavelength (nm) |
|---------|-----------------|---------|-----------------|
| Xe2 | 172 | XeCl | 308 |
| KrCl | 222 | I2 | 342 |
| Cl2 | 259 | | |

Flash lamps:

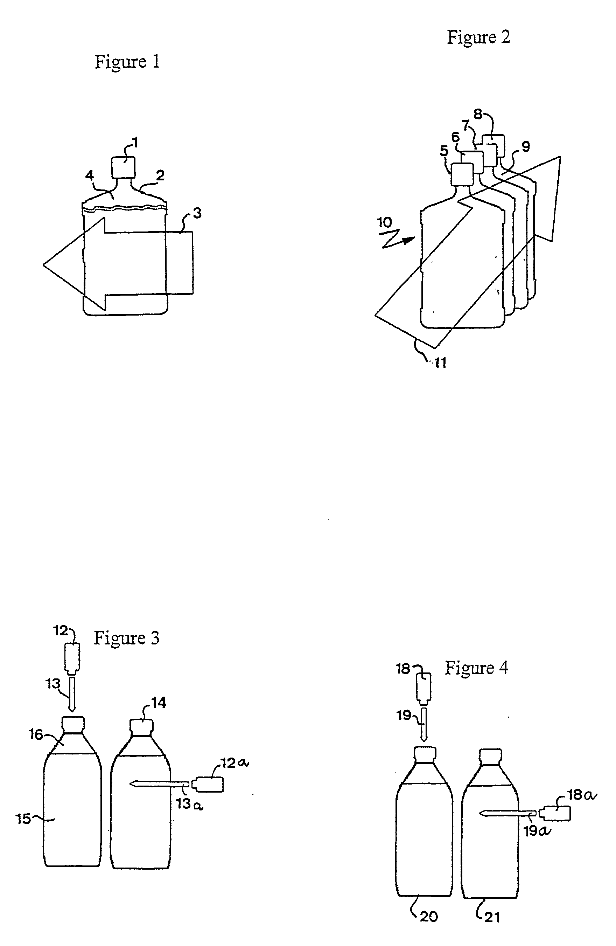

Flash lamps are similar to continuous wave (CW), but could also operate in (PW), pulsed mode operation, and are lamps that consist of a cylindrical quartz tube with electrodes at each end and filled with a ga.½ (e.g.. xenon). A power supply "fires' the lamps by discharging a large amount of electrical energy in a very short period of time (several us) by applying a very high voltage (10 - 30 kV}. The resulting plasma reaches temperatures of 10,000 - 13,000 K and the emission is essentially that of a blackbody (see Fig. 4). In commercial flash lamp Systems the lamp , a typical "flashed" about 30 times per s, but given a special electronic pulsing circuitry is added, repetition rates could reach Khz regime.

Biologically enhanced or photo-chemically polished drinking water or breathing air means any liquid or gas which have been passed through or processed by the method of the present invention such as water and/or air), more specifically such processes involved in the polishing and enhancing may include; Optical inactivation, disinfection, inactivation of DNA and/or RNA replication sequences, Photocatalysis, electro catalysis, a hybrid of photo and electro-catalysis, optical dissociation, physiological dissociation, biomass expansion, filtration (pre/post), physical separation and sorting, reactivation, activation, sonication, acoustics, electroacoustics, electro-optical treatment (by photons of light), transgressing, or transversing said liquids and gasses through a photonic band gaped wave guides having an aerobic, non toxic passage for light and liquids or gasses or combination together, synchronously and or separately.

Peak power means, the energy generated when squeezing (i.e. such as when pulsing) electromagnetic energy in short duration of time, for example: a pulse of a given average energy and power - lasting, or having a pulse width of around 1 second (1s) will generate several watts in peak power, a pulse lasting or having width of microseconds (ms) will generate peak powers reaching the kilo-watts scale, while a pulse lasting nano (ns) seconds will generate peak powers reaching into the hundreds of million of watts which is especially beneficial for purposes such as optical dissociation, optical inactivation, optical polishing, and optical secretion and spectroscopy for control and diagnostics, so in short the shorter the pulse duration the higher its respective peak power.

A hybrid of light sources means a plurality of light sources and wherein their total spectral emitence, or total spectral distribution, or their total irradiance will cause multiphotonic absorption processes by means of super imposing their time domain (example: 1 light source is slow = 1s pulse duration, and additional light sources are very fast =5ns laser for example), their total irradiance is great and beneficial for the processes of the method of the present invention to occur efficiently, further more, such hybridization could includes, lamps and lasers, lasers and flash lamps, or any combination of CW, or PW type of light source working together, in synchronisity, and or sequentially or link or resolved by time domain manipulation for maximizing photonic interaction in matter, especially beneficial for triggering the catalytic scintillating elemental compound according to the present invention.

Photo catalysis means: The use of energy of a photon of light to catalyze chemical reactions. More specifically, such reaction may include the decomposition of water into hydrogen, and oxygen, and the complete oxidation of organic contaminants in aqueous environments. More specifically, the first step in Photocatalysis is for the catalyst material to absorb photon of light in order to excite an electron from the velance band (VB), to the conduction band (CB), thus creating electron-hole-pairs. Each Species must then migrate to the surface before recombination occurs. If this condition is met, the electron can be transferred to a surface adsorbed molecule, reducing it. The overall process is illustrated , it is important to note that for the processes to occur efficiently (preventing pre-matured recombination of the said electron-hole pairs), the rates of reduction, and the oxidation must be comperable .The position of the band edges is critical for each step of the process, a photocatalyst material which is stable in water is tio2, (or known as Titanium Oxide).

Absorbence in the context of the present invention means the ability of a medium to absorb radiation depending on temperature and wavelength. expressed as the negative common logarithm of the transmittance.

Active medium in the context of the present invention means a medium in which lasing will take place, rather than absorption, at a given wavelength.

Air-cooled laser in the context of the present invention means a laser using fans to force air over the laser tube and through the power supply. air-cooled lasers have the benefit of needing no water supply, although the fan noise can sometimes be a disadvantage. usually only small and medium power lasers are air-cooled. very small lasers, typically helium-neon, need no fans. although technically they are "air cooled" via convection, the term is usually applied only to fan-forced cooling.

Average Power in the context of the present invention means the sums of the energy of all single discrete pulses, in one second, of a pulsed laser.

Beam Expander in the context of the present invention means optical device increasing beam diameter and reducing divergence.

Beam Splitting in the context of the present invention means optically splitting a laser beam into two or more beams, of various or identical energies.

CW in the context of the present invention means an abbreviation of continuous wave of a laser as opposed to pulsed operation.

D.T.P means disinfection through packaging .

Duty Cycle in the context of the present invention means the length of time the laser beam is actually cutting, drilling, welding, or heat-treating, as compared to the entire work cycle time.

Flashlamp in the context of the present invention means source of powerful light; often in the form of a helical coil and used to excite photon emission in a solid-state laser.

OPB in the context of the present invention means Opto-Pro-Biotic such that after processing of photo-treatment through packaging (DTP) by the methodology of the present invention contributes to the opulent, or positive immune responses such as generated by MHC type I II III (Major Hysto Compatibility Complex) or positive immune responses of the acquired immune system driven by traceable recognition of the dissociated components of noxious species therein (i.e. in the packaging) without damage to the geometrical integrity of said components.

Packaging in the context of the present invention reffers to any type of material which may hold liquides, gasses or solids, or a combination thereof permanently or temporarily, such as bottled water package or capsoule for bio-medical utilization, or an envelope.

Package in the context of the present invention means the same as packaging.

Mode in the context of the present invention means a particular functioning arrangement, setup, or condition for laser operation, such as continuous emission, pulses, or grouped pulses. "mode" also describes the cross-sectional shape of the beam. (see "tem.")

Modulation in the context of the present invention means the ability to superimpose an external signal on the output beam of the laser as a control.

nd:glass laser in the context of the present invention means a solid-state laser of neodymium:glass offering high power or short pulses, or both, for specific industrial applications.

nd:yag laser in the context of the present invention means a solid-state laser of neodymium:yttrium-aluminum garnet, similar to the nd:glass laser. both are pumped by flash lamps, or diode lasers.

Optical Pumping in the context of the present invention means exciting the lasing medium by the application of light rather than electrical discharge from anode and cathode.

Peak Power in the context of the present invention means the power of an individual pulse in a pulsed laser. it is obtained by dividing the pulse energy in joules to the pulse width in seconds. typical values can be reach mega and giga watts.

Power Density: the amount of radiant power concentrated on a surface. units watts per square meters or square centimeters

Pulse Energy in the context of the present invention means the energy of a single, brief emission from a laser programmed for pulsed behavior rather than continuous operation. pulse power can be several times greater than cw emission.

Refraction in the context of the present invention means the change of direction of propagation of any wave, such as an electromagnetic wave, when it passes from one medium to another in which the wave velocity is different. simply put, the bending of incident rays as they pass from one medium to another, such as air to water.

Solid-state Laser in the context of the present invention means a laser where the lasing medium is a solid material such as a ruby rod. these can be optically pumped by a flashlamp or diodes. solid state lasers also include diode lasers as they use electrically pumped solids to produce light. currently, solid-state lasers are too expensive for most light show uses. this may change over the next few years. the most promising solid-state laser uses a material called nd:yag, which produces infrared light. this can be frequency doubled (second harmonic generation) to produce up to 60 watts of green light at 532 nm. the green light can again frequency doubled (fourth harmonic generation) to produce uv light at 266 nm, up to several watts.

Opt-pro-biotic or optoprobiotic means in the context of the present invention that the sterilization, or disinfection, or dissociation, or oxygenation, or reduction, or equalization, or acoustic-optical action of processes according to the methodology of the present invention is aiding or facilitating water content and products beneficial for the human immune system enhancements and strengthening and that this photochemical effects are achieved through the packaging without damaging said packaging or content therein (i.e. in the packaging).

Instrument means in the context of the present invention the radiation unit, light source or laser, optics, and the beam management or combination optronics used to direct the light spatially at the appropriate angular orientation or coupling of the delivered light form said radiation unit or light source through the packaging without causing damage throughout.

DEPTH OF FIELD In the context of the present invention means The working range of the beam, a function of wavelength, diameter of the unfocused beam and focal length of the lens. To achieve a small diameter spot size, and thus a high power density, a short depth of field must be accepted.

Dichroic filters and mirrors In the context of the present invention means A piece of glass with an optical thindim coating that transmits certain colours (wavelengths), and reflects the remaining colours.

Dichroic filters are used to combine or eliminate specific colours as needed in a laser projector. Dichroic mirrors are used to maximise the amount of light reflected from a laser of a particular wavelength. Dichroics should be handled with care to prevent damage to the coating. [see Also: Colour box]

DIODE LASER In the context of the present invention means A semiconductor similar to an LED (light-emitting diode) but which produces coherent light. Diode lasers are small and efficient, which has led to their use in compact disc players and pen-type laser pointers. Currently, diode lasers are too dim or expensive for most light show uses. This is likely to change over the next few years. [see also: Solid-state laser]

DIVERGENCE In the context of the present invention means The angle at which the laser beam spreads in the far field; the bending of rays away from each other, as by a concave lens or convex mirror.

DRIFT, ANGULAR In the context of the present invention means All undesirable variations in output (either amplitude or frequency); angular drift of the beam, measured in milliradians before, during, and after warm-up.

DUTY CYCLE In the context of the present invention means The length of time the laser beam is actually cuffing, drilling, welding, or heat-treating, as compared to the entire work cycle time

ELECTROMAGNETIC WAVE In the context of the present invention means A disturbance which propagates outward from an electric charge which oscillates or is accelerated. Includes radio waves; X-rays; gamma rays; and infrared, ultraviolet, and visible light.

EMISSIVITY, EMITTANCE In the context of the present invention means Rate at which emission takes place; the ratio of the radiant energy emitted by a source or surface to that emitted by a blackbody at the same temperature.

EXPOSURE In the context of the present invention means A measure of the total radiant energy incident on a surface per unit area; radiant exposure.

FAR-FIELD IMAGING In the context of the present invention means An imaging technique with solid-state lasers that has several limitations: non-uniform energy distribution, very short working distances, and poor control of hole geometry.

FIBER OPTIC CABLE In the context of the present invention means Flexible glass or plastic strands made into a cable, used to carry light from one place to another. There are two main types; Step index and Graded index fiber. Within these two main types there are two further subgroups:

Transmission fibers carry the beam with as little loss as possible. They are used to transmit the laser's light to remotely located projection devices.

Display fibers [known as side glow fibers] have no cable jacket, so some light scatters out the side of the strands. The strands themselves look like microscopic neon tubing and become a special effect such as laser-lit "whip" or a glowing "rope" wrapped around objects.

FLASHLAMP In the context of the present invention means Source of powerful light; often in the form of a helical coil and used to excite photon emission in a solid-state laser

FLUORESCENCE In the context of the present invention means The glow induced in a material when bombarded by light. Brewster windows of fused silica fluoresce in UV light, increasing absorption of laser radiation and degrading laser mode and output.

FLUX In the context of the present invention means The radiant, or luminous, power of a light beam; the time rate of the flow of radiant energy across a given surface.

FOCUS In the context of the present Invention means Noun: The point where rays of light meet which have been Converged by a lens. Verb: To adjust focal length for the clearest image.

FOCAL POINT In the context of the present invention means (Same as first definition under "focus" in laser work,) the focal point of the beam relative to the work surface has a critical effect, such as the depth and shape of drilled holes. When the focal point is at the surface, holes are of uniform diameter, When the focus is below the surface, Conical holes are drilled.

FOLDED RESONATOR in the context of the present invention means Construction in which the interior optical path is bent by mirrors mounted on corner blocks bolted into pre-aligned position, permitting compact packaging of a long laser cavity

FREQUENCY In the context of the present invention means The number of light waves passing a fixed point unit of time, or the number of complete vibrations in that period of time.

GAIN In the context of the present invention means Another term for amplification, usually referring to the efficiency of a lasing medium in attaining a population inversion. High gain is typically more than 50% per pass of the light wave between cavity mirrors

GAS DISCHARGE LASER In the context of the present invention means A laser containing a gaseous lasing medium in a glass tube in which a constant flow of gas replenishes the molecules depleted by the electricity or chemicals used for excitation. The discharged gas can be filtered and 90% recycled for economy.

GAS JET ASSIST In the context of the present invention means An assisting coaxial gas, such as oxygen, argon, or nitrogen, which may be used to achieve very high power levels for cutting certain metals.

GAS TRANSPORT In the context of the present invention means A laser design, which generates Very high beam power within a fairly small resonator structure. Long electrodes parallel the axis and gas is circulated across the resonator cavity.

GAUSSIAN In the context at the present invention means The "normal curve" or normal distribution, an example of which is the symmetrical bell shape of the holes created by the uncorrected, unfocused laser beam in its optimum mode. A Gaussian laser beam has most of its energy in the center

HAZ In the context of the present invention means Heat-Affected Zone, or the area where laser beam and metal (or other) surface are in contact.

HELIUM-NEON LASER In the context of the present invention means ("HeNe"), Laser in which the active medium is a mixture of helium and neon, which is in the visible range. Used widely in industry for alignment, recording, printing, and measuring, it is also valuable as a pointer or aligner of invisible $CO_2$ laser light.

HEAT SINK In the context of the present Invention means A substance or device used to dissipate or absorb unwanted heat, as from a manufacturing process (or with lasers, from reflected rays).

HERTZ In the context of the present invention means The approved international term, abbreviated Hz, which replaces CPS for cycles per second.

IMAGE In the context of the present Invention means The optical reproduction of an object, produced by a lens or mirror. A typical positive lens converges rays to form a "real" image which can be photographed. A negative lens spreads rays to form a "virtual" image which can't be projected.

INCIDENT LIGHT In the context of the present invention means A ray of light that falls on the surface of a lens - or any other object. The "angle of incidence" is the angle made by the ray with a perpendicular to the surface.

INTENSITY In the context of the present invention means The magnitude of radiant energy (light) per unit, such as time or reflecting surface.

ION LASER In the context of the present Invention means A type of laser employing a very high discharge current, passing down a small bore to ionize a noble gas such as argon or krypton. The ionization process creates a population inversion for lasing to occur. A research laser useful for some industrial applications.

IONIZATION In the context of the present invention means The process by which ions are formed.

IRRADIATION In the context of the present invention means Exposure to radiant energy, such as heat, X-rays, or light; the product of irradiance and time.

JOULE In the context of the present invention means One watt per second; a measurement frequently given for laser output in pulsed operation.

KRYPTON LASER In the context of the present invention means A laser filled primarily with krypton gas. When used with "all-line' or 'white" optics, it produces red, yellow, green and blue light. A "red-only" krypton laser uses with specially tailored optics to output a very strong red line at 647 nm. Kryptons are similar to argons (the same tube design can be used for both). However krypton gas produce less light (output power) than an equivalent volume of argon gas. Krypton lasers are primarily used when a powerful red light is needed.

LASER In the context of the present invention means "Laser" is an acronym derived from "Light Amplification by Stimulated Emission of Radiation"

A device which produces a coherent beam of light. The beam remains parallel for long distances and contains one or more extremely pure colours.

Light show lasers are usually gas-filled tubes using high voltage current to ionise the gas (cause the gas to glow). Mirrors at each end of the tube help amplify a process called "stimulated emission". Most of the stimulated emission light travels between the two mirrors; between 1% and 4% comes out of one of the mirrors to create the beam of light that we see.

The gas used determines the colour (or colours) of the beam. Gas lasers remain the overwhelming choice for display applications. The four main types used are a helium neon mixture, argon, krypton; and an argon-krypton "mixed gas" mixture.

LASIER OSCILLATION In the context of the present invention means The buildup of the coherent wave between laser cavity end mirrors. In CW mode, the wave bounding back and forth between mirrors transmits a fraction of its energy on each trip; in pulsed operation, emission happens instantaneously.

LASER ROD In the context of the present invention means A solid-state, rod-shaped lasing medium in which ion excitation is caused by a source of intense light, such as a flashlamp Various materials are used for the rod, the earliest of which was synthetic ruby crystal.

LIGHT In the context of the present invention means The range of visible electromagnetic radiation frequencies detected by the eye, or the wavelength range from about 400 to 750 nanometers It is sometimes extended to include photovoltaic effects and radiation beyond visible limits.

LIGHT REGULATION In the context of the present invention means A form of power regulation in which output power is maintained at a constant level by controlling discharge current.

LUMINANCE In the context of the present invention means Commonly called illumination; the luminous or visible flux per unit area on a receiving surface at any given point.

MENISCUS LENS In the context of the present invention means The lens used primarily in CO2 lasers by Coherent Inc. It has one side convex, the other concave.

METASTABLE, METASTABLE STATE In the context of the present invention means Unstable condition in which the energy of a molecule is at some discrete level above the lowest or ground state. It is this condition which is necessary for emission of photons in a laser. (From quantum theory.)

MILLIJOULE: One thousandth of a Joule

MILLIWATT In the context of the present invention means One thousand milliwatts equal one watt. Small lasers' beam powers are measured in milliwatts. For example, a 50 mW laser is one-twentieth of a watt; a 500 mW laser is one-half watt

MODE In the context of the present invention means A particular functioning arrangement, setup. or condition for laser operation, such as continuous emission, pulses7 or grouped pulses. "Mode" also describes the cross-sectional shape of the beam. (See "TEM.")

MODULATION In the context of the present invention means The ability to superimpose an external signal on the output beam of the laser as a control.

MONOCHROMATIC LIGHT In the context of the present invention means Theoretically, light consisting of just one wavelength. Since no light is completely monochromatic, it usually consists of a very narrow band of wavelengths. Lasers provide the narrowest bands.

NANOMETER In the context of the present invention means A unit of length in the International System of Units (SI) equal to one billionth of a meter (10-9 meter). Once called a millimicron, it is used to represent wavelength. Abbreviated "nm."

NEAR FIELD IMAGING In the context of the present invention means A solid-state Laser imaging technique offering control of spot size and hole geometry, adjustable working distance, uniform energy distribution, and easily produced range of spot sizes.

Nd:GLASS LASER In the context of the present invention means A solid-state laser of Neodymium: glass offering high power or short pulses, or both, for specific industrial applications.

Nd:YAG LASER In the context of the present invention means A solid-state laser of Neodymium:Yttrium-Aluminum Garnet, similar to the Nd:glass laser. Both are pumped by flash lamps, or diode lasers.

NEMA In the context of the present invention means National Electrical Manufacturers' Association, a group which defines and recommends safety standards for electrical equipment.

NOISE In the context of the present invention means Unwanted, minor currents or voltages in an electrical system.

OBJECT In the context of the present Invention means The subject matter or figure imaged by, or seen through, an optical system.

OPTICAL DENSITY In the Context of the present invention means Protection factor provided by a filter (such as used in eyewear, viewing windows, etc.) at a specific wavelength Each unit of CD represents a 1Ox increase in protection.

OPTICAL PUMPING In the context of the present Invention means Exciting the lasing medium by the application of light rather than electrical discharge from anode and cathode.

OUTPUT COUPLER In the context of the present invention means The resonator mirror which transmits light; the one at the Opposite end is totally reflective.

OUTPUT POWER In the Context of the present invention means The energy per second emitted from the laser in the form of coherent light, usually measured in watts for continuous-wave operation and joules for pulsed operation

PEEK POWER In the context of the present invention means The power of an individual pulse n a pulsed laser. it is obtained by dividing the pulse energy in Joules to the pulse width in seconds. Typical values can be reach Mega and Giga Watts.

PHOTOACOUSTIC EFFECTS In the context of the present invention means Arises with the use of very short-duration high-energy laser pulses, at pulse durations typically below 10 microseconds. Significant amounts of energy are absorbed and a rapid expansion occurs in the tissue generating an acoustic shock wave that causes mechanical disruption to cellular structures.

PHOTOCHEMICAL EFFECTS In the context of the present invention means Effects that occur from long exposure durations at incident power levels insufficient to cause damaging Photothermal effects. It is an energy dependent process (a function of the total quantity of radiation absorbed rather than its rate of absorption).

PHOTOMETER In the context of the present invention means An instrument which measures luminous intensity.

PHOTON In the context of the present invention means In quantum theory, the elemental unit of light, having both wave and particle behavior. It has motion, but no mass or charge.

PHOTOTHERMAL EFFECTS In the context of the present invention means The damage mechanism for acute laser injury (i.e. for injury immediately following exposure). The radiation incident at the surface is absorbed in the underlying tissue, increasing the temperature of the tissue to the level at which damage can occur, and laser burns result. It is a power dependent process (a function of the RATE at which energy is absorbed rather than the total quantity of energy involved).

PLASMA In the context of the present invention means In laser welding, a metal vapor that forms above the spot where the beam reacts with the metal surface. Also used to describe the laser tube (plasma tube, discharge tube) which contains the completely ionized gas in certain lasers.

POLARIZATION In the context of the present invention means Restriction of the vibrations of the electromagnetic field to a single plane, rather than the innumerable planes rotating about the vector axis. This prevents optical losses at interfaces between the lasing medium and optical elements. Various forms of polarization include random, linear (plane), vertical, horizontal, elliptical, and circular. Of two polarization components (so-called), S and P, the P component has zero losses at Brewster's angle.

POPULATION INVERSION In the context of the present invention means When more molecules (atoms, ions) in a laser are in a metastable state than in the ground state (a situation needed for sustaining a high rate of stimulated emissions), a "population inversion" is said to exist. Without a population inversion, there can be no lasing action.

POWER DENSITY: The amount of radiant power concentrated on a surface. Units Watts per square meters or square centimeters.

PULSE ENERGY In the context of the present invention means The energy of a single, brief emission from a laser programmed for pulsed behavior rather than continuous operation. Pulse power can be several times greater than CW emission.

PULSE TAIL In the context of the present invention means Pulse decay time, which can be shortened (by using a special mixture of gases) to allow for fast repetition of laser pulses within a given length of time.

Q-SWITCH In the context of the present invention means A device that has the effect of a shutter moving rapidly in and out of the beam to "spoil" the resonator's normal Q, keeping it low to prevent lasing action until a high level of energy is stored. Result: a giant pulse of power when normal Q is restored.

QUASI CW In the context of the present invention means The pulsating of a continious light into pulsed light by acusto optic, electrooptic, electronic, or mechano optical means, so peak powers are reduced, by number of pulses (see rep. Rate) are increased

RADIANCE In the context of the present invention means Brightness; the radiant energy per unit solid angle and per unit projected area of a radiating surface.

RADIANT ENERGY In the context of the present invention means Energy traveling as wave motion; specifically, the energy of electromagnetic waves (light, X-rays, radio, gamma rays).

RADIANT FLUX: The rate of emission or transmission of radiant energy.

RADIANT INTENSITY In the context of the present invention means Radiant power, or flux, expressed as emission per unit solid angle about the direction of the light in a given length of time

RADIANT POWER In the context of the present invention means The amount of radiant energy available per unit; the radiant flux.

REFLECTANCE In the context of the present invention means The ratio of the reflected flux to the incident flux, or the ratio of reflected light to light falling on the object.

Reflection In the context of the present invention means the return of radiant energy (incident light) by a surface, with no change in wavelength.

REFRACTION In the context of the present invention means The change of direction of propagation of any wave, such as an electromagnetic wave, when it passes from one medium to another in which the wave velocity is different. Simply put, the bending of incident rays as they pass from one medium to another, such as air to water.

RESOLUTION In the context of the present invention means Resolving power, or the quantitative measure of the ability of an optical instrument to produce separable images of different points on an object; the capability of making distinguishable the individual parts of an object, closely adjacent images, or sources of light.

RESONATOR In the context of the present invention means The mirrors (or reflectors) making up the laser cavity containing the laser rod or tube. The mirrors reflect light back and forth to build up amplification under an external stimulus. Emission is through one of them, called a coupler, which is partially transmissive.

ROCKWELL C In the context of the present invention means A scale or test used to define hardness in metals, particularly steel and titanium.

SOLID-STATE LASER In the context of the present invention means A laser where the lasing medium is a solid material such as a ruby rod. These can be optically pumped by a flashlamp or diodes. Solid state lasers also include diode lasers as they use electrically pumped solids to produce light. Currently, solid-state lasers are too expensive for most light show uses. This may change over the next few years. The most promising solid-state laser uses a material called Nd:YAG, which produces infrared light. This can be frequency doubled (Second Harmonic Generation) to produce up to 60 watts of green light at 532 nm The green light can again frequency doubled (Fourth Harmonic Generation) to produce UV light at 266 nm, up to several watts.

SPECTRAL RESPONSE In the context of the present invention means The response of a device or material to monochromatic light as a function of wavelength.

STIMULATED EMISSION In the context of the present invention means When an atom, ion, or molecule capable of lasing is excited to a higher energy level by an electric charge or other means, it will Spontaneously emit a photon as it decays to the normal ground state. If that photon passes near another atom of the same frequency which is also at some metastable energy level, the second atom will be stimulated to emit a photon. Both photons will be of the same wavelength, phase, and spatial coherence. Light amplified in this manner is intense, coherent (collimated or parallel), and monochromatic. In short, laser light.

TEM In the context of the present invention means Abbreviation for Transverse

Electromagnetic Mode, the cross-sectional shape of the working laser beam. An infinite number of shapes can be produced, but only a relatively small number are needed for industrial applications. In general, "the higher the TEM, the coarser the focusing"

TEMOO: A Gaussian-curve mode that is the best collimated and produces the smallest spot of high power density for druling, welding and cutting.

TEMOI Divided into two equal beams for special applications.

THRESHOLD In the context of the present invention means During excitation of the laser medium, this is the point where lasing begins.

TRANSMISSION In the context of the present invention means In optics, the passage of radiant energy (light) through a medium.

TRANSMITTANCE In the context of the present invention means The ratio of transmitted radiant energy to incident radiant energy, or the fraction of light that passes through a medium.

VIGNETTING In the context of the present invention means The loss of light through an optical element when the entire bundle does not pass through; an image or picture that shades off gradually into the background.

VISIBLE LIGHT TRANSMISSION/TRANSMITTANCE In the context of the present invention means The amount of visible light usable to the eye that passes through a filter. As a rule of thumb, as optical density increases, visible light transmission decreases - but not always.

WATT In the context of the present invention means An objective measure of power; in lasers usually refers to the optical output power, or strength, of a laser beam. Watts are also used in a more conventional sense, to measure electrical power used by a laser. For example, a 10 W (optical) argon laser consumes around 10,000 W of electrical power.

WAVE In the context of the present invention means An undulation or vibration a form of movement by which all radiant energy of the electromagnetic spectrum is thought to travel.

WAVELENGTH In the context of the present invention means The fundamental property of light-the length of the light wave, which determines its color Common units of measurement (which is usually from crest to crest) are the micron, the nanometer, and (earlier) the angstrom. Visible light has wavelengths extending from about 700 nanometers (red) through orange ($\sim$600 nm), yellow ($\sim$580 nm), green ($\sim$550 nm), blue ($\sim$450 nm) and violet ($\sim$400 nm).

WHITE-LIGHT BEAM In the context of the present invention means Broadly, a laser beam which contains a number of different wavelengths (colours) so the beam appears white. If the beam is passed through a prism or diffraction grating, it is separated into individual laser beams, each of a single specific wavelength. More specifically, a white-light beam ideally contains twice as much red as green and blue light for correct colour balance (see appendix). It can be from a single white-light laser or from two or three lasers whose beams have been combined into a single beam. White-light beams are primarily used in RGB laser projectors See the definition of white-light laser for more information on what constitutes an "equal mixture" of light.

WHITE-LIGHT LASER In the context of the present invention means Many lasers can produce a number of wavelengths (colours) simultaneously. A white-light laser is designed to give a good balance of red, green and blue wavelengths. Usually the laser is intended for an RGB laser projector. (Some models also deliberately add yellow light for specialised 4-colour projectors.) Most white-light lasers use an anrgon/krypton gas mixture. It is somewhat difficult to produce an equal balance of desired colours, and to keep this balance consistent during the lifetime of the laser tube. At present, there are no standards defining the exact wavelengths and colour proportions for a laser to be called "white-light" In addition, the sought-after colour balance can be defined either as equal amounts on a photometer, or as visually equal amounts. Since the eye is much more sensitive to green, a visually equal or "photopically balanced" laser has roughly five times more power in red and blue than in green. Most white-light lasers today are not photopically balanced.

WINDOW In the context of the present invention means A piece of glass with plane parallel sides which admits light into or through an optical system and excludes dirt and moisture.

In the context of the present invention Acquired immunodeficiency Syndrome means A life threatening disease caused by virus and characterized by breakdown of the body's immune defenses, specie specific calibration standards mean in the context of the present invention the efficient reduction of viruses which causing immunodeficiency syndromes without interfering with the body external surfaces.

In the context of the present invention Active immunity means that the immunity produced by the body in response to stimulation by a disease causing organism or vaccine, or treatment according to the present invention wherein blood is circulated in the body, or in loop to/from the body through optronic dialysis according to the methodology of the present invention and therapeutic responses are following as a result of the treatment according to the methodology of the present invention.

In the context of the present invention Agamaglobulinemia means An almost total lack of immunoglobulines, and/or antibodies.

In the context of the present invention Allergen means any substance that causes an Allergy, and/or allergic reaction by the misfunctioning of the MHC type 1, 2, 3, and/or any combination thereof.

In the context of the present invention Allergy means An inappropriate and harmful response of the immune system to normally harmless substances (i.e. see Alergic reaction as an example)

In the context of the present invention Anaphylactic Shoch a life threatening Alergic reaction characterized by swelling of the body tissues including the throat, difficulties in breathing, and a sudden fall in blood pressure.

In the context of the present invention Anergy means A state of unresponsiveness, induced when the T cell antigen receptor is stimulated, that effectively freezes T cell responses, pending a 2ND Signal from the Antigen presenting cell.

In the context of the present invention Antibody means a soluble protein molecule produced and secreted by B cell in the response to an antigen, which is capable of binding to the specific antigen.

In the context of the present invention Antibody-dependent Cell - mediated Citotoxicity (ADCC) means in the context of the present invention An immune response in which antibody, by coating target cells makes them vandurable to attack by immune cell (see coating and marking cells)

In the context of the present invention Antigen means any substance that, when in traduced into the body is recognized by the immune system.

In the context of the present invention Antigen presenting cells means B cells of the monocyt lineage (including macrophages as s dantritic cells), and various other body cells that present antigen in a form that T cells can recognize.

In the context of the present invention Anti nuclear antibody (ANA) means an autoantibody directed against a substance in the cell nucleus.

In the context of the present invention Antiserum means a Serum that contains antibodies.

In the con text of the present invention the refractiv index of water is 1.3, and the refractive index of air is 1.00, thus when light is coupled to a real time flowing liquid wave guide it getslocked, and relected by T.I.R (Total Internal Reflections) thus creating a lighjet (uvjet) according to the methodology of the present invention.

In the context of the present invention Antitoxins mean Antibodies that in terlockk with and inactivate toxins produced by certain bacteria.

In the context of the present invention Appendix means a lymphoid organ in the intestine.

In the context of the present invention Attenuated means a weakened; no longer infectious (innocuous form)

In the context of the present invention Autoantibody an antibody that reacts against persons own tissue.

In the context of the present invention Auoto immune desease a desease that result when the immune system mistakenly attacked the bodies own tissues. For example the Rheumatoid arthritis and systemic lupus erythematosus aree auto immune diseases.

In the context of the present invention Bacterium means a microscopic noxious microorganism composed of a single cell. Mant but not all bacteria cause desease.

In the context of the present invention Bassophile means A white blood cell that contributes to inflammatory reactions. A long with mast cells, Basophiles are responsible for the symptoms of Allergy. (and/or Alergic reactions).

In the context of the present invention B cells means small white blood cells crucial to the immune defenses. Also known as B lymphocites, they are derived from bon Marrow and develop into plasma cells that are the source of antibodies.

In the context of the present invention Biological response modifiers means a substances, either natural or synthesized, that boost, direct, or restore normal immune defenses. BRMs include interferons, interleukins, thymus hormones and monoclonal antibodies.

In the context of the present invention Biotechnology means the use of living organisms or their products to make or modify a substance. Biotechnology includes recombinant DNA technique (such as genetic engineering), and hybridoma technology.

In the context of the present invention Bon-marrow means soft tissue located in the cavities of the bones. The bone marrow is the source of all blood cells.

In the context of the present invention Cellular immunity means immune protection provided by the direct action of the immune cells (as distinct from soluble molecules such as antibodies).

In the context of the present invention Chromosomes mean physical structures in the cell's nucleus that have the genes. Each Human cell has 23 pares of chromosomes.

In the context of the present invention Clone A group of genetically identical cells or organisms descended from a single common ancestor., (v) to reproduce multiple identical copies.

In the context of the present invention Complement means a complex series of blood proteins whose action complements the work of antibodies. Complement destroys bacteria produces inflammation, and regulates the immune reactions.

In the context of the present invention Complement cascade means a precise sequence of events usually triggered by an antigen/antibody complex in which each component of the complement system is activated in turns.

In the context of the present invention Constant region means that part of an Antibody structure that is characteristic for each antibody class.

In the context of the present invention Co stimulation means the delivery of a 2nd signal from an antigen-presenting cell to a T cell. The 2nd signal rescues the activated T cell from Anergy, allowing it to produce the lymphokinds necessary for the growth of additional T cells.

In the context of the present invention Cytikines means powerful chemical substances secreted by Cells. Cytokines include lymphokines produced by lymphocytes and monokines produced by monycites and macrophages.

In the context of the present invention Dandritic cells means] white blood cells found in the spleen and other lymphoid organs. Dandritic cells typically use thread like tentacles to enmesh antigen, which they present to T cells.

In the context of the present invention DNA (deoxyribonucleic acid) means a nucleic acid that is found in the cell nucleus and that is the carrier or co. represents genetic information. (I.e. see RNA, Ribonucleic Acid or any combination thereof)

In the context of the present invention Enzyme means a protein; produced by living cells that promotes the chemical processes of life without itself being altered.

In the context of the present invention Eosinophil means a white blood cell that contains granules filled with chemicals damaging to parasites, and enzymes that damp down inflammatory reactions.

In the context of the present invention Epitop means a unique shape or marker carried on antigen's surface, which triggers a corresponding antibody response.

In the context of the present invention Fungus means a member of a class of relatively primitive vegetable organism. Fungi include mushrooms, yeast, rusts, molds and smuts.

In the context of the present invention Gene means a unit of genetic material (DNA) that carries the directions a cell uses to perform a specific function, such as making a given protein.

In the context of the present invention Graft-versus-host disease (GVHD) means a life threatening reaction in which transplanted immunocompetent cells attack the tissues of the recipient (such as in transplant medical procedures).

In the context of the present invention Granulocytes means A white blood cell file with granules containing potent chemicals that allow the cell to digest micro organisms (noxious types and non noxious types) or to produce inflammatory reactions. Neutrophiles eosinophiles and basophiles are examples of granulocytes.

In the context of the present invention Helper T cells means A subset of T cells that tipically carry the T4 marker and are essential for turning on the antibody production, activating cytotoxic T cells, and initiating many other immune responses.

In the context of the present invention Hematopoiesis means the formation and development of blood cells, usually takes place in the bone marrow.

In the context of the present invention Histo Compatibility Testing means A method of matching the self-antigens (HLA) on the tissues of a transplant doner with those of the recipient. The closer the natch, the better the chance that the transplant procedure will be successful "and will take"

In the context of the present invention HIV means (Human Immunodificiancy virus) it is the virus that causes AIDS.

In the context of the present invention Human lococyte antigens (HLA) means a protein in markers of self use in histocompatibility testing. Soma HLA types also correlate with certain autoimmune diseases.

In the context of the present invention Humoral immunity means an immune protection provided by the soluble factors such as antobodies, which circulate in the body fluids or Humors, primarily Serum and lymph.

In the context of the present invention Hybridoma means A Hybrid cell created by fusing B lymphocytes with as long lived neoplastic plasma cell or T lymphocyte with a lymphoma cell. B cell Hybridoma secretes a signal specific antibody.

In the context of the present invention VOCs reduction means reduction of volatile organic compounds by the use of a flowing liquid wave guide (could be used also for TOC reduction in the same principle)

In the context of the present invention Hypogammmaglobuilinemia means is an abnormally low level of immunoglobuline.

In the context of the present invention Ideotypes means a unique and characteristic part of an antibody variable region, which can dam cells serve as antigens.

In the context of the present invention Immune Complex (IC) means a Cluster of interleukines, antigens and antibodies.

In the context of the present invention Immune response means the reactions of the immune system to foreign substances.

In the context of the present invention Immunoassay means A test using antibodies to identify and quantify substances. Often the antibody is link to a marker such as a fluorescent molecule, a radio active molecule or an enzyme type, or combination of the above

In the context of the present invention Immunocompetent means the capability of developing an immune response.

In the context of the present invention Immunoglobulines means a family of large protein molecules also known as antibodies.

In the context of the present invention Immunosupression means A reduction of the immunresponses for instance by giving drugs to prevent transplant rejection/s.

In the context of the present invention Immunotoxines A monoclonal antibody linked to a natural toxins, a toxic drugs or radioactive substance or combination.

In the context of the present invention Inflammatory response means A redness, warmth, swelling, pain, and/or loss of function produced in response to infection as the result of increased flood flow and an influx of immune cells and secretions.

In the context of the present invention I8nterleukines means A major group of lymphokines and monokines.

In the context of the present invention Kupffer cells means specialized macrophages in the liver.

In the context of the present invention LAK cells means lymphocytes transformed in the laboratory into lymphokines activated Killer cells, which attack tumor cells.

In the context of the present invention Langerhans cells means A drandritic cells in the skin that pick up antigen and transport it to lymph nodes.

In the context of the present invention Leukocytes and all white blood cells.

In the context of the present invention Lymph means a transparent, slightly yellow fluid that carries lymphocytes, bathes the body tissues, and drains into the lymphatic vessels.

In the context of the present invention In the context of the present invention Lymphatic vessels mean a body-wide network of channels, similar to the blood vessels, which transport lymph to the immune organs and into the bloodstream.

In the context of the present invention Lymph nodes mean small bean-shaped organs of the immune system, distributed widely throughout the body and linked by lymphatic vessels. Lymph nodes are garrisons of B, T, and other immune cells.

In the context of the present invention Lymphocyte means small white blood cells produced in the lymphoid organs and paramount in the immune defenses.

In the context of the present invention Lymphoid organs mean the organs of the immune system, where lymphocytes develop and congregate. They include the bone marrow, thymus, lymph nodes, spleen, and various other clusters of lymphoid tissue. The blood vessel and lymphatic vessels can also be considered lymphoid organs.

In the context of the present invention Lymphokines means powerful chemical substances secreted by lymphocytes. These soluble molecules help direct and regulate the immune responses.

In the context of the present invention Macrophages mean a large and versatile immune cell that acts as a microbe-devouring phagocyte, an antigen-presenting cell, and an important source of immune secretions.

In the context of the present invention Major histocompatibility complex (MHS) means a group of genes that controls several aspects of the immune response. MHC genes code for self-markers on all body cells.

In the context of the present invention Mast cells means a granule-containing cell found in tissue. The contents of mast cells, along with those of basophils, are responsible for the symptoms of allergy.

In the context of the present invention Microbes means minute living organisms, including bacteria, viruses, fungi and protozoa.

In the context of the present invention Microorganisms means microscopic plants or animals.

In the context of the present invention Molecule means the smallest amount of a specific chemical substance that can exist alone. (The break a molecule down into its constituent atoms is to change its character. A molecule of water, for instance, reverts to oxygen and hydrogen).

In the context of the present invention Monoclonal antibodies mean antibodies produced by a single cell or its identical progeny, specific for a given antigen. As a tool for binding to specific protein molecules, monoclonal antibodies are invaluable in research, medicine and industry.

Monocyte means a large phagocytic white blood cell which, when it enters tissue, develops into a macrophage.

In the context of the present invention Monokines means powerful chemical substances secreted by monocytes and macrophages. These soluble molecules help direct and regulate the immune responses.

In the context of the present invention Natural killer (NK) cells mean large granule-filled lymphocytes that take on tumor cells and infected body cells. They are known as natural killers because they attack without first having to recognize specific antigens.

In the context of the present invention Neutrophil means a white blood cell that is an abundant and important phagocyte.

In the context of the present invention Nucleic acids means large, naturally occurring molecules composed of chemical building blocks known as nucleotides. There are two kinds of nucleic acids, DNA and RNA.

In the context of the present invention OKT3 means a monoclonal antibody that targets mature T cells.

In the context of the present invention Opportunistic infection means an infection in an immunosuppressed person caused by an organism that does not usually trouble people with healthy immune systems.

In the context of the present invention Opsonize means to coat an organism with antibodies or complement protein so as to make it palatable to phagocytes.

In the context of the present invention Organism means an individual living thing.

In the context of the present invention Parasite means a plant or animal that lives, grows, and feeds on or within another living organism.

In the context of the present invention Passive immunity means immunity resulting from the transfer of antibodies or antiserum produced by another individual.

In the context of the present invention Peyer's patches means a collection of lymphoides tissues in the intestinal tract.

In the context of the present invention Phagosites means a large white blood cells that contribute to the immune defenses by ingesting microbes or other cells and/or forign particles.

In the context of the present invention Plasma cells means a large antibody producing cell that develops from B cell.

In the context of the present invention Platelets mean A granule containing cellular fragments critical for blood clotting and sealing of wounds. Platelets also contribute to the immune response.

In the context of the present invention Plymorphes means short for Poly Morpho Neuclear locosytes or granulocytes.

In the context of the present invention Proteins means an organic compound made up of aminoacids. Proteins are one of the major constituents of plants and animals and human cells.

In the context of the present invention Protozoa means a group of one-celled animals few of, which can cause Human desease (including maleria and sleepoing sickness types)

In the context of the present invention Rheumatoid factor means an auto antibody found in the serum of most persons with rheumatoid arthritis.

In the context of the present invention RNA (Ribonucleic Acid) a nucleic acid that is found in the cytoplasm and also in the nucleus of some cells. One function of RNA is to direct the synthesis of proteins.

In the context of the present invention Scavenger cells means any of the diverse groups of cells that have the capability to engulf and destroy foreign materials, dead tissues, or other cells.

In the context of the present invention SCID mouse a laboratory animal that, lacking enzyme necessary to fashion an immune system of its own, can be turned into a model of the human immune system when injected with human cells or tissues.

In the context of the present invention Serum means a clear liquid that separates from the blood when it is allowed to clot these fluids retains any antibodies that were present in the whole blood.

In the context of the present invention Severe combined immunodefficency desease (SCID) means A life threatening condition in which infants are born lacking all major immune defenses.

In the context of the present invention Splin means a lymphoid organ in the abdominal cavity that is important center for immune system activities.

In the context of the present invention Stem cells mean Cells from which all blood cells are derived. The bone marrow is rich in stem cells.

In the context of the present invention Suunit vaccine means a vaccine that uses marely one component of an infectious agent rather then the whole to stimulate an immune response.

In the context of the present invention Supeantigens means a class of antigens, including certain bacterial toxins that unleash a massive and damaging immune response.

In the context of the present invention Suppresser T cells means a subset of T cells that turn off antibody production and other immune response.

In the context of the present invention T cells means small white blood cells that orchestrate and/or directly participate in the immune defenses. Also known as T lymphocytes, they are processed in the thymus and secret lymphokines.

In the context of the present invention Thymus means a primary lymphoides organ, high in the chest where T lymphocyte proliferate and mature.

In the context of the present invention TIL means a tumor Infiltrating Lymphocytes. These immune cells are extracted from a tumor tissue, treated in laboratory and re-injected into the cancer patient.

In the context of the present invention Tissue typing means (see MHC, Major Histocompatibility Testing HCT).

In the context of the present invention Tolerance a state of nonresponsivnes to a particular antigen or group of antigens.

In the context of the present invention Tonsils and adenoids means are a prominent oval massive of lymphoid tissues on either side of the throat.

In the context of the present invention Toxins means agents produced by plants and bacteria, normally very damaging to mammalian cells, that can be delivered directly to target cells by lionkiun gthenm to monoclonal antibodies or lymphokines.

In the context of the present invention Vaccine means a substance that contains antigenic components from an infectious organism by stimulating an immune response (but not disease) it protect against subsequent infection by that organism.

In the context of the present invention Variable region means that part of an antibody structure that differs from one antibody to another.

In the context of the present invention Virus means a sub-microscopic microbe that causes infectious disease. A virus can reproduce only in living cells.

In the context of the present invention Libido- pranic a state of increased vitality (i.e. the word prana, pranic means vitality, or states of ample vitality), or such states in which the body is tuned, and the immune system is at rest, empowered by biological traces of specific origin, and nature. Libido is the sensual drive induced as a result of the body being tuned, and/or referenced, and systems in the body aren't over loaded, and thus a state of improved feelings is induced, i.e. a libido-pranic state.

In the context of the present invention Resonativistic means a state of resonance created when energy of high peak powers is being applied, projected into, coupled to, or are being generated into biomass, such resonance is subject to the expansion In the context of the present invention ration, or density of the holding media, or medium, the term represent a state in which light and sound, ultrasound and mechanical, and physiological process are occurring and causing resonance which when said resonance utilizes the individual resonance of elements, organs, or cells of the noxious species, and could identify, or recognize, separate, sort and inactivate, dissociate, or vibrate said biomass,

In the context of the present invention The following terms included to improve the understanding of photochemistry the specific context have been selected for benefits of clarity and familiarization.

Physical Constants of interest in Ultraviolet and Photochemistry means

| Constant | Symbol | Value | Units |
|---|---|---|---|
| Speed of light | c | 2.99792458x108 | M s (-1) |
| Charge on electron | e | 1.60217733x10(-19) | C |
| Planck constant | h | 6.6260755 x10 (-34) | J s |
| Boltzmann constant | k | k 1.380658x10 (-23) | J K (-1) |
| Avogadro number | NA | 6.0221367 x 1023 | mol (-1) |
| Gas constant | R | 8.31451 | J mol (-1) K (-1) |

Further Characteristics of light and context explanatory notes are herewith included:

In the context of the present invention Planck Law of Radiation means in the context of the present invention:

Light which has both particle and wave properties. It is transmitted in discrete packets of energy (photons) and yet has a frequency and wavelength. The connection between these two properties is embodied in the Planck flaw of Radiation

In the context of the present invention Photochemical wave changes means in the context of the present invention:

In the context of the present invention The usual wavelength range in Photochemistry is 100 - 1000 nm. Light photons with wavelengths longer than 1000 nm have a photon energy too Small to cause chemical change when absorbed, and photons with wavelengths shorter than 100 nm have so much energy that ionization and molecular disruptions characteristic of radiation chemistry prevail. The total In the context of the present invention photochemical wavelength range is divided up into bands with specific names as given below.

In the context of the present invention Spectral ranges or interest in Photochemistry means in the context of the present invention:

| Range Name | Wavelength |
|---|---|
| Near Infrared | 700-10000 |
| Visible | 400 - 700 |
| Ultraviolet | |
| UVA | 315-400 |
| UVB | 280-315 |
| UVC | 100-280 |

Little photochemistry occurs in the Near Infrared. Except for some photosynthetic bacteria. Which are capable of storing solar energy at wavelengths out to 980 nm. The Visible range is completely active for photosynthesis in green plants and algae. Also many dyes can undergo photochemical transformations themselves or sensitize reactions in other molecules. Most studies in photochemistry involve the Ultraviolet range. The division into three sub-ranges [UVA, UVB, UVC] is connected with the human skin's sensitivity to ultraviolet light. The UVA range causes changes in the skin that lead to sun tanning. The UVB range can cause sun burning and is known to eventually induce skin cancer. The UVC range is extremely dangerous since it is absorbed by proteins, RNA and DNA and can lead to cell mutations and/or cell death. The UVC range is sometimes called the germicidal range, since it is very effective in inactivating bacteria and viruses. The Vacuum Ultraviolet range is absorbed by almost all substances (including water and air).

Thus it can only be transmitted in a vacuum. The absorption of a VUV photon causes one or more bond

Breaks. However, even though photons with wavelengths less than 561.6 nm are capable of splitting the $H_2O_2$ molecule, no photolysis, or proteolysis occurs in this wavelength region because $H_2O_2$ does not begin to absorb ultraviolet light until below 300 nm. This illustrates the first Law of Photochemistry; namely that no photochemical reaction can occur unless a photon/s of light is absorbed.

In the context of the present invention Coherent and incoherent light means Light sources used in photochemistry can either be coherent (all emitted photons are in phase with each other as they propagate) or incoherent (all emitted photons have random phases). All lasers emit coherent radiation and usually at one wavelength. The dispersion is very small so that a laser beam remains at or near its original diameter as it propagates; the light emitted by all other light sources is almost always incoherent. Most of these sources are either "hot element" sources (e.g., the incandescent light bulb) or "plasma" sources (e.g., a fluorescent light tube).

In the context of the present invention Point sources, means Light sources have finite dimensions (e.g., often a cylindrical shape). Emission from such a source is difficult to treat mathematically. It is convenient to mode] these sources as a collection of point sources, in which all light is emitted from the point equally in all directions. The optics treatment for point sources is especially simple.

In the context of the present invention The Terms and concepts associated with the emission of tight, are herewith included for clarity of explanation and to simplify the understanding of the method of the present invention, especially wherein photochemistry is involved, or photochemical polishing is active in the processing according to the present invention:

The light emitted from a source can be viewed in many different ways. In this Section, the various terms that may be used to describe this emission are defined and explained.

In the context of the present invention Radiant energy means:

Radiant energy (Q) is a total amount of radiant emission (J) from a source over a given period of time,

In the context of the present invention Radiant power means:

The radiant power (P) of a source is the rate of radiant energy or total radiant power (W) emitted in all directions by a light source. For example, the radiant power of the Sun is 3-842 x 1026 w. in theory, P, should include all wavelengths emitted by the source; however, ~ is usually restricted to the wavelength range of interest to photochemistry. For example, if a light source were being used for ultraviolet photochemistry, P would be specified for emission in the 200 - 400 nm ultraviolet ranges.

In the context of the present invention Radiant power efficiency means:

The radiant power efficiency (q) is defined as

$$Q = P/e$$

Where e is the input electrical power (W) supply.

Radiant emittance or excitance means:

The r Radiant emittance or excitance of a source is the radiant power emitted from an infinitesimal area on the surface of the source.

In the context of the present invention Radiant Intensity means:

The radiant intensity (I) (W sr^(-1)) is the total radiant power P emitted by a source in a given direction about an infinitesimal solid angle.

Radiance means:

Radiance (L) is defined as the radiant power $d^2P$, emitted from an infinitesimal area dA of the source surface in a given direction about the solid angle di, divided both by the solid angle di) and the orthogonal projected area.

The emittance M from an infinitesimal surface element dA is obtained by integrating L in spherical polar coordinates over the hemisphere of all outward-bound directions above dA.

An isotropic light source is defined as one in which the radiance L is uniform over all

Outward directions. Terms and concepts associated with the receipt of light When light is emitted from a source, it radiates outward at the speed of light, when it impinges on an object, it may be reflected, transmitted or absorbed. There are several terms that relate to the receipt of light.

Fluence Rate means:

Fluence Rate (E) (W m^(-2) is the radiant power of all wavelengths passing from all
Directions through an infinitesimally small sphere of cross-sectional area d, divided by CM

Irradiance means:

Irradiance (symbol E; units W m^(-2) is defined as the total radiant power of wavelengths incident on an infinitesimal element of surface of area as containing the point under consideration divided by as. The following are some important points regarding characteristics and differences between "irradiance" and "fluence rate":
Examples: For a parallel and perpendicularly incident beam, not scattered or reflected, irradiance and fluence rate becomes identical. For any UV source within a three-dimensional volume, the integration of UV irradiance over the interior surface of the volume yields the UV power of the larnp., This is not true for UV fluence rate.
The appropriate term for UV disinfection is "UV fluence rate" because a
Microorganism can receive UV power from any direction, especially when there is more than one UV lamp in the vicinity. In general usage, the irradiance or fluence rate may be expressed as MW cm^(-2). The irradiance is often incorrectly termed "light intensity" see the proper definition of "radiant intensity" above.

Light dose or fluence means:

The light dose or fluence (symbol H. units J m-2) is the total radiant energy of all
Wavelengths passing from all directions through an infinitesimally small sphere of cross-sectional area dA, divided by dA It is given by the average fluence rate times the exposure tune in seconds. The term UV dose is often used in UV disinfection literature. It represents the UV exposure of a given organism in the germicidal range.

Spectral units means:

All of the terms for tight emission or incidence refer to all relevant wavelengths. One can define spectral derivatives for each of these terms. For example. the light power emission of a LIV lamp is often expressed as the spectral power (W nm^(-1), defined as the power output in a narrow wavelength band divided by the width of the band. The solar spectrum received at the Earth's surface is described in terms of the solar spectrum irradiance. Also the spectral distribution of a lamp emission is often given an a plot of spectral power versus wavelength.

Photon based units means:

Photochemistry involves the interaction of photons of light with molecules and means: the definitions units that are based on photons.

Photon irradiance, photon fluence rate and photon flow means:

Each of the spectral terms can be convened to a corresponding equivalent photon flow and fluence rate by dividing the term by the average photon energy in the narrow wavelength band.

Quantum yield means:

The quantum yield (unites) Q is a measure of the photon efficiency of a photochemical reaction. e is defined as the number of moles of product formed or reactant removed (P) per Einstein of photons absorbed

Line sources means:

When atoms are raised to an excited state, they emit only in very narrow lines with
Virtually no emission between the lines. The low-pressure mercury lamp is a very
Common lamp of this type. Table 3 gives the wavelengths and relative emittance for the emission lines of a low-pressure mercury vapor lamp.
Certain radiation units and associated light sources, (lasers) and lamps emit at longer wavelengths, This is the basis of the very popular fluorescent lamp.
For example, The emission lines of a mercury lamp are only sharp when the pressure of the gas is low (<10 tore). If the pressure is increased, the lamp can carry much more power, but the emission lines broaden. For the same length of lamp (about 120cm), a medium pressure lamp (pressure about 1000 tore) can carry up to 30,000W. These lamps are very common in commercial Systems utilizing ultraviolet light. Figure 5 shows a comparison of the emission of low pressure and medium pressure lamps in the ultraviolet region.

Excimer lamps means:

Excimer lamps arc unique in that they emit in a narrow band of wavelengths. An excimer is an atomic dimmer that is stable only in the excited state and dissociates on decaying to the ground state. Table 4 gives the wavelengths of sonic of the common excimer lamps.

Examples: Emission wavelengths (or some common excimer lamps

| Excimer | Wavelength (nm) | Excimer | Wavelength (nm) |
|---------|-----------------|---------|-----------------|
| Xe2     | 172             | XeCl    | 308             |
| KrCl    | 222             | I2      | 342             |
| Cl2     | 259             |         |                 |

Flash lamps

Flash lamps are similar to continuous wave (CW), but could also operate in (PW), pulsed mode operation, and are lamps that consist of a cylindrical quartz tube with electrodes at each end and filled with a ga.½ (e.g.. xenon). A power supply "fires' the lamps by discharging a large amount of electrical energy in a very short period of time (several us) by applying a very high voltage {10 - 30 kV}. The resulting plasma reaches temperatures of 10,000 - 13,000 K and the emission is essentially that of a blackbody (see Fig. 4). In commercial flash lamp Systems the lamp , a typical "flashed" about 30 times per s, but given a special electronic pulsing circuitry is added, repetition rates could reach Khz regime.

FEL means Free Electron Laser and its derivatives, wherein space charged technologies (such as the Electrostatically Accelerated Free Electron Laser) include an electronic pulsing circuits, charging, or an accelerator (Such as R.F. Linac) is involved in the production of photons (around 100, 000,000 photons per electrons, in contradistinction of a conventional crystal based laser having around 1 photon per electron, it is a laser which have less maintenance associated with its operation and its wall plug efficiency is reaching around the 40-51% respectively, and respectfully of the exact pumping geometry used.

The term EAFEL means Electrostaticely Accelerated Free Electron Laser and is an extremely efficient laser pumping geometry wherein recycling of the Accelerated electrons is being performed by utilizing deceleration techniques and its wall plug efficiency is estimated to reach in access of the 55% (amount of light being produced or converted from the electricity being consumed for its operation.

Biologically enhanced or photo-chemically polished drinking water or breathing air means any liquid or gas which have been passed through or processed by the method of the present invention such as water and/or air), more specifically such processes involved in the polishing and enhancing may include; Optical inactivation, disinfection, inactivation of DNA and/or RNA replication sequences, Photocatalysis, electro catalysis, a hybrid of photo and electrocatalysis, optical dissociation, physiological dissociation, biomass expansion, filtration (pre/post), physical separation and sorting, reactivation, activation, sonication, acoustics, electroacoustics, electro-optical treatment (by photons of light), transgressing, or transversing said liquids and gasses through a photonic band gaped wave guides having an aerobic, non toxic passage for light and liquids or gasses or combination together, synchronously and or separately.

Peak power means, the energy generated when squeezing (i.e. such as when pulsing) electromagnetic energy in short duration of time, for example: a pulse of a given average energy and power - lasting, or having a pulse width of around 1 second (1s) will generate several watts in peak power, a pulse lasting or having width of microseconds (ms) will generate peak powers reaching the kilo-watts scale, while a pulse lasting nano (ns) seconds will generate peak powers reaching into the hundreds of million of watts which is especially beneficial for purposes such as optical dissociation, optical inactivation, optical polishing, and optical secretion and spectroscopy for control and diagnostics, so in short the shorter the pulse duration the higher its respective peak power.

Multi-photon - absorption - processes means a processes which when harnessed could be very beneficial for the photochemistry involved in the processing according to the present invention, for example when 10mj of energy (250,000 photons) are projected into a liquid or gas, the time it takes this projection is very important, if these photons will be furnished over 1 second time domain, then it leaves sufficient time for the electrons in said liquid or gasses, to relax back to the relaxed state, but if we apply these photons in a time domain of 5 nano seconds, then we do not leave time for the electrons to relaxed and the processes is called Multiphoton absorption processes, this processes are non linear in nature and yield much higher quantum yield , or efficiencies, or speed of the reactivation, or a more efficient methodology for optical treatment, processing and polishing

A hybrid of light sources means plurality of light sources and wherein their total spectral emitence, or total spectral distribution, or their total irradiance will cause multiphotonic absorption processes by means of super imposing their time domain (example: 1 light source is slow = 1s pulse duration, and additional light sources are very fast =5ns laser for example), their total irradiance is great and beneficial for the processes of the method of the present invention to occur efficiently, further more, such hybridization could includes, lamps and lasers, lasers and flash lamps, or any combination of CW, or PW type of light source working together, in synchronisity, and or sequentially or link or resolved by time domain manipulation for maximizing photonic interaction in matter, especially beneficial for triggering th catalytic scentiliting elemental compound according to the present onvention.

Photo catalysis means: The use of energy of a photon of light to catalyze chemical reactions. More specifically, such reaction may include the decomposition of water into hydrogen, and oxygen, and the complete oxidation of organic contaminants in aqueous environments. More specifically, the first step in Photocatalysis is for the catalyst material to absorb photon of light in order to excite an electron from the velance band (VB), to the conduction band (CB), thus creating electron-hole-pairs. Each Species must then migrate to the surface before recombination occurs. If this condition is met, the electron can be transferred to a surface adsorbed molecule, reducing it. The overall process is illustrated , it is important to note that for the processes to occur efficiently (preventing pre-matured recombination of the said electron-hole pairs), the rates of reduction, and the oxidation must be comperable. The position of the band edges is critical for each step of the process, a photocatalyst material which is stable in water is tio2, (or known as Titanium Oxide).

Electrocatalysis, similar to that explained in photo-catalysis, but instead of photons, an electrical charge is used, through the use of semiconductor material which has been specially selected (its band gap) for the charge applied, for the context of the present invention an electrocatalysis, stable in water is I.T.O, or known in its chemical name and signature Indium Tinoxide). Further more, it is especially beneficial to combine, and operate both electro-catalysis, and photo-catalysis simultaneously, or serially, or sequentially or in unison, or each separate catalytic is triggered separately in order to maximize the collective efficiencies, thus harnessing and improving the performance of current and future catalytic technologcal evolution according to the methodology of the present invention.

<TBODY>ablation in the context of the present invention means the removal of material or tissue by melting, evaporation, or vaporization.

absorb in the context of the present invention means to transform radiant energy into a different form, usually with a resultant rise in temperature.

absorbance in the context of the present invention means the ability of a medium to absorb radiation depending on temperature and wavelength. expressed as the negative common logarithm of the transmittance.

absorption coefficient in the context of the present invention means the amount of radiant energy absorbed per unit or path-length.

active medium in the context of the present invention means a medium in which lasing will take place, rather than absorption, at a given wavelength.

afocal in the context of the present invention means literally, "without a focal length"; an optical system with its object and image point at infinity.

air-cooled laser in the context of the present invention means a laser using fans to force air over the laser tube and through the power supply. air-cooled lasers have the benefit of needing no water supply, although the fan noise can sometimes be a disadvantage. usually only small and medium power lasers are air-cooled. very small lasers, typically helium-neon, need no fans. although technically they are "air cooled" via convection, the term is usually applied only to fan-forced cooling.

amplification in the context of the present invention means the growth of the radiation field in the laser resonator cavity. as the light wave bounces back and forth between the cavity mirrors, it is amp stimulated emission on each pass through the active medium.

amplitude in the context of the present invention means the maximum value of the electromagnetic wave, measured from the mean to the extreme; put simply, the height of the wave.

angstrom unit in the context of the present invention means a unit of measurement for a wavelength of light (written å), equal to one ten billionth of a meter (10-10 meter). occasionally still used.

anode in the context of the present invention means an electrical element in laser excitation which attracts electrons from a cathode. an anode can be cooled directly by water or by radiation.

ar coatings in the context of the present invention means anti-reflection coatings, used on the backs of laser output mirrors to suppress unwanted multiple reflections which reduce power.

argon laser in the context of the present invention means a laser filled with argon gas. it gives off green and blue light. the strongest lines are at 514 nm (green) and 488 nm (blue). argons range from small 15 milliwatt 110 volt air-cooled models to large 50 watt 440 volt water-cooled systems. argon lasers are the most common type of light show lasers since they provide unable brightness at a reasonable cost.

average power in the context of the present invention means the sums of the energy of all single discrete pulses, in one second, of a pulsed laser.

autocollimator in the context of the present invention means a single instrument combining the functions of a telescope and a collimator to detect small angular displacements of a mirror by means of its own collimated light.

axial-flow laser in the context of the present invention means the simplest and most efficient of the gas lasers. an axial flow of gas is maintained through the tube to replace those gas molecules depleted by the electrical discharge used to excite the gas molecules to the lasing state.

axis, optical axis in the context of the present invention means the optical center-line for a lens system; the line passing through the centers of curvature of the optical surfaces of a lens.

beam diameter in the context of the present invention means the diameter of that portion of the beam which contains 86% of the output power.

beam expander in the context of the present invention means optical device increasing beam diameter and reducing divergence.

beam splitting in the context of the present invention means optically splitting a laser beam into two or more beams, of various or identical energys.

brewster windows in the context of the present invention means the transmissive end (or both ends) of the laser tube, made of transparent optical material and set at brewster's angle in gas lasers to achieve zero reflective loss of vertically polarized light. non-standard on industrial lasers, but a must if polarization is desired.

brightness in the context of the present invention means the visual sensation of the luminous power of a light beam, as opposed to scientifically measured power of the beam.

calorimeter in the context of the present invention means an instrument which measures the heat generated by absorption of the laser beam—another way to measure laser power.

cathode in the context of the present invention means the element providing the electrons for the electrical discharge used to excite the lasing medium.

co2 laser in the context of the present invention means a laser largely used in industry in which the primary lasing medium is carbon dioxide.

coaxial gas in the context of the present invention means most laser welding is done with a shield of inert gas flowing over the work surface to prevent plasma oxidation and absorption, to blow away debris, and to control heat reaction. the gas jet has the same axis as the beam so the two can be aimed together.

coherent light, coherent radiation in the context of the present invention means radiation composed of wave trains vibrating in phase with each other. coherent light waves all travel the same direction (spatial coherence) at the same frequency and in phase (temporal coherence). a laser produces coherent light, conventional light sources produce incoherent light.

collimated light in the context of the present invention means beam light rays traveling parallel to each other.

collimation in the context of the present invention means the process by which divergent rays are converted into parallel rays.

convergence in the context of the present invention means the bending of light rays toward each other, as by a positive (convex) lens.

current saturation in the context of the present invention means maximum flow of electric force in a conductor; in a laser, the point at which further electrical charge will not increase lasing action.

cw in the context of the present invention means an abbreviation of continuous wave of a laser as opposed to pulsed operation.

depth of field in the context of the present invention means the working range of the beam, a function of wavelength, diameter of the unfocused beam, and focal length of the lens. to achieve a small diameter spot size, and thus a high power density, a short depth of field must be accepted.

Water laser in the context of the present invention means the creation of lasing in the water (i.e. while flowing), thus harnessing the flowing liquid wave guide for the formation of a flowing light guiding cavity whicg could be utilized to create a water laser.

dichroic filters and mirrors in the context of the present invention means a piece of glass with an optical thin-film coating that transmits certain colours (wavelengths), and reflects the remaining colours. dichroic filters are used to combine or eliminate specific colours as needed in a laser projector. dichroic mirrors are used to maximise the amount of light reflected from a laser of a particular wavelength.

dichroics should be handled with care to prevent damage to the coating. [see also: colour box]

diode laser in the context of the present invention means a semiconductor similar to an led (light-emitting diode) but which produces coherent light. diode lasers are small and efficient, which has led to their use in compact disc players and pen-type laser pointers. currently, diode lasers are too dim or expensive for most light show uses. this is likely to change over the next few years. [see also: solid-state laser]

divergence in the context of the present invention means the angle at which the laser beam spreads in the far field; the bending of rays away from each other, as by a concave lens or convex mirror.

drift, angular in the context of the present invention means all undesirable variations in output (either amplitude or frequency); angular drift of the beam, measured in milliradians before, during, and after warm-up.

duty cycle in the context of the present invention means the length of time the laser beam is actually cutting, drilling, welding, or heat-treating, as compared to the entire work cycle time.

electromagnetic wave in the context of the present invention means a disturbance which propagates outward from an electric charge which oscillates or is accelerated. includes radio waves; x-rays; gamma rays; and infrared, ultraviolet, and visible light.

emissivity, emittance in the context of the present invention means rate at which emission takes place; the ratio of the radiant energy emitted by a source or surface to that emitted by a blackbody at the same temperature.

exposure in the context of the present invention means a measure of the total radiant energy incident on a surface per unit area; radiant exposure.

far-field imaging in the context of the present invention means an imaging technique with solid-state lasers that has several limitations: non-uniform energy distribution, very short working distances, and poor control of hole geometry.

fiber optic cable in the context of the present invention means flexible glass or plastic strands made into a cable, used to carry light from one place to another. there are two main types; step index and graded index fiber. within these two main types there are two further subgroups:

transmission fibers carry the beam with as little loss as possible. they are used to transmit the laser's light to remotely located projection devices.

display fibers [also knows as side glow fibers] have no cable jacket, so some light scatters out the side of the strands. the strands themselves look like microscopic neon tubing and become a special effect, such as a laser-lit "whip" or a glowing "rope" wrapped around objects.

flashlamp in the context of the present invention means source of powerful light; often in the form of a helical coil and used to excite photon emission in a solid-state laser.

fluorescence in the context of the present invention means the glow induced in a material when bombarded by light. brewster windows of fused silica fluoresce in uv light, increasing absorption of laser radiation and degrading laser mode and output.

flux in the context of the present invention means the radiant, or luminous, power of a light beam; the time rate of the flow of radiant energy across a given surface.

focus in the context of the present invention means noun: the point where rays of light meet which have been converged by a lens. verb: to adjust focal length for the clearest image.

focal point in the context of the present invention means (same as first definition under "focus;" in laser work,) the focal point of the beam relative to the work surface has a critical effect, such as the depth and shape of drilled holes. when the focal point is at the surface, holes are of uniform diameter. when the focus is below the surface, conical holes are drilled.

folded resonator in the context of the present invention means construction in which the interior optical path is bent by mirrors mounted on corner blocks bolted into pre-aligned position, permitting compact packaging of a long laser cavity.

frequency in the context of the present invention means the number of light waves passing a fixed point unit of time, or the number of complete vibrations in that period of time.

gain in the context of the present invention means another term for amplification, usually referring to the efficiency of a lasing medium in attaining a population inversion. high gain is typically more than 50% per pass of the light wave between cavity mirrors.

gas discharge laser in the context of the present invention means a laser containing a gaseous lasing medium in a glass tube in which a constant flow of gas replenishes the molecules depleted by the electricity or chemicals used for excitation. the discharged gas can be filtered and 90% recycled for economy.

gas jet assist in the context of the present invention means an assisting coaxial gas, such as oxygen, argon, or nitrogen, which may be used to achieve very high power levels for cutting certain metals.

gas transport in the context of the present invention means a laser design, which generates very high beam power within a fairly small resonator structure. long electrodes parallel the axis and gas is circulated across the resonator cavity.

gaussian in the context of the present invention means the "normal curve," or normal distribution, an example of which is the symmetrical bell shape of the holes created by the uncorrected, unfocused laser beam in its optimum mode. a gaussian laser beam has most of its energy in the center.

haz in the context of the present invention means heat-affected zone, or the area where laser beam and metal (or other) surface are in contact.

helium-neon laser in the context of the present invention means ("hene"), laser in which the active medium is a mixture of helium and neon, which is in the visible range. used widely in industry for alignment, recording, printing, and measuring, it is also valuable as a pointer or aligner of invisible co2 laser light.

heat sink in the context of the present invention means a substance or device used to dissipate or absorb unwanted heat, as from a manufacturing process (or, with lasers, from reflected rays).

hertz in the context of the present invention means the approved international term, abbreviated hz, which replaces cps for cycles per second.

image in the context of the present invention means the optical reproduction of an object, produced by a lens or mirror. a typical positive lens converges rays to form a "real" image which can be photographed. a negative lens spreads rays to form a "virtual" image which can't be projected.

incident light in the context of the present invention means a ray of light that falls on the surface of a lens — or any other object. the "angle of incidence" is the angle made by the ray with a perpendicular to the surface.

intensity in the context of the present invention means the magnitude of radiant energy (light) per unit, such as time or reflecting surface.

ion laser in the context of the present invention means a type of laser employing a very high discharge current, passing down a small bore to ionize a noble gas such as argon or krypton. the ionization process creates a population inversion for lasing to occur. a research laser useful for some industrial applications.

ionization in the context of the present invention means the process by which ions are formed.

irradiation in the context of the present invention means exposure to radiant energy, such as heat, x-rays, or light; the product of irradiance and time.

joule in the context of the present invention means one watt per second; a measurement frequently given for laser output in pulsed operation.

krypton laser in the context of the present invention means a laser filled primarily with krypton gas. when used with "all-line" or "white" optics, it produces red, yellow, green and blue light. a "red-only" krypton laser uses with specially tailored optics to output a very strong red line at 647 nm. kryptons are similar to argons (the same tube design can be used for both) however, krypton gas produce less light (output power) than an equivalent volume of argon gas. krypton lasers are primarily used when a powerful red light is needed.

laser in the context of the present invention means "laser" is an acronym derived from "light amplification by stimulated emission of radiation".
a device which produces a coherent beam of light. the beam remains parallel for long distances and contains one or more extremely pure colours. light show lasers are usually gas-filled tubes using high voltage current to ionise the gas (cause the gas to glow). mirrors at each end of the tube help amplify a process called "stimulated emission". most of the stimulated emission light travels between the two mirrors; between 1% and 4% comes out of one of the mirrors to create the beam of light that we see. the gas used determines the colour (or colours) of the beam. gas lasers remain the overwhelming choice for display applications. the four main types used are a helium-neon mixture, argon, krypton, and an argon-krypton "mixed gas" mixture.

aser oscillation in the context of the present invention means the buildup of the coherent wave between laser cavity end mirrors. in cw mode, the wave bounding back and forth between mirrors transmits a fraction of its energy on each trip; in pulsed operation, emission happens instantaneously.

laser rod in the context of the present invention means a solid-state, rod-shaped lasing medium in which ion excitation is caused by a source of intense light, such as a flashlamp. various materials are used for the rod, the earliest of which was synthetic ruby crystal.

light in the context of the present invention means the range of visable electromagnetic radiation frequencies detected by the eye, or the wavelength range from about 400 to 750 nanometers. it is sometimes extended to include photovoltaic effects and radiation beyond visible limits.

light regulation in the context of the present invention means a form of power regulation in which output power is maintained at a constant level by controlling discharge current.

luminance in the context of the present invention means commonly called illumination; the luminous or visible flux per unit area on a receiving surface at any given point.

meniscus lens in the context of the present invention means the lens used primarily in co2 lasers by coherent, inc. it has one side convex, the other concave.

metastable, metastable state in the context of the present invention means unstable condition in which the energy of a molecule is at some discrete level above the lowest, or ground state. it is this condition which is necessary for emission of photons in a laser. (from quantum theory.)

millijoule: one thousandth of a joule.

milliwatt in the context of the present invention means one thousand milliwatts equal one watt. small lasers' beam powers are measured in milliwatts. for example, a 50 mw laser is one-twentieth of a watt; a 500 mw laser is one-half watt.

mode in the context of the present invention means a particular functioning arrangement, setup, or condition for laser operation, such as continuous emission, pulses, or grouped pulses. "mode" also describes the cross-sectional shape of the beam. (see "tem.")

modulation in the context of the present invention means the ability to superimpose an external signal on the output beam of the laser as a control.

monochromatic light in the context of the present invention means theoretically, light consisting of just one wavelength. since no light is completely monochromatic, it usually consists of a very narrow band of wavelengths. lasers provide the narrowest bands.

nanometer in the context of the present invention means a unit of length in the international system of units (si) equal to one billionth of a meter (10-9 meter). once called a millimicron, it is used to represent wavelength. abbreviated "nm."

near field imaging in the context of the present invention means a solid-state laser imaging technique offering control of spot size and hole geometry, adjustable working distance, uniform energy distribution, and easily produced range of spot sizes.

nd:glass laser in the context of the present invention means a solid-state laser of neodymium:glass offering high power or short pulses, or both, for specific industrial applications.

nd:yag laser in the context of the present invention means a solid-state laser of neodymium:yttrium-aluminum garnet, similar to the nd:glass laser. both are pumped by flash lamps, or diode lasers.

nema in the context of the present invention means national electrical manufacturers' association, a group which defines and recommends safety standards for electrical equipment.

noise in the context of the present invention means unwanted, minor currents or voltages in an electrical system.

object in the context of the present invention means the subject matter or figure imaged by, or seen through, an optical system.

optical density in the context of the present invention means protection factor provided by a filter (such as used in eyewear, viewing windows, etc.) at a specific wavelength. each unit of od represents a 10x increase in protection.

optical pumping in the context of the present invention means exciting the lasing medium by the application of light rather than electrical discharge from anode and cathode.

output coupler in the context of the present invention means the resonator mirror which transmits light; the one at the opposite end is totally reflective.

output power in the context of the present invention means the energy per second emitted from the laser in the form of coherent light, usually measured in watts for continuous-wave operation and joules for pulsed operation.

peek power in the context of the present invention means the power of an individual pulse in a pulsed laser. it is obtained by dividing the pulse energy in joules to the pulse width in seconds. typical values can be reach mega and giga watts.

photoacoustic effects in the context of the present invention means arises with the use of very short-duration high-energy laser pulses, at pulse durations typically below 10 microseconds. significant amounts of energy are absorbed and a rapid expansion occurs in the tissue, generating an acoustic shock wave that causes mechanical disruption to cellular structures.

photochemical effects in the context of the present invention means effects that occur from long exposure durations at incident power levels insufficient to cause damaging photothermal effects. it is an energy dependent process (a function of the total quantity of radiation absorbed rather than its rate of absorption).

photometer in the context of the present invention means an instrument which measures luminous intensity.

photon in the context of the present invention means in quantum theory, the elemental unit of light, having both wave and particle behavior. it has motion, but no mass or charge.

photothermal effects in the context of the present invention means the damage mechanism for acute laser injury (i.e. for injury immediately following exposure). the radiation incident at the surface is absorbed in the underlying tissue, increasing the temperature of the tissue to the level at which damage can occur, and laser burns result. it is a power dependent process (a function of the rate at which energy is absorbed rather than the total quantity of energy involved).

plasma in the context of the present invention means in laser welding, a metal vapor that forms above the spot where the beam reacts with the metal surface, also used to describe the laser tube (plasma tube, discharge tube) which contains the completely ionized gas in certain lasers.

polarization in the context of the present invention means restriction of the vibrations of the electromagnetic field to a single plane, rather than the innumerable planes rotating about the vector axis. this prevents optical losses at interfaces between the lasing medium and optical elements. various forms of polarization include random, linear (plane), vertical, horizontal, elliptical, and circular. of two polarization components (so-called), s and p, the p component has zero losses at brewster's angle.

population inversion in the context of the present invention means when more molecules (atoms, ions) in a laser are in a metastable state than in the ground state (a situation needed for sustaining a high rate of stimulated emissions), a "population inversion" is said to exist. without a population inversion, there can be no lasing action.

power density: the amount of radiant power concentrated on a surface. units watts per square meters or square centimeters

pulse energy in the context of the present invention means the energy of a single, brief emission from a laser programmed for pulsed behavior rather than continuous operation. pulse power can be several times greater than cw emission.

pulse tail in the context of the present invention means pulse decay time, which can be shortened (by using a special mixture of gases) to allow for fast repetition of laser pulses within a given length of time.

q-switch in the context of the present invention means a device that has the effect of a shutter moving rapidly in and out of the beam to "spoil" the resonator's normal q, keeping it low to prevent lasing action until a high level of energy is stored. result: a giant pulse of power when normal q is restored.

quasi cw in the context of the present invention means the pulsating of a continious light into pulsed light by acusto optic, electrooptic, electronic, or mechano optical means, so peak powers are reduced, by number of pulses (see rep. rate) are increased.

radiance in the context of the present invention means brightness; the radiant energy per unit solid angle and per unit projected area of a radiating surface.

radiant energy in the context of the present invention means energy traveling as wave motion; specifically, the energy of electromagnetic waves (light, x-rays, radio, gamma rays).

radiant flux: the rate of emission or transmission of radiant energy.

radiant intensity in the context of the present invention means radiant power, or flux, expressed as emission per unit solid angle about the direction of the light in a given length of time.

radiant power in the context of the present invention means the amount of radiant energy available per unit; the radiant flux.

reflectance in the context of the present invention means the ratio of the reflected flux to the incident flux, or the ratio of reflected light to light falling on the object.

reflection in the context of the present invention means the return of radiant energy (incident light) by a surface, with no change in wavelength.

refraction in the context of the present invention means the change of direction of propagation of any wave, such as an electromagnetic wave, when it passes from one medium to another in which the wave velocity is different. simply put, the bending of incident rays as they pass from one medium to another, such as air to water.

resolution in the context of the present invention means resolving power, or the quantitative measure of the ability of an optical instrument to produce separable images of different points on an object; the capability of making distinguishable the individual parts of an object, closely adjacent images, or sources of light.

resonator in the context of the present invention means the mirrors (or reflectors) making up the laser cavity containing the laser rod or tube. the mirrors reflect light back and forth to build up amplification under an external stimulus. emission is through one of them, called a coupler, which is partially transmissive.

rockwell c in the context of the present invention means a scale or test used to define hardness in metals, particularly steel and titanium.

solid-state laser in the context of the present invention means a laser where the lasing medium is a solid material such as a ruby rod. these can be optically pumped by a flashlamp or diodes. solid state lasers also include diode lasers as they use electrically pumped solids to produce light. currently, solid-state lasers are too expensive for most light show uses. this may change over the next few years. the most promising solid-state laser uses a material called nd:yag, which produces infrared light. this can be frequency doubled (second harmonic generation) to produce up to 60 watts of green light at 532 nm. the green light can again frequency doubled (fourth harmonic generation) to produce uv light at 266 nm, up to several watts.

spectral response in the context of the present invention means the response of a device or material to monochromatic light as a function of wavelength.

stimulated emission in the context of the present invention means when an atom, ion, or molecule capable of lasing is excited to a higher energy level by an electric charge or other means, it will spontaneously emit a photon as it decays to the normal ground state. if that photon passes near another atom of the same frequency which is also at some metastable energy level, the second atom will be stimulated to emit a photon. both photons will be of the same wavelength, phase, and spatial coherence. light amplified in this manner is intense, coherent (collimated or parallel), and monochromatic. in short, laser light.

tem in the context of the present invention means abbreviation for transverse electromagnetic mode, the cross-sectional shape of the working laser beam. an infinite number of shapes can be produced, but only a relatively small number are needed for industrial applications. in general, "the higher the tem, the coarser the focusing."

- tem00: a gaussian-curve mode that is the best collimated and produces the smallest spot of high power density for drilling, welding and cutting.

- tem01: divided into two equal beams for special applications.

threshold in the context of the present invention means during excitation of the laser medium, this is the point where lasing begins.

transmission in the context of the present invention means in optics, the passage of radiant energy (light) through a medium.

transmittance in the context of the present invention means the ratio of transmitted radiant energy to incident radiant energy, or the fraction of light that passes through a medium.

vignetting in the context of the present invention means the loss of light through an optical element when the entire bundle does not pass through; an image or picture that shades off gradually into the background.

visible light transmission/transmittance in the context of the present invention means the amount of visible light usable to the eye that passes through a filter. as a rule of thumb, as optical density increases, visible light transmission decreases — but not always.

watt in the context of the present invention means an objective measure of power; in lasers, usually refers to the optical output power, or strength, of a laser beam. watts are also used in a more conventional sense, to measure electrical power used by a laser. for example, a 10 w (optical) argon laser consumes around 10,000 w of electrical power.

wave in the context of the present invention means an undulation or vibration, a form of movement by which all radiant energy of the electromagnetic spectrum is thought to travel.

wavelength in the context of the present invention means the fundamental property of light—the length of the light wave, which determines its color. common units of measurement (which is usually from crest to crest) are the micron, the nanometer, and (earlier) the angstrom. visible light has wavelengths extending from about 700 nanometers (red) through orange (~600 nm), yellow (~580 nm), green (~550 nm), blue (~450 nm) and violet (~400 nm).

white-light beam in the context of the present invention means broadly, a laser beam which contains a number of different wavelengths (colours) so the beam appears white. if the beam is passed through a prism or diffraction grating, it is separated into individual laser beams, each of a single specific wavelength.

more specifically, a white-light beam ideally contains twice as much red as green and blue light for correct colour balance (see appendix). it can be from a single white-light laser or from two or three lasers whose beams have been combined into a single beam. white-light beams are primarily used in rgb laser projectors. see the definition of white-light laser for more information on what constitutes an "equal mixture" of light.

white-light laser In the context of the present invention means Many lasers can produce a number of wavelengths (colours) simultaneously. A white-light laser is designed to give a good balance of red, green and blue wavelengths. Usually the laser is intended for an RGB laser projector. (Some models also deliberately add yellow light for specialised 4-colour projectors.) Most white-light lasers use an anrgon/krypton gas mixture. It is somewhat

difficult to produce an equal balance of desired colours, and to keep this balance consistent during the lifetime of the laser tube. At present, there are no standards defining the exact wavelengths and colour proportions for a laser to be called"white-light".

In addition, the sought-after colour balance can be defined either as equal amounts on a photometer, or as visually equal amounts. Since the eye is much more sensitive to green, a visually equal or "photopically balanced" laser has roughly five times more power in red and blue than in green. Most white-light lasers today are not photopically balanced.

window In the context of the present invention means A piece of glass with plane parallel sides which admits light into or through an optical system and excludes dirt and moisture.

DETAILED DESCRIPTION OF THE INVENTION

It is appreciated that the detailed description that follows is intended only to illustrate certain preferred embodiments of the present invention. It is in no way intended to limit the scope of the invention, as set out in the claims.

Problems that exist in prior art methods for disinfecting products (solids, liquids or gasses) after they have been packaged are as follows: Often the wavelength used does not allow effective transmission through the packaging material. The presence of the packaging thus requires use of higher levels of energy, which then tend to be absorbed by the packaging material, which then releases toxic molecules into the liquid or gas within. As an example, the majority of the optical output of a conventional mercury UV light is at a wavelength of 254.3nm. This wavelength cannot penetrate PET, the major polymer currently used in the packaging industry.

The inventors of the present invention have noted that PET absorbs almost 100% of energy having a wavelength shorter than 312nm, however PET transmits close to 90% of light energy when the wavelength is 355nm. The present invention proposes using a short sub-microsecond pulse width at 355nm to grant effective disinfection with an appropriate dose of energy. Thus brief, high energy pulses, at a novel wavelength, can be used to penetrate and sterilize packaging materials which were previously thought to be impenetrable using sterilizing light-energy sources. Brief pulses are used to prevent heat build-up, which would result in damage to the packaging and migration of harmful molecules from the packaging to within the contents of the packaged material. The method of the present invention can be applied to many packaging materials (described below), such as materials used for packaging water and water-based products.

The methodology according to the method of the present invention discloses a simple creative and step to form high energy density zones which disinfect. These high energy zones are preferably created using sub-microsecond pulses of UV laser (though other light sources can be used as well). The high energy pulses are hydro-optically distributed uniformly and spatially equalized to deliver the appropriate bio dosimetric energy threshold for inactivation through a predetermined space or within a predetermined volume over a predetermined period of time. The method of the present invention facilitates disinfection by reduction, oxygenation, photocatalysis, photo-electro-catalysis, sterilization, photolysis, dissociation, advanced Oxidation Technology (AOT), photo-excitation or equalizing or controlling the concentration of biological or chemical contaminants. The method can be used to reduce toxic organic compounds and to inactivate bacteria, cysts, germs, spores, viruses, pathogens, moulds and many other noxious species, which threaten human health.

The method of the present invention proposes using a pulsed light source, selected from: lasers having sub-microsecond pulse width time duration (such as a UVA laser), a continuous wave type of light source, or a hybrid combination platform thereof.

According to a preferred embodiment of the present invention, a high repetition rate, high peak power laser of the Nd:Yag, or Nd:Glass, or Nd: YLF, type or any combination thereof is used; driving the uvjet, or a spatially processing pulsed laser light therein, or throughout. The laser can operate in the Fourth Harmonic generation mode (i.e. FHG), or in another embodiment, the solid state (e.g. Nd: Yag type) laser can operate in the Third Harmonic Generation mode (i.e. THG). Additionally, in another embodiment, an electrical discharge laser such as an excimer laser, is used. In such case, the wavelength should be from about 193nm to 308nm, or 351nm. In another embodiment, a hybrid laser system is used for driving the uvjet, thus operating wherein (a) a solid state laser operating at THG (355nm), is attached, or aligned to the transparent area of the product undergoing sterilization. An electrical discharge laser is also operating together in the hybrid format, to maximize the efficiency of the disinfection. In other embodiments the laser light source is selected from (a) Gas discharge laser, (b) diode pumped lasers, (c) plasma discharged lasers, (d) solid state lasers, (e) semi conductor lasers, (f) crystal type of lasers, (g), X-rays pumped lasers, (h) E-beam pumped gas lasers types, (I) FEL (Free Electron Laser amplifier), (j) EA/FEL (Electrostatically Accelerated Free Electron Laser), or organic laser types or any combination thereof.

In another preferred embodiment, the packaging acts as a waveguide, or a concentrator.

Preferably, the laser light source is tunable from about 1nm to about 3000nm, most preferably from about 333nm to about 360nm. Preferably the peak power density of individual pulses reaches from about 1 nJ/Cm2, to about 50Js/Cm2, and preferably the pulsed laser emits pulses at repetition rates from about 1 Hz to about 300MHz. Thus the preferred embodiment of the present invention is suitable for a wide range of applications involving different packaging materials. The species- specific optical calibration standards can be calculated (to sterilize against a specific species of pathogen), to give a specific biodosimetric value or curve which corresponds to (is lower than) the damage threshold of the packaging material used.

The light source is harmonized so as to fit the action spectrum (i.e. absorption, transmittance, reflectance or optical properties of packaging) of the packaging material used at specific manufacturing sites. The present invention utilizes 2nd order interactions, and multiphoton absorption processes harnessed and hydro-optically distributed uniformly. A refractive index profile vortex diffuser is created to deliver the required biodosimetric curve or threshold in line with the specific inactivation objective of a given noxious species or microorganism.

The method of the present invention is especially beneficial for sterilization of water-based products, flavored water, mineral and spring water, bottled water and treated water products to be treated in their packaging, oxygenated water, supplemented water, magnetized water, enhanced water, reconstructed water, or any combination thereof. Also useful for beverages, wine, juice, or alcohol. In addition, a wide variety of bio-medical applications include blood and blood products, medical preparations (e.g. insulin products, blood products, plasma products), and liquids and gases used for medical procedures. Also useful for drug delivery using water based, and/or expanded or flavored water drinks containing vitamins or nutrients; for air products, gases for propelling medications, for sprays, or any solids, liquid or gasses or hybrid combination thereof. In all applications of the present invention, calibration must be performed for the action-spectrum, the absorption, transmittance, transparency, refractive index, or refractive index profiles of the packaging, (and the packaged liquid or gas interface) with the laser wavelength, energy, pulse width, and spectral and spatial characteristics. This will guarantee effective and economical disinfection and sterilization through packaging occurs. The present invention allows high flow rates to be maintained, and does not effect sensitive natural or wholesome components in the product (such as sugar or natural flavorings in beverages). The method does not alter the taste and flavor of products treated, and does not cause sensory effects.

An additional application is for surface treatment to improve the hygiene of the mouth. Preferably, this includes: (a) harnessing the illumination or irradiation of a waveguiding dielectric brush [WDB] having a delivery capacity from

**EP 1 334 031 B1**

about one quarter of a million of photons per Cm2/Second, to about 999 trillion photons per Cm2. This light source is applied to the mouth for 1 picosecond, or 1 Femtosecond, or 1 Atosecond. This swiftly sterilizes the complexly curved inner surfaces. Optionally, a new generation tooth paste is applied prior to the treatment to control the depth of penetration of the disaffection. The toothpaste comprises one of the following as an active ingredient: a Catalytic scintillated compound [CCC], a multi-component compound yielding Oxygen Charge (SYOCH) in a U.P.W, pH stabilized, held temporarily or permanently in a 3D polymeric frame work of biodegradable biocompatible carbomer or BI-polymers expanded to contain photo-catalytic, and/or scintillating conversion elements, each having predetermined electron charge transfer co.-efficiencies and absorption, refractive index profile, and acoustic properties, selected for their quantum objective efficiencies (from super-conductive, to dielectric, or semi conducting), wherein the flexibility of water may provide generic structurally yielding Oxygen Charge, accommodating into manageable forms the decomposed species inactivated radically (SYOCH1), within water, liquid, gas or air suspension, bodily fluids, or inside the mouth.

In another medical application, a light source is used to penetrate the surface of the skin in human, and animals and swiftly inactivate DNA & RNA sequences in blood therein (in the blood, i.e. under the skin, non invasively). Preferably a Nd:Yag laser is used, having a third Harmonic generator producing a 355nm wavelength. One or more laser units can be used, which have a beam of pulsed UVA light split, and distributed to a plurality of locations, waveguided through HGFS optical fibers, photonic band gap waveguides, or polymer waveguides, or hybrid combinations. The tip of each of these waveguides is attached, supported, or threaded, in proximity to areas in the body where blood vessels are relatively exposed, and are conveniently positioned around the surface (i.e. improving transmission, and coupling conditions). A further embodiment of the present invention is wherein several lasers are used, each having high repetition rates, or high peak powers, and pulse durations from about 1 ms, to about 1 fs, or Atosecond. Their respective beams are collected, deflected, diverted or stirred to form a three dimension element of UVA light at high energy density. This high energy density is below the damage threshold of blood components, nutrients, conditioners, compliment systems, MHC type 1,2,3, and any vital cell present in blood or blood products, yet they are efficient for inactivation of noxious species therein, i.e. within the blood, or blood products.

The method of the present invention is also beneficial for disinfection and inactivation of DNA and RNA sequences (i.e. disinfection) on articles having problematic surfaces, such as surfaces having complex curvature of the packaging materials. Other problematic surfaces are caps, lids, corks, jugs, bottles, and other conduits or chamber type packaging. Preferably, packaging bottles to be sterilized according to the present invention include transparent caps, lids, bottles, top/bottom or sides in order to facilitate optical delivery of the appropriate dose of energy (light) throughput the volume of the packaged product therein. The methodology of the present invention can nevertheless overcome limitations imposed by standard techniques of manufacturing packagings, and can creatively penetrate and diffuse light in packages having various types of geometry. The packaging can contain liquids, gases, solids, or a combination, to be disinfected, or the packaging may be sterilized according to the present invention, in a separate sterilization step than that performed on the contents.

The methodology according to the present invention discloses the technological know-how to safely penetrate and sterilize by light, packaging materials selected from: PET, Polyolefin, Polyamide, Polycarbonate, Polyeteramid, Polyester or PE/HD or any polymer resin combination thereof. Further more, the method of the present invention can also penetrate effectively and economically glass bottles, and a wide variety of additional polymers used for packaging water, liquids and gases and agro-food products.

A further preferred mode of operation for devices or instruments using the method of the present invention for use in a production line or filling line or a packaging line for bottled water, would be as follows. The production lines are synchronized or resolved to a central workstation networking interface, and include at least one radiation unit. The radiation unit has at least one pulsed laser unit with a high intensity source of light, able to emit from about 0.1 mJ Cm2 to about one thousand Joules Cm2. This predetermined energy emission is angled to spatially or dimensionally transcend the surface of the packaging, penetrate it and become diffused uniformly within it. The energy emitted must be calibrated so that it is sufficient to be distributed through the inner volume of liquids or gases within the packaging or bottles. The method proposes synchronization of the optronic, mechanical, hydraulic, and optic and electro-optics platforms (i.e. lasers and light sources, mechanical transport, and ultrasound angular sources) to maximize the efficiency of a device according to the present invention. This will ensure that high flow rates in commercial installation be accommodated. In a preferred mode of operation, a hybrid platform made of a plurality of light sources, is used. In such case, each of the light sources is synchronized to its counterpart in an interactive network so as to increase energy utilization, flexibility of design and interconnectivity and interoperability resolution, to maximize the efficiency of the disinfectionprocess.

Preferably, the volume of the packaging undergoing sterilization is about 10ml to 1 cubic meter. The packaging material or polymers need have an adequate action spectrum, absorption, transmission, refractive index, or transparency so that the high intensity source of light, will penetrate the packaging. As mentioned above, the wavelength and frequency of light appropriate for penetration of the most common types of packaging polymers, is from about 100nm to 400nm; most preferably is at 355nm.

In view of the possible refractive index profiles of the products undergoing sterilization, and that of their packaging, optical manipulation or beam management need be selected. Any of the following beam management techniques can be used: diffusing, scattering, reflecting, refracting, focusing, defocusing, diffracting, stirring, collimating, splitting or combining, confining, super-imposing, interfering or alternating, zooming, or pointing amplifying, directing or targeting beams of light from said radiation unit throughout the packaging and throughout the content of the packaging. Preferably, a biodosimetric curve is drawn according to species-specific calibration standards such that a particular biological pathogen or a chemically toxic species (organic compound) will be reduced, oxidized, or photolyzed, or disinfected or sterilized by Disinfecton Through Packaging [DTP]. The methodology of the present invention may utilize optical fibers or wave guides selected from solid state guides, photonic band gap trajectories, closed non toxic, non aerobic liquid waveguides, or aerobic flowing liquid waveguides to deliver the light from the high intensity light source to the boundaries of the packaged product (or to the packaging alone). This facilitates network distribution of pulsed UVA light to a plurality of exposed packaging surfaces which may have angular positioning. Thus appropriate biodosimetric curves need be established according to the method of the present invention for particular noxious biological or chemical (organic) species of concern. The methodology of the present invention may us hydraulic, mechanic, pneumatic, or magnetic, acoustic, super sonic, or ultrasonic means in order to mix, stir, move or position the product before or during sterilization to achieve optimal delivery and dispersal of the light energy to all areas of the product.

Preferably, energy dosimetric values are calculated for devices used, by ignoring the overall number of photons delivered. Rather, what is relevant is the actual number of photons delivered within a predetermined space over a predetermined period of time, opening interactive non linear 2nd and 3rd order interactions.

Preferably, a solid state laser is used, wherein frequency doubling and harmonic conversion have been performed efficiently in order to achieve the desired wavelength of 355nm. In devices operated using the methodology of the present invention, the time domain, pulse width, pulse intensity, pulse repetition rates or pulsed laser light energy parameters can be amplified, compressed, expanded, modulated or coded, or recorded for later reply as sequential data strings, recurrent data strings, in presets, or as a real time non recurrent operation. Preferably, the pulse duration (or pulse width) of the pulsed UVA laser is between about 1 microsecond to several femtoseconds (fs), or from about 7nano seconds (ns) to about 11 Pico-seconds (Ps). Said time domain can be adjusted or calibrated to species- specific calibration standards, according to inactivation or disinfection objectives or quality standards.

The optical energy necessary for disinfection must be distributed to reach the entire contents of the packaging, after the packaging material has being penetrated. The inventors propose utilizing the refractive index profile variation created, to guide and diffuse light uniformly throughout the entire volume of the packaging (i.e. the liquid or gas inside the packaging) to be disinfected. The dispersion technique hereby disclosed, involves creating a vortex in a packaged liquid product, then directing the light beam towards the air present in the center of the vortex (parallel to the vortex center). For example: The water inside a PET or polymeric packaged type mineral water bottle or jug has a refraction index of 1.3, [N1]. The air capsule at the top of the bottle has a refractive index of 1.00, [N2] and the refractive index of the polymeric packaging material is around 1.45 [N3]. Thus creative refractive index sculpturing (N1/N2/N3) is made possible using the methodology of the present invention. The refractive index profiles thus created facilitate the uniform distribution of light therein (inside the packaging). More specifically, the refractive index profile of the air/water interface in a typical mineral water bottle is thus N1=Water, N2=Air, N3=PET. The volume of the water in the bottle is spun at an appropriate speed to create a vortex inside the bottle (between about 18- 1800 RPM). The highest refractive index value is then external to the path-length or direction of light entering the bottle from the outside, and the lower refractive index value (air at 1.00) is at the center of the bottle, forming an efficient diffuser. (This is reminiscent of an optical fiber refractive index profile, however taken inside-out). This facilitates uniform distribution of the laser pulsed UVA light throughout the entirety of the volume and facilitate calibration of the dosimetric values over wide range of energies (for example 1 big pulse for transparent water, and many micro-pulses for flavored water having lower transparency). Many thresholds thus could be created according to the method of the present invention to fit requirement of producers, and end users proficiently.

The vortex within the liquid is formed by applying acoustic energy at a predetermined angle, to stir the liquid therein, without damaging the structure of the polymer packaging from without. This hydro-optical aspect is original in the sense that no other method of photo-sterilization utilizes the air capsule normally present at the top of the bottle. This air capsule also tends to contain 21% of free available oxygen, which suggests pathways for interactive photochemistry therein (in the bottle conduit or chamber packaging interface). Utilization of a vortex to distribute the light energy allows reduction of the power and energy required to reach and disinfect the entire package.

Flash lamps of different types have been produced to try and mimic the intensity effects achieved with the methodology of the present invention, but these trials have failed as a result of the relatively long time duration it takes to ignite and spark or pump the mercury. More specifically, conventional pulsed mercury based flash-lamps cannot offer disinfection at sub-microseconds time domain or pulse width. In effect, often the shortest time duration, or pulse width, achieved with a conventional flashlamp is 10-30 microseconds, thus still remaining within the 1st order interaction of CW type of lamps. The only flash-lamps reaching short pulse durations are the quasi-CW type of flash-lamps wherein the continuos wave operation of this lamp is modulated electronically.

The figures according to the method of the present invention illustrate preferred embodiments of the present invention, and of devices using the methodology of the present invention, and are not intended to limit the scope of the present invention whatsoever. Figures 1-3, together with scientific support data herewith disclosed, are presented for clarity and for illustration purposes.

A preferred embodiment of the present invention illustrating a preferred mode for devices using the method of the present invention wherein sterilizing and disinfecting of water based products in their packaging without causing damage or migration or photochemical damage products or by products from said packaging or its content according to the present invention is being disinfected or sterilized throughout wherein the packaging material having a volume capacity of between about 1ml to about 10,000 liters and wherein said packaging is selected from PET, Polyolefin, and Polyamide, Polycarbonate, Polyeteramid, or Polyester or PE/HD, nylon or plastic, silicon, paper or silk cellulite or composite combination, or glass or any polymer resins combination thereof and containing sugar or flavoring or food additives or vitamins or minerals or supplements or nutritional additives and wherein each of said proportional components in the content of said packaging contains sugar in said water is from about 1 Gram per liter to about 987Gram Liter per volume concentration to about 99% per volume concentration thus flavored water with up to 100 Gram of sugar per liter and Glucose packages having higher concentration are an example of the scope and diversity of the present invention. A further environmental protection embodiment of the present invention is wherein a warehouse, or storage or filling or packaging, or delivery lines are equipped with devices according to the method of the present invention and wherein bottled water products at volumes from about 100ml to about 100 liters are being disinfected or sterilized through the packaging and thus made safe for drinking or consumption increasing its quality standards.

A preferred embodiment of the present invention wherein a solid state pulsed UVA laser light source operating at about 355nm is projecting light through an optical channeling and amplification system having at least one liquid flowing waveguide such that high energy density is coupled to a predetermined packaging material containing bottled water, treated water, flavored water, or carbonated water and wherein sparkling or still combination is enclosed within said packaging and being sterilized or disinfected according to the methodology of the present invention and wherein the volumes of the packaging to be disinfected throughout is selected from 100ml, or half liter, or 1 liter, or 1.5 liters or from about half a gallon to about 20 Gallon, and wherein said light is bouncing or reflecting, deflecting or diffusing, scattering or being absorb in a predetermined statistical portion of predetermined concentration of noxious biological or chemical components therein (I.e. in the content within the packaging), and wherein the components of the packaging, or the components in the content inside the packaging remain stable and unharmed while disinfection, sterilization or oxidation occurs cumuli attentively in a pre-calibrated dose response curve of said laser energy characteristic and the packaging, multi components therein (in the liquid or gas in the packaging) and adequate inactivation of DNA/RNA replication sequences, or mineralization of toxic organic compounds therein (in the liquids or gases in the packaging). A further preferred embodiment of the present invention is wherein the laser pulses repetitoon rate is between about 1 Hz to about 1 T Hz, and wherein its energy density reach from about 1 nJ to about 1000Js Cm2 .

A preferred mode for operation is when lasers or light sources operate at about 355nm, and if pulsed have a pulse width or pulse duration at sub-microsecond, however it may be that additional evolutionary steps in packaging material fabrication techniques may facilitate additional materials for packaging of liquids or gases not yet utilized by industry, agricultural or medical fields, such materials may have additional transmission windows (so that packaging will not be damaged) other then the 355nm, that why the methodology of the present invention facilitate an evolutionary step by utilizing a faster light transposing, or pumping, shifting or optronically or spatially manipulating, or delivering or targeting or diffusing or absorbing then the molecular thermal dynamic of the packaging material thus no damage to packaging occurs while disinfection or sterilization performance is engaged.

A preferred embodiment of the methodology of the present invention for a preferred mode of operation for devices using the method of the present invention for disinfection through packaging (DTP) is wherein the packaging for water based products is selected from ½, 1 liter, 1.5 liter, or 2 liter bottles or 1,2, 5,10 or 20 gallons, or wherein a conduit or camber shape pipe is interconnected or inter-operated for the delivery throughout of liquids or gases thereunto the packaging, or to/from said packaging aseptically or untreated, flavored or carbonated, oxygenated or enriched, supplemented or expanded with components or multi-components chemically and or biologically harmonious to the global optronic qualities and optival characteristics therein (in the packaging)

An especially beneficial environmental embodiment of the present invention is wherein the packaging is selected from a capsule, or pill, bottle or jug, container of tank, shaped as conduit or chamber or combinations or it may be made out of paper such as mail items or envelopes for letters, the addition of oxygenated water vapor at predetermined pressure throughout the process according to the present invention may be beneficial for making Advance Oxidation Technologies (AOT) therein and throughout the packaging using light and oxygen enriched water (H2O2). An improved embodiment of the present invention is having preferred mode for operating devices using the method of the present invention for disinfection or sterilization through packaging (DTP) without causing damage to said packaging material or causing molecular migration into said packaging from their liquid or gas content; a preferred mode for operating devices using the methodology of the present invention is wherein non

A preferred embodiment of the present invention is wherein the packaging item is a bottle or jug, or container, or a conduit or chamber type shape made from selection of monomers, carbomer, polymers, resins, or combination, and for example; any biocompatible packaging having ability of holding temporarily, or permanently water, air, liquids or gases for later consumption by end users, producers, industrialists, technologists, medical personnel, patients, and agro-food production market destination, and scientists and engineers. An additional preferred embodiment for disinfection through packaging is wherein the packaging is selected from a pill, or a capsule, or a bag, or combination, or perforated sausage like bags, or the packaging material could be selected from polymer or plastic or combination. An environmental protection preferred embodiment is wherein the packaging material is used for containing or transferring, holding or channeling water for drinking, flavored water, carbonated water, beverages, Juices, agro-food products, sweets, or confectionery products. A preferred embodiment of the methodology of the present invention is wherein the packaging is holding glucose, biomedical preparation, vaccination agents, or preparation for vaccination or medical optoprobiotic compounds according to the method of the present invention.

A preferred embodiment of the present invention is wherein the packaging is exposed to at least one radiation unit or light source selected from solid state lasers, electrical discharge lasers, plasma driven lasers, semiconductor lasers, organic lasers, electron beam pumped lasers, free electron lasers, , fiber lasers doped, or SASE/EA/FEL lasers, fiber lasers, diode pumped lasers, crystal based lasers, doped glass based lasers, FELs lasers, polymer lasers, PW/CW type lasers, quantum dote lasers, laser arrays, flush lamp pumped lasers, water lasers, photonic bandgap lasers, seeded or amplified lasers, time compressed or expanded lasers, q-switch type lasers, interactive harmonic lasers, acoustic-optical lasers, ultrasonic lasers, X-ray pumped lasers, Y pumped lasers, E-beam pumped lasers, catalytic lasers, photoelectrocatalytic lasers, air lasers, ground stationary lasers, mobile and sub-miniaturized lasers, thin film type lasers, vapor lasers, water lasers, photonic bandgapped lasers, CW lamps, PW lamps, Quasi CW lamps, LPHO lamps, Medium pressure UV lamps, Low pressure UV lamps, hybrid combination of CW, PW, Quasi CW lamps and lasers or combinations, and wherein light from said radiation unit or light sources is coupled to waveguides extending from said light units to reaching the proximity of said packaging, and wherein the high intensity light is reaching to cover the entirety of the packaging and content therein for disinfection and sterilization of said content or volume or packaging surfaces within a predetermined space, over a predetermined period of time.

A preferred embodiment of the methodology of the present invention is wherein the light from the radiation units or light sources or hybrid combination of lasers and lamps is delivered through optical processing units able to control aspects of the delivered or distributed light such as to manipulate their optical or spatial properties so as to maximize the distribution, or uniformity, or to adjust the cumulative, or immediate biodosimetric curves adequate for the inactivation, sterilization or disinfection or dissociation or oxidation of noxious species present inside the packaging, in the content of said packaging or on the surfaces of said packaging.

A preferred embodiment especially beneficial for the provision and facilitation of vaccination production methodologies and techniques are being enhanced by using the methodology of the present invention wherein the remains, and the residual, or the bio-mass or chemical-mass within said content, or packaging surfaces, or liquids or gasses, or solids therein are being processed and exposed according to the methodology of the present invention such that opto biotic beneficial effects are facilitated to the agro-food, or bio-medical domains production.

A further preferred embodiment of the present invention is wherein opto-pro-biotic products are being produced according to the method of the present invention wherein noxious species within the packaging and content to be disinfected are exposed and traceable recognition will occur upon consumption, or preceding treatment of the methodology of the present invention. A preferred mode for devices operating according to the method of the present ibvehntion is wherein the radiation unit or laser or light source is a pulsed, or continuos wave light source, or a further preferred mode is wherein the light radiation instrument includes a sub-microsecond pulsed source.

A preferred embodiment of the present invention is wherein the optical processing unit, which delivers the light to the packaging or content or space or volume or combination, is selected from diffractive optical elements, lenses or cubes, splitting or recombining optics and electro-optical elements, diffusers, reflectors, prisms, telescopic zooms, and zoom expanders, refractive optics, conversion or harmonic generators, pumping or amplified signals, amplifiers, converters, spatial processors, scanners, stirrers, beam positioning, location equation and transport interfaces, robotic arms with optical support means, optical bread boards, waveguids (HGFS, SFS,), photonic band gap, crystals, liquid waveguides, flowing liquid waveguides, or any combination thereof

A preferred embodiment of the present invention wherein the lasers or lamps operating is CW, PW, quasi CW, hybrid, liquid or gas based lasers, or the radiation unit or light sources combined or integrated include diamond based lasers, fiber lasers, TSA/SE/EA/FELs lasers, or electrical discharge lasers, or water lasers, or diod pumped or organic type lasers, or plasma lasers, or semi-conductor lasers, or amplified or harmonically generated lasers, or CW, PW lamps, or combination

A preferred embodiment of the present invention wherein the instrument or device is optically synchronized, or electronically linked or referenced, triggered or resolved to a particular machine control protocol for enhancing delivery and distribution of optical energy through packaging, and for the calibration and adjustment of the biodosimetric or chemical dosimetric curve adequate for sterilization or disinfection, dissociation or oxygenation of noxious species through the packaging without damaging said packaging or multi-components therein (in the packaging content or volume).

A preferred embodiment of the present invention wherein the packaging to be disinfected is an envelope or a mail item containing spores, bacteria or harmful compounds of biological or chemical origin. A Further beneficial biomedical preferred embodiment is wherein the effective range of the phototreatment effects according to the methodology of the present invention wherein opto-biotic, and/or opto probiotic water are produces according to the methodology of the present invention, such water may be beneficial for the immune system of consumers as the noxious species will be dissociated by the photochemical action or effects of processes according to the present invention, however, while the parent compound (example: before treatment, while liquids or gases or solids are in the packaging) before, or during, or after being treated, does not need to be transferred, or poured to, or transported via additional steps (i.e. additional chambers or conduits, bottles or jugs, containers or pipes for example), thus the methodology of the present invention is beneficial for the production of opto-pro-biotic wherein the sensitivity, or size of components facilitate treatment in the final packaging enclosure saving time, eliminating the need to keep transferring the volume to additional processes related steps and increasing the bio-compatibility and bio-safety, or purity, or freshness, or nutrient, or flavor, color or taste, smells or visibility or look, composite or density. A beneficial preferred embodiment for biological, physiological, and medical fields according to the methodology of the present invention is wherein multi-components compounds and medical and immunology fields thus optical dissociation of noxious species could be performed wherein the biodosimetric values required for dissociation are achieved using the methodology of the present invention. The benefits could extend from using the present invention to dissociate M.R.na type or any other multi component composite substances or chemical signatures thus that the dissociated remaining bio and/or chemical mass is appropriate for immunization processes upon consumption. More specifically, the present invention could be utilized for the production processes of opto-pro-biotic produce, products aseptically beneficial for medical, agricultural and industrial applications, beneficial for the bottling industry and for production processes of agro-food production lines. Further more triggering positive immune response actions and effects (MHC II, III, I) for example such as for Major-Histo-Compatibility Complex or MHCC, the acquired Human Immune system may be triggered according to the methodology of the present invention from liquid or gases or solids in a predetermined packaging containing a pre-form, or compounding volumes of noxious species therein (in the packaging content/volume/inner space), such beneficial basic preparation could be triggered processed, dissociated or oxygenated, or sterilized, or modified, or photochemical adjusted, or treated, until reaching by the utilization of the light which sound creates, and the sound which light creates i.e., for example the attack transient acoustically thus created via utilization of light in the present invention, and Sono-luminescence or light produced by applying vibration excitation measures, such that the appropriate proportions upon consumption by end users, or producers, or human, plant or animal their immune system is enhanced, in simple words the final processing according to the present invention is occurring in the respective packaging, through said packaging material, and without causing damage, or molecular migration from said packaging to the liquid or gas or solid or combination therein such that freshness, natural components, integrity in terms of geometry of multi components is maintained, prolonged, and higher quality of production is facilitated by the methodology of the present invention of Disinfection, and sterilization, and phototreatment through packaging (DTP) and therefore as well as longer and safer shelf life, the methodology of the present invention surpass limitation of current methodologies in the field and require less steps, less time, and less transport of processed items such that the probability of cross contamination, and transfer of infected, or toxic compounds is substantially lower as the final processing is done (in) Through the packaging (DTP).

A preferred embodiment of the present invention wherein a line of mass production for mineral Water bottles, is equipped according to the method of the present invention with at least one high intensity source of light, or laser and said light source is pulsed, having peak power density from about $0.01mJ/Cm2$, to around $50Js/Cm2$, and wherein said light source is monochromatic (i.e. laser), having wavelength from about 222nm, to about 355nm, and said light pulses are having a repetition rate from between about Hz, to about 1 GHz and said light is directed at a transparent, or translucent portion, or lid, or cork of said bottles, thus ensuring that harmful or noxious bacteria is inactivated, or eliminated therein, and DNA, & RNA replication Sequences of noxious species are thus inactivated inside the bottles by using appropriate wavelength and characteristics of light, before, during, or after they have been filled, corked, and/or sealed for use at later date.

A further environmental embodiment of the method of the present invention wherein the content of the bottles, conduits, or chambers may be selected from: beverages, wine, medical preparation, juice, drinking water, mineral water, insulin products or medical preperation. Spring water, flavored water, flavored beverages, biological traceable compounds, water, and/or drinks containing vitamines or nutrients, alcohol, blood products, plasma products, air products, gases for propelling medications, sprays, or any liquid or gasses or hybrid combination thereof.

A Novel environmental embodiment of the present Invention is having a high repetition rate, high peak power laser of the Nd:Yag, or Nd:Glass, or Nd: YLF, type or any combination thereof operating in the Fourth Harmonic generation mode (i.e. FHG), a further preferred embodiment of the present invention is having said solid state (i.e. Nd: Yag type for example), working in the Third Harmonic Generation mode (i.e. THG), a preferred embodiment according to the method of the present invention is having an electrical discharge laser such as an excimer laser operating in wavelength from about 193nm, through to about the 308, and 351 nm, and wherein each of said pulses of light is aligned into the content of the bottled liquids or gasses for purifying, disinfecting, and ensuring that DNA, and RNA replication sequences are thus inactivated providing a non invasive disinfecting methodology wherein light pulses from the laser are penetrating the material from which the bottles, (i.e. conduits or chambers, or bottles, or pipes) are made.

A preferred embodiment according to the method of the present Invention is having a hybrid laser system operating wherein (a) a solid state laser operating at THG (355nm), is attached, or aligned to the transparent lid, or cork, or encapsulation, or conduit, or chamber, and wherein together in hybrid format an electrical discharge laser is also operating for maximizing the efficiencies associated with the non destructive disinfection according to the method of the present invention Further preferred embodiment according to the present invention wherein the packaging is a liquid waveguide, or concentrator.

A preferred embodiment of the present invention wherein a Nd:Yag laser is used having a third Harmonic generator producing 355nm wavelength which penetrates the packaging materials of PET types and inactivating DNA & RNA replication sequences therein (in the bottle, or packaging). Such preferred embodiment of the present invention could be implemented with a single laser unit having its beam of pulsed UVA light split, and distributed to a plurality of location by means of waveguiding light through HGFS optical fibers, photonic band gap waveguides, or polymer waveguides, or hybrid combinations. A further preferred embodiment of the present invention is wherein several lasers are used having high repetition rates, or high peak powers, and pulse duration from about 1 ms, to about 1 fs, and wherein their respective beams are collected, or deflected, or diverted or stirred to form a three dimension element of UVA light at high energy density, and wherein said high energy density is below the damage threshold of PET packaging, or polymeric hybrid combination used as packaging materials.

A preferred embodiment of the present invention wherein the laser light source is sellected from (a) Gas discharge laser (b) diode pumped lasers, (c) plasma discharged lasers, (d) solid state lasers, (e) semi conductor lasers, (f) crystal type of lasers, (9), X-rays pumped lasers, (h) E-beam pumped gas lasers types, (I) FEL (Free Electron Laser amplifier), (J) EA/FEL (Electrostatically Accelerated Free Electron Laser), or organic laser types or any combination thereof.

A preferred embodiment of the present invention wherein the laser light source is tunable from about 1 nm to about 3000 nm, a further preferred embodiment of the present invention is wherein the laser light source is tunable from about 333nm to about 360nm, and wherein the peak power density of individual pulses reach from about 1 nJ/Cm2, to about 5OJs/Cm2' and wherein said pulsed laser light source is pulsing at repetition rates from about 1 Hz to about 300MHz thus the proffered embodiment of the ptresent invention is suitable for wide ranging application involving different packaging materials, thus their specie specific optical calibration standards are calculated for a specific bio-dosimetric value or curve to appropriately correspond (being lower) with damage threshold of the packaging material used in a specific application.

A further environmental embodiment of the method of the present invention wherein the content of the bottles, conduits, or chambers may be selected from: beverages, wine, medical preparation, juice, drinking water, mineral water, insuline products or medical preperation. Spring water, flavored water, flavored beverages, biological traceable compounds, Drug delivery using water based, and/or expanded or flavored water drinks containing vitamines or nutrients, alcohol, blood products, plasma products, air products, gases for propelling medications, sprays, or any liquid or gasses or hybrid combination thereof.

A Novel environmental embodiment of the present Invention is having a high repetition rate, high peak power laser of the Nd:Yag, or Nd:Glass, or Nd: YLF, type or any combination thereof driving the uvjet, or spatially processing pulsed laser light therein, or throughout and operating in the Fourth Harmonic generation mode (i.e. FHG), a further preferred embodiment of the present invention is having said solid state (i.e. Nd: Yag type for example), working in the Third Harmonic Generation mode (i.e. THG), a preferred embodiment according to the method of the present invention is having an electrical discharge laser such as an excimer laser operating in wavelength from about 193nm, through to about the 308, and 351 nm, and wherein each of said pulses of light is aligned

into the content of the bottled liquids or gasses for purifying, disinfecting, and ensuring that DNA, and RNA replication sequences are thus inactivated, providing a non invasive disinfecting methodology wherein light pulses from the laser are penetrating the material from which the bottles, (i.e. conduits or chambers, or bottles, or pipes) are made.

A preferred embodiment according to the method of the present Invention is having a hybrid laser system driving the uvjet, thus operating wherein (a) a solid state laser operating at THG (355nm), is attached, or aligned to the transparent lid, or cork, or encapsulation, or conduit, or chamber, and wherein together in hybrid format an electrical discharge laser is also operating for maximizing the efficiencies associated with the non destructive disinfection according to the method of the present invention. Further prefered embodiment according to the present invention wherein the packeging is a liquid waveguide, or concventrator.

A preffered embodiment of the present invention wherein a Nd:Yag laser is used having a third Harmonic generator producing 355nm wavelength which pentrates the surface of the skin in human, and animals and thus swiftly inactivates DNA & RNA replication sequences in blood therein (in the blood, i.e. under the skin, non invasively). Such preffered embodiment of the present invention could be implemented with at least one laser unit having its beam of pulsed UVA fight split, and distributed to a plurality of location by means of waveguiding light through HGFS optical fibers, photonic band gap waveguides, or polymer waveguides, or hybrid combinations, each distal tip is attached, supported, or threaded, in proximity or distributed to cover areas inthe body wherein blood vessels are relatively exposed, and are conviniently positioned around the surface (i.e. inporoving transmission, and coupling conditions). A further preffered embodiment of the present invention is wherein several lasers are used having high repetition rates, or high peak powers, and pulse duration from about 1 ms, to about 1 fs, or Atosecond and wherein their respective beams are collected, or diflected, or diverted or sttired to form a three dimension element of UVA light at high energy density, and wherein said high energy density is below the damage threshold of blood components, nutrients, conditioners, complimental systems, MHC type 1,2,3, and any vital cell present in blood or blood products, yet they are efficient for inactivation of noxious species therein, i.e. within the blood, or blood products.

A preffered embodiment of the present invention wherein the laser light source is sellcted from (a) Gas discherge laser, (b) diod pumped lasers, (c) plasma discharged lasers, (d) solid state lasers, (e) semi conductor lasers, (f) crystal type of lasers, (g), X-rays pumped lasers, (h) E-beam pumped gas lasers types,

FEL (Free Electron Laser amplifier), (j) EA/FEL (Electrostatically Accelerated Free Electron Laser), or organic laser types or any combination thereof.

A preffered embodiment of the present invention wherein the laser light source is tunable from about 1nm to about 3000nm, a further proffered embodiment of the pesent invention is wherein the laser light source is tunable from about 333nm to about 360nm, and wherein the peak power density of individual pulses reach from about 1nJ/Cm2, to about 50Js/Cm2, and wherein said pulsed laser light source is pulsing at repetition rates from about 1 Hz to about 300MHz thus the preffered embodiment of the ptresent invention is suitable for wide ranging application involving different packeging materials, thus their specie specific optical caliberation standards are calculated for a specific biodosimetric value or curve to appropriately corasponde (being lower) with damage threshold of the substrate material used in a specific application, or tool, or device.

DETAILED DESCRIPTION OF THE FIGURES

A preferred embodiment of the present invention illustrating a preferred mode for devices using the method of the present invention wherein sterilizing and disinfecting of water based products in their packaging without causing damage or migration or photochemical damage products or by products from said packaging or its content according to the present invention is being disinfected or sterilized throughout wherein the packaging material having a volume capacity of between about 1ml to about 10,000 liters and wherein said packaging is selected from PET, Polyolefin, and Polyamide, Polycarbonate, Polyeteramid, or Polyester or PE/HD, nylon or plastic, silicon, paper or silk cellulite or composite combination, or glass or any polymer resins combination thereof and containing sugar or flavoring or food additives or vitamins or minerals or supplements or nutritional additives and wherein each of said proportional components in the content of said packaging contains sugar in said water is from about 1Gram per liter to about 987Gram Liter per volume concentration to about 99% per volume concentration thus flavored water with up to 100 Gram of sugar per liter and Glucose packages having higher concentration are an example of the scope and diversity of the present invention. A further environmental protection embodiment of the present invention is wherein a warehouse, or storage or filling or packaging, or delivery lines are equipped with devices according to the method of the present invention and wherein bottled water products at volumes from about 100ml to about 100 liters are being disinfected or sterilized through the packaging and thus made safe for drinking or consumption increasing its quality standards.

A preferred embodiment of the present invention wherein a solid state pulsed UVA laser light source operating at about 355nm is projecting light through an optical channeling and amplification system having at least one liquid flowing waveguide such that high energy density is coupled to a predetermined packaging material containing bottled water, treated water, flavored water, or carbonated water and wherein sparkling or still combination is enclosed within said packaging and being sterilized or disinfected according to the methodology of the present invention and wherein the volumes of the packaging to be disinfected throughout is selected from 100ml, or half liter, or 1 liter, or 1.5 liters or from about half a gallon to about 20 Gallon, and wherein said light is bouncing or reflecting, deflecting or diffusing, scattering or being absorb in a predetermined statistical portion of predetermined concentration of noxious biological or chemical components therein (I.e. in the content within the packaging), and wherein the components of the packaging, or the components in the content inside the packaging remain stable and unharmed while disinfection, sterilization or oxidation occurs cumuli attentively in a pre-calibrated dose response curve of said laser energy characteristic and the packaging, multi components therein (in the liquid or gas in the packaging) and adequate inactivation of DNA/RNA replication sequences, or mineralization of toxic organic compounds therein (in the liquids or gases in the packaging). A further preferred embodiment of the present invention is wherein the laser pulses repetitoon rate is between about 1 Hz to about 1 T Hz, and wherein its energy density reach from about 1 nJ to about 1000Js Cm2 .

A preferred mode for operation is when lasers or light sources operate at about 355nm, and if pulsed have a pulse width or pulse duration at sub-microsecond, however it may be that additional evolutionary steps in packaging material fabrication techniques may facilitate additional materials for packaging of liquids or gases not yet utilized by industry, agricultural or medical fields, such materials may have additional transmission windows (so that packaging will not be damaged) other then the 355nm, that why the methodology of the present invention facilitate an evolutionary step by utilizing a faster light transposing, or pumping, shifting or optronically or spatially manipulating, or delivering or targeting or diffusing or absorbing then the molecular thermal dynamic of the packaging material thus no damage to packaging occurs while disinfection or sterilization performance is engaged.

A preferred embodiment of the methodology of the present invention for a preferred mode of operation for devices using the method of the present invention for disinfection through packaging (DTP) is wherein the packaging for water based products is selected from ½, 1 liter, 1.5 liter, or 2 liter bottles or 1,2, 5,10 or 20 gallons, or wherein a conduit or camber shape pipe is interconnected or inter-operated for the delivery throughout of liquids or gases thereunto the packaging, or to/from said packaging aseptically or untreated, flavored or carbonated, oxygenated or enriched, supplemented or expanded with components or multi-components chemically and or biologically harmonious to the global optronic qualities and optival characteristics therein (in the packaging)

An especially beneficial environmental embodiment of the present invention is wherein the packaging is selected from a capsule, or pill, bottle or jug, container of tank, shaped as conduit or chamber or combinations or it may be made out of paper such as mail items or envelopes for letters, the addition of oxygenated water vapor at predetermined pressure throughout the process according to the present invention may be beneficial for making Advance Oxidation Technologies (AOT) therein and throughout the packaging using light and oxygen enriched water (H2O2). An improved embodiment of the present invention is having preferred mode for operating devices using the method of the present invention for disinfection or sterilization through packaging (DTP) without causing damage to said packaging material or causing molecular migration into said packaging from their liquid or gas content; a preferred mode for operating devices using the methodology of the present invention.

A preferred embodiment of the present invention is wherein the packaging item is a bottle or jug, or container, or a conduit or chamber type shape made from selection of monomers, carbomer, polymers, resins, or combination, and for example; any biocompatible packaging having ability of holding temporarily, or permanently water, air, liquids or gases for later consumption by end users, producers, industrialists, technologists, medical personnel, patients, and agro-food production market destination, and scientists and engineers. An additional preferred embodiment for disinfection through packaging is wherein the packaging is selected from a pill, or a capsule, or a bag, or combination, or perforated sausage like bags, or the packaging material could be selected from polymer or plastic or combination.

An environmental protection preferred embodiment is wherein the packaging material is used for containing or transferring, holding or channeling water for drinking, flavored water, carbonated water, beverages, Juices, agro-food products, sweets, or confectionery products. A preferred embodiment of the methodology of the present invention is wherein the packaging is holding glucose, biomedical preparation, vaccination agents, or preparation for vaccination or medical optoprobiotic compounds according to the method of the present invention.

A preferred embodiment of the present invention is wherein the packaging is exposed to at least one radiation unit or light source selected from solid state lasers, electrical discharge lasers, plasma driven lasers, semiconductor lasers, organic lasers, electron beam pumped lasers, free electron lasers, , fiber lasers doped, or SASE/EA/FEL lasers, fiber lasers, diode pumped lasers, crystal based lasers, doped glass based lasers, FELs lasers, polymer lasers, PW/CW type lasers, quantum dote lasers, laser arrays, flush lamp pumped lasers, water lasers, photonic bandgap lasers, seeded or amplified lasers, time compressed or expanded lasers, q-switch type lasers, interactive harmonic lasers, acoustic-optical lasers, ultrasonic lasers, X-ray pumped lasers, Y pumped lasers, E-beam pumped lasers, catalytic lasers, photoelectrocatalytic lasers, air lasers, ground stationary lasers, mobile and sub-miniaturized lasers, thin film type lasers, vapor lasers, water lasers, photonic bandgapped lasers, CW lamps, PW lamps, Quasi CW lamps, LPHO lamps, Medium pressure UV lamps, Low pressure UV lamps, hybrid combination of CW, PW, Quasi CW lamps and lasers or combinations, and wherein light from said radiation unit or light sources is coupled to waveguides extending from said light units to reaching the proximity of said packaging, and wherein the high intensity light is reaching to cover the entirety of the packaging and content therein for disinfection and sterilization of said content or volume or packaging surfaces within a predetermined space, over a predetermined period of time.

A preferred embodiment of the methodology of the present invention is wherein the light from the radiation units or light sources or hybrid combination of lasers and lamps is delivered through optical processing units able to control aspects of the delivered or distributed light such as to manipulate their optical or spatial properties so as to maximize the distribution, or uniformity, or to adjust the cumulative, or immediate biodosimetric curves adequate for the inactivation, sterilization or disinfection or dissociation or oxidation of noxious species present inside the packaging, in the content of said packaging or on the surfaces of said packaging.

A preferred embodiment especially beneficial for the provision and facilitation of vaccination production methodologies and techniques are being enhanced by using the methodology of the present invention wherein the remains, and the residual, or the bio-mass or chemical-mass within said content, or packaging surfaces, or liquids or gasses, or solids therein are being processed and exposed according to the methodology of the present invention such that opto biotic beneficial effects are facilitated to the agro-food, or bio-medical domains production.

A further preferred embodiment of the present invention is wherein opto-pro-biotic products are being produced according to the method of the present invention wherein noxious species within the packaging and content to be disinfected are exposed and traceable recognition will occur upon consumption, or preceding treatment of the methodology of the present invention. A preferred mode for devices operating according to the method of the present ibvehntion is wherein the radiation unit or laser or light source is a pulsed, or continuos wave light source, or a further preferred mode is wherein the light radiation instrument includes a sub-microsecond pulsed source.

A preferred embodiment of the present invention is wherein the optical processing unit, which delivers the light to the packaging or content or space or volume or combination, is selected from diffractive optical elements, lenses or cubes, splitting or recombining optics and electro-optical elements, diffusers, reflectors, prisms, telescopic zooms, and zoom expanders, refractive optics, conversion or harmonic generators, pumping or amplified signals, amplifiers, converters, spatial processors, scanners, stirrers, beam positioning, location equation and transport interfaces, robotic arms with optical support means, optical bread boards, waveguids (HGFS, SFS,), photonic band gap, crystals, liquid waveguides, flowing liquid waveguides, or any combination thereof

A preferred embodiment of the present invention wherein the lasers or lamps operating is CW, PW, quasi CW, hybrid, liquid or gas based lasers, or the radiation unit or light sources combined or integrated include diamond based lasers, fiber lasers, TSA/SE/EA/FELs lasers, or electrical discharge lasers, or water lasers, or diod pumped or organic type lasers, or plasma lasers, or semi-conductor lasers, or amplified or harmonically generated lasers, or CW, PW lamps, or combination

A preferred embodiment of the present invention wherein the instrument or device is optically synchronized, or electronically linked or referenced, triggered or resolved to a particular machine control protocol for enhancing delivery and distribution of optical energy through packaging, and for the calibration and adjustment of the biodosimetric or chemical dosimetric curve adequate for sterilization or disinfection, dissociation or oxygenation of noxious species through the packaging without damaging said packaging or multi-components therein (in the packaging content or volume).

A preferred embodiment of the present invention wherein the packaging to be disinfected is an envelope or a mail item containing spores, bacteria or harmful compounds of biological or chemical origin. A Further beneficial biomedical preferred embodiment is wherein the effective range of the phototreatment effects according to the methodology of the present invention wherein opto-biotic, and/or opto probiotic water are produces according to the methodology of the present invention, such water may be beneficial for the immune system of consumers as the noxious species will be dissociated by the photochemical action or effects of processes according to the present invention, however, while the parent compound (example: before treatment, while liquids or gases or solids are in the packaging) before, or during, or after being treated, does not need to be transferred, or poured to, or transported via additional steps (i.e. additional chambers or conduits, bottles or jugs, containers or pipes for example), thus the methodology of the present invention is beneficial for the production of opto-pro-biotic wherein the sensitivity, or size of components facilitate treatment in the final packaging enclosure saving time, eliminating the need to keep transferring the volume to additional processes related steps and increasing the bio-compatibility and bio-safety, or purity, or freshness, or nutrient, or flavor, color or taste, smells or visibility or look, composite or density. A beneficial preferred embodiment for biological, physiological, and medical fields according to the methodology of the present invention is wherein multi-components compounds and medical and immunology fields thus optical dissociation of noxious species could be performed wherein the biodosimetric values required for dissociation are achieved using the methodology of the present invention. The benefits could extend from using the present invention to dissociate M.R.na type or any other multi component composite substances or chemical signatures thus that the dissociated remaining bio and/or chemical mass is appropriate for immunization processes upon consumption. More specifically, the present invention could be utilized for the production processes of opto-pro-biotic produce, products aseptically beneficial for medical, agricultural and industrial applications, beneficial for the bottling industry and for production processes of agro-food production lines. Further more triggering positive immune response actions and effects (MHC II, III, I) for example such as for Major-Histo-Compatibility Complex or MHCC, the acquired Human Immune system may be triggered according to the methodology of the present invention from liquid or gases or solids in a predetermined packaging containing a pre-form, or compounding volumes of noxious species therein (in the packaging content/volume/inner space), such beneficial basic preparation could be triggered processed, dissociated or oxygenated, or sterilized, or modified, or photochemical adjusted, or treated, until reaching by the utilization of the light which sound creates, and the sound which light creates i.e., for example the attack transient acoustically thus created via utilization of light in the present invention, and Sono-luminescence or light produced by applying vibration excitation measures, such that the appropriate proportions upon consumption by end users, or producers, or human, plant or animal their immune system is enhanced, in simple words the final processing according to the present invention is occurring in the respective packaging, through said packaging material, and without causing damage, or molecular migration from said packaging to the liquid or gas or solid or combination therein such that freshness, natural components, integrity in terms of geometry of multi components is maintained, prolonged, and higher quality of production is facilitated by the methodology of the present invention of Disinfection, and sterilization, and phototreatment through packaging (DTP) and therefore as well as longer and safer shelf life, the methodology of the present invention surpass limitation of current methodologies in the field and require less steps, less time, and less transport of processed items such that the probability of cross contamination, and transfer of infected, or toxic compounds is substantially lower as the final processing is done (in) Through the packaging (DTP).

A preferred embodiment of the present invention wherein a line of mass production for mineral Water bottles, is equipped according to the method of the present invention with at least one high intensity source of light, or laser and said light source is pulsed, having peak power density from about 0.01mJ/Cm2, to around 50Js/Cm2, and wherein said light source is monochromatic (i.e. laser), having wavelength from about 222nm, to about 355nm, and said light pulses are having a repetition rate from between about Hz, to about 1 GHz and said light is directed at a transparent, or translucent portion, or lid, or cork of said bottles, thus ensuring that harmful or noxious bacteria is inactivated, or eliminated therein, and DNA, & RNA replication Sequences of noxious species are thus inactivated inside the bottles by using appropriate wavelength and characteristics of light, before, during, or after they have been filled, corked, and/ or sealed for use at later date.

A further environmental embodiment of the method of the present invention wherein the content of the bottles, conduits, or chambers may be selected from: beverages, wine, medical preparation, juice, drinking water, mineral water, insulin products or medical preperation. Spring water, flavored water, flavored beverages, biological traceable compounds, water, and/or drinks containing vitamines or nutrients, alcohol, blood products, plasma products, air products, gases for propelling medications, sprays, or any liquid or gasses or hybrid combination thereof.

A Novel environmental embodiment of the present Invention is having a high repetition rate, high peak power laser of the Nd:Yag, or Nd:Glass, or Nd: YLF, type or any combination thereof operating in the Fourth Harmonic generation mode (i.e. FHG), a further preferred embodiment of the present invention is having said solid state (i.e. Nd: Yag type for example), working in the Third Harmonic Generation mode (i.e. THG), a preferred embodiment according to the method of the present invention is having an electrical discharge laser such as an excimer laser operating in wavelength from about 193nm, through to about the 308, and 351nm, and wherein each of said pulses of light is aligned into the content of the bottled liquids or gasses for purifying, disinfecting, and ensuring that DNA, and RNA replication sequences are thus inactivated providing a non invasive disinfecting methodology wherein light pulses from the laser are penetrating the material from which the bottles, (i.e. conduits or chambers, or bottles, or pipes) are made.

A preferred embodiment according to the method of the present Invention is having a hybrid laser system operating wherein (a) a solid state laser operating at THG (355nm), is attached, or aligned to the transparent lid, or cork, or encapsulation, or conduit, or chamber, and wherein together in hybrid format an electrical discharge laser is also operating for maximizing the efficiencies associated with the non destructive disinfection according to the method of the present invention Further preferred embodiment according to the present invention wherein the packaging is a liquid waveguide, or concentrator.

A preferred embodiment of the present invention wherein a Nd:Yag laser is used having a third Harmonic generator producing 355nm wavelength which penetrates the packaging materials of PET types and inactivating DNA & RNA replication sequences therein (in the bottle, or packaging). Such preferred embodiment of the present invention could be implemented with a single laser unit having its beam of pulsed UVA light split, and distributed to a plurality of location by means of waveguiding light through HGFS optical fibers, photonic band gap waveguides, or polymer waveguides, or hybrid combinations. A further preferred embodiment of the present invention is wherein several lasers are used having high repetition rates, or high peak powers, and pulse duration from about 1 ms, to about 1 fs, and wherein their respective beams are collected, or deflected, or diverted or stirred to form a three dimension element of UVA light at high energy density, and wherein said high energy density is below the damage threshold of PET packaging, or polymeric hybrid combination used as packaging materials.

A preferred embodiment of the present invention wherein the laser light source is sellected from (a) Gas discharge laser (b) diode pumped lasers, (c) plasma discharged lasers, (d) solid state lasers, (e) semi conductor lasers, (f) crystal type of lasers, (9), X-rays pumped lasers, (h) E-beam pumped gas lasers types, (I) FEL (Free Electron Laser amplifier), (J) EA/FEL (Electrostatically Accelerated Free Electron Laser), or organic laser types or any combination thereof.

A preferred embodiment of the present invention wherein the laser light source is tunable from about 1 nm to about 3000 nm, a further preferred embodiment of the present invention is wherein the laser light source is tunable from about 333nm to about 360nm, and wherein the peak power density of individual pulses reach from about 1nJ/Cm2, to about 5OJs/Cm2' and wherein said pulsed laser light source is pulsing at repetition rates from about 1 Hz to about 300MHz thus the proffered embodiment of the ptresent invention is suitable for wide ranging application involving different packaging materials, thus their specie specific optical calibration standards are calculated for a specific bio-dosimetric value or curve to appropriately correspond (being lower) with damage threshold of the packaging material used in a specific application.

A further environmental embodiment of the method of the present invention wherein the content of the bottles, conduits, or chambers may be selected from: beverages, wine, medical preparation, juice, drinking water, mineral water, insuline products or medical preperation. Spring water, flavored water, flavored beverages, biological traceable compounds, Drug delivery using water based, and/or expanded or flavored water drinks containing vitamines or nutrients, alcohol, blood products, plasma products, air products, gases for propelling medications, sprays, or any liquid or gasses or hybrid combination thereof.

A Novel environmental embodiment of the present Invention is having a high repetition rate, high peak power laser of the Nd:Yag, or Nd:Glass, or Nd: YLF, type or any combination thereof driving the uvjet, or spatially processing pulsed laser light therein, or throughout and operating in the Fourth Harmonic generation mode (i.e. FHG), a further preferred embodiment of the present invention is having said solid state (i.e. Nd: Yag type for example), working in the Third Harmonic Generation mode (i.e. THG), a preferred embodiment according to the method of the present invention is having an electrical discharge laser such as an excimer laser operating in wavelength from about 193nm, through to about the 308, and 351 nm, and wherein each of said pulses of light is aligned

into the content of the bottled liquids or gasses for purifying, disinfecting, and ensuring that DNA, and RNA replication sequences are thus inactivated, providing a non invasive disinfecting methodology wherein light pulses from the laser are penetrating the material from which the bottles, (i.e. conduits or chambers, or bottles, or pipes) are made.

A preferred embodiment according to the method of the present Invention is having a hybrid laser system driving the uvjet, thus operating wherein (a) a solid state laser operating at THG (355nm), is attached, or aligned to the transparent lid, or cork, or encapsulation, or conduit, or chamber, and wherein together in hybrid format an electrical discharge laser is also operating for maximizing the efficiencies associated with the non destructive disinfection according to the method of the present invention. Further prefered embodiment according to the present invention wherein the packeging is a liquid waveguide, or concventrator.

A preffered embodiment of the present invention wherein a Nd:Yag laser is used having a third Harmonic generator producing 355nm wavelength which pentrates the surface of the skin in human, and animals and thus swiftly inactivates DNA & RNA replication sequences in blood therein (in the blood, i.e. under the skin, non invasively). Such preffered embodiment of the present invention could be implemented with at least one laser unit having its beam of pulsed UVA light split, and distributed to a plurality of location by means of waveguiding light through HGFS optical fibers, photonic band gap waveguides, or polymer waveguides, or hybrid combinations, each distal tip is attached, supported, or threaded, in proximity or distributed to cover areas inthe body wherein blood vessels are relatively exposed, and are conviniently positioned around the surface (i.e. inporoving transmission, and coupling conditions). A further preffered embodiment of the present invention is wherein several lasers are used having high repetition rates, or high peak powers, and pulse duration from about 1 ms, to about 1 fs, or Atosecond and wherein their respective beams are collected, or directed, or diverted or sttired to form a three dimension element of UVA light at high energy density, and wherein said high energy density is below the damage threshold of blood components, nutrients, conditioners, complimental systems, MHC type 1,2,3, and any vital cell present in blood or blood products, yet they are efficient for inactivation of noxious species therein, i.e. within the blood, or blood products.

A preffered embodiment of the present invention wherein the laser light source is sellcted from (a) Gas discherge laser, (b) diod pumped lasers, (c) plasma discharged lasers, (d) solid state lasers, (e) semi conductor lasers, (f) crystal type of lasers, (g), X-rays pumped lasers, (h) E-beam pumped gas lasers types,

FEL (Free Electron Laser amplifier), (j) EA/FEL (Electrostatically Accelerated Free Electron Laser), or organic laser types or any combination thereof.

A preffered embodiment of the present invention wherein the laser light source is tunable from about 1 nm to about 3000nm, a further preffered embodiment of the pesent invention is wherein the laser light source is tunable from about 333nm to about 360nm, and wherein the peak power density of individual pulses reach from about 1 nJ/Cm2, to about 50Js/Cm2, and wherein said pulsed laser light source is pulsing at repetition rates from about 1 Hz to about 300MHz thus the preffered embodiment of the ptresent invention is suitable for wide ranging application involving different packeging materials, thus their specie specific optical caliberation standards are calculated for a specific biodosimetric value or curve to appropriately corasponde (being lower) with damage threshold of the substrate material used in a specific application, or tool, or device.

The present invention disclosed a novel methodology for non-invasive disinfection, purification, and inactivation or equlization of (DNA, & RNA) replication sequences of noxious species in myriad of biomedical, and biotechnological applications involving end users, producers, and researchers in associated fields comprising the following steps:

Filling, distributing into, holding, or storing - in a predetermined chamber or conduit, a predetermined volume of liquids, or gasses to be non-invasively non residually treated.

Closing said conduit, or chamber with a transparent polymer or glass type lid, having a predetermined action spectrum, able to deliver, and transmit throughout any wavelength of light from about 260nm, to about the 360nm.

Implementation of the novel methodology of the present invention for surface treatment and for improving the hygiene of the mouth, may include (a) harnessing the illumination or irradiation of a waveguiding dielectric brush [WDB] having delivery capacity from about one quarter of a million of photons per Cm2/Second, to about 999 trillion potons per Cm2/1 picosecond, or Femtosecond, or Atosecond, thus swiftly sterilizing its complex curvature inner surfaces, and volumes, having variable depth of penetration catalytically using new generation tooth paste comprising; Catalytic centilayted compound [CCC] according to the methodology of the present invention, and wherein

Constructing or integrating a multi-component compound structurally modular, containing predetermined portion of yielding Oxygen Charge (SYOCH) in a U.P.W, PH stabilized, held temporarily or permanently in a 3D polymeric frame work of biodegradable biocompatible carbomer or BI-polymers expanded to contain photo-catalytic, and, or centilating conversion elementally each having predetermined electron charge transfer co.-efficiencies and absorption, refractive index profile, and acoustic properties, selected pre-production for quantum objective application specific efficiencies driven thus from supper conductive, to dielectric, or semi conducting, wherein the flexibility of water may provide generic structurally yielding Oxygen Charge accommodating into manageable forms the decomposed species inactivated radically (SYOCH1), within water, liquid or gas or air suspension, body fluids,or inside mouth.

DETAILED DESCRIPTION OF THE FIGURES:

Thew present invention will be further described in detail by Figures 1 - 15. These Figures are solely intend to illustrates some preferred embodiments of the present invention, and in no manner intend to limit the scope of the invention.

Figure 1 illustrates a method of the present invention as illustrated as figure 1 herein comprising: A method for disinfection and sterilization through packaging, non invasively, without causing damage to said packaging and content comprising: A radiation unit having a high intensity source of light (not Shawn) wherein its light delivery or pulse duration is shorter then the thermal dynamics of the packaging according to the methodology of the present invention for projecting or delivering a pulsed light beam (3), illustrated penetrating the packaging without causing damage or migration or change in composition of said packaging or content; light is Shawn delivered (3) via direct trajection or projection of light or through the use of optical waveguide (not Shawn). Bacteria, viruses, cysts, molds, germs, spores, and toxic or noxious species of chemical and biological origins which may be present in said packaging or chamber or conduit (not Shawn) are sterilized, inactivated, disinfected or dissociated therein (in the packaging and on packaging surfaces) are thus being made innocuous according to the present invention The bottle packaging (2) is illustrating as holding water, mineral water, flavored water, carbonated water, spring water, and bottled or treated water according to the methodology of the present invention. (4) Represent the air capsule, or space remaining between the top inner surface of the liquid in the bottle and the cap (1), air having the refractive index of 1.00, and water having a refractive index of about 1.3, thus when twisted to form a diffuser or to spatially equalize and distribute uniformly through out packaging and content volume, thus the packaging and its content are sterilized, or disinfected so liquids and gases or solids or surfaces are being sterilized or disinfected through packaging, non invasively, through packaging bounderies without causing damage to packaging and content or volume therein (in the packaging). By the plurality of refractive index profiles thus created according to the methodology of the present invention for non invasive through the packaging DTP photochemical interactions, light could be guided, and distributed throughout the bottle, jug, container, pipes, or any chamber or conduit having adequate spectral properties or transparency, and appropriately composed so as to be biocompatible for holding agro-food production items or packaged water products. Especially beneficial for agro-food industries and for biomedical domain hence methodology of the present invention is environmental friendly, and has a non chemical, non toxic, non residual processing capabilities through packaging (1-4).

Figure 2 illustrates a schematic view of the method of the present invention implemented in a mass production line of mineral water, flavored water, beverages, spring water, bottled water, or carbonated water, or enhanced water, or any combination thereof or in biotechnology production lines for medical preparation, or for blood processing; A schematic view of the method of the present invention for non invasive, non damaging disinfection through packaging DTP is herewith disclosed comprising: 5 gallons or a series of water containers, or chambers are Shawn (5,6,7,8,10) wherein a beam of light (11) is illustrated penetrating (5-11) the packaging (5,6,7,8,10) wherein 9 illustrate the air capsule or pocket left in any bottled water, or packaged beverage or water thus illustrating the potential diversity of refractive index profiles thus being generated by twisting the volume (and/or the packaging with the content) therein (in the packaging) or by delivering light throughout from adequate angular orientation, thus disinfecting and sterilizing, dissociating or oxidizing noxious species on the packaging surfaces, or throughout the packaging content, or combination, according to the present invention the time domain of the delivered light (pulsed sub-microsecond time domain) is shorter then the thermal dynamics of he packaging material (for example , PET penetrated by a diod pumped Yag at 355nm, 75mHz repetition rate, and 3 ns pulse width), thus disinfection, or phototreatment (i.e. Oxidation, dissociation, reduction, elimination, inactivation, sterilization, photolysis, photo activation), optoprobiotic effects are achieved, and occurs according to the present invention non invasively without causing damage to said packaging or content. Especially beneficial for agro-food industries, for bottling industry (5,6,7,8,10) and for the production of bottled water (5,6,7,8,10) and biomedical preparation of optoprobiotic water based or bottled water products (5,6,7,8,10).

Figure 3 illustrates a method for disinfection and sterilization through packaging, non invasively, without causing damage to said packaging and content comprising: A radiation unit (12) having a high intensity source of light (13), or laser, or hybrid platform having pulsed light at wavelength of about 355nm, having pulsed duration or width of between 1 microsecond and about 1 Ato-second, As, wherein its light delivery or pulse duration is shorter then the thermal dynamics of the packaging (15) to be disinfected or sterilized or phototreated according to the methodology of the present invention for projecting or delivering a pulsed light beam (15), illustrated penetrating the packaging without causing damage or migration or change in composition of said packaging or content; light is Shawn delivered (15) via direct trajection or projection of appropriate spectral distribution over a predetermined space over a predetermined period of time thus appropriate biodosimetric curve, or chemical dosimetric values (not Shawn) for noxious species Bacteria, viruses, cysts, molds, germs, spores, and toxic or noxious species of chemical and biological origins which may be present in said packaging or chamber or conduit (not Shawn), or content or volume therein is being sterilized, inactivated, disinfected or dissociated therein (in the packaging and on packaging surfaces) are thus being made innocuous according to the present invention The bottle packaging (2) is illustrating as holding water, , (bottom of the bottles are not Shawn) mineral water, flavored water, carbonated water, spring water, and bottled or treated water according to the methodology of the present invention. (16) Represent the air capsule, or space remaining between the top inner surface of the liquid in the bottle, chamber or conduit, and the cap (14), air (16) having the refractive index of 1.00, and water having a refractive index of about 1.3, thus when twisted to form a diffuser or to spatially equalize and distribute uniformly through out packaging (15) and content volume, thus the packaging and its content are sterilized, or disinfected so liquids and gases or solids or surfaces are being sterilized or disinfected through packaging (15 a,b,c), non invasively, through packaging bounderies without causing damage to packaging and content or volume therein (in the packaging). By the plurality of refractive index profiles (16,15), (13a, 13b, 13c) thus created according to the methodology of the present invention for non invasive through the packaging DTP photochemical interactions, light could be guided, and distributed (13a, 13b, 13c) throughout the bottle, jug, container, pipes, or any chamber or conduit having adequate spectral properties or transparency, and appropriately composed so as to be biocompatible for holding agro-food production items or packaged water products. Especially beneficial for agro-food industries and for biomedical domain hence methodology of the present invention is environmental friendly, and has a non chemical, non toxic, non residual processing capabilities through packaging (15,a,b,c).

Figure 4 illustrates transmission through packaging and sterilization and disinfection through packaging DTP without damaging said packaging or content therein, non invasively comprising a packaging (20, 21) selected from polymers having appropriate transparency or optical properties (20, 21) or combination including polyethylene naphtalat (PEN), poilyalkyl naphtalat (PETG) having adequate refractive index profile throughout for the delivery, penetration or distribution of light throughout for disinfection, or sterilization, or dissociation, or oxygenation through packaging or DTP (18-21) (Disinfection Through Packaging) according to the present invention (1-80), a radiation unit (18) is illustrated as having a sub-microsecond pulse duration or width and a wavelength (not Shawn) at about 355nm, and repetition rates from about 1 Hz to about THz, exposure of the bottles can be accessed from any angular orientation as the diffuser therein created by twisting the content or volume of liquids or gasses therein (in the packaging), or twisting said packaging will deliver the light throughout uniformly, and spatially be distributed within the predetermined inner space or volume of the packaging (20, 21) over a predetermined period of time, each optronic event or pulse is shorter then the thermal dynamic curve of said polymers or combination or any other packaging material or conduit or chamber throughout so as to disinfect or sterilize the packaging (20, 21) or content therein non invasively using light. Especially beneficial for agro-food industries, biomedical, or for bottled water products containing sugar or flavoring, or for carbonated water or for beverages, or for milk based products or for medical preparation of opto-pro-biotic items, or for optoprobiotic water or drinks wherein the non invasive methodology of the present invention is utilized for processing by light (19) or disinfecting water and water based products through packaging (20, 21), DTP (Disinfection Through Packaging) according to the methodology of the present invention.

Figure 5 illustrates schematic view of the method of the present invention; A method for non invasive disinfection through packaging (DTP) is schematically illustrated featuring transmission through packaging (22-28) and sterilization and disinfection through packaging (24, 25, 26, 27,) DTP without damaging said packaging (24-27) or content therein, non invasively comprising a packaging (24, 25, 26, 27,) selected from polymers having appropriate transparency or optical properties (24-28) or combination including polyethylene naphtalat (PEN), poilyalkyl naphtalat (PETG), or Polycarbonate, or PET, or PET HD, or combination having adequate refractive index profile throughout for the delivery, penetration or distribution of light (22a) at about 355nm (22a) throughout (24, 25,) for disinfection, or sterilization, or dissociation, or oxygenation through packaging or DTP (1-80) (Disinfection Through Packaging) according to the present invention (1-80), a radiation unit (18) is illustrated as having a sub-microsecond pulse duration or width and a wavelength (not Shawn) at about 355nm, and repetition rates from about 1 Hz to about THz, exposure of the bottles can be accessed from any angular orientation as the diffuser therein created by twisting the content or volume of liquids or gasses therein (is not Shawn).Especially beneficial for disinfection through packaging (DTP) of bottled water products, liquids or gases for medical preparation, and for agro-food production lines having packaged water product with volumes reaching from 1 micro-liter to about 20 liters, or from a single drop to about 1000 liters, wherein each liter may contain sugar or flavoring or additives or supplements or enhancements from about 1 mg per liter to about 999 Grams per liter. Especially beneficial for bottling industry or for biomedical domain for the preparation of opto probiotic and immune system enhancement in water and bottled water products. An instrument being a laser or lamp or hybrid combination is illustrated (22) has having light coupled to the packaging (24-27) from the right side, the light (22a) is illustrated penetrating the packaging without causing damage to said packaging, thus non invasively facilitates disinfection through packaging (DTP).

Figure 6 illustrates Disinfection through packaging (DTP) without damaging said packaging or content therein, non invasively comprising a packaging (31) selected from polymers having appropriate transparency or optical properties (38, 31) or combination including polyethylene naphtalat (PEN), poilyalkyl naphtalat (PETG) having adequate refractive index profile throughout for the delivery, penetration or distribution of light throughout for disinfection, or sterilization, or dissociation, or oxygenation through packaging or DTP (29-39) (Disinfection Through Packaging) according to the present invention (1-80), a radiation unit (not shown) is illustrated as having a sub-microsecond pulse duration or width and a wavelength (not Shown) at about 355nm, (29) represents the pulsed (sub-microsecond) light penetrating from the cap, and said cap is transparent from about 318nm to about 787nm, and wherein the air capsule or pocket (41) is illustrated right underneath the cap, the bottle bounderies is illustrated in (31-38), wherein the hollow star in the packaging illustrated by (34) is representing a harmful bacteria, cysts, virus or noxious biological species origin, and (35) illustrated in the black star - represent toxic chemicals or noxious organic compounds of chemical origin, especially beneficial for protecting packaging content and surfaces without damage (envelopes not shown) (35) represent flavoring in a 5 tips shape in black (37), and the moon shape on the lower right (36) represent flavoring molecule undamaged by the delivered light throughout, the top right black star type shape (33) illustrate preservative or additives, nutrient or enhancement also undamaged by the distributed light throughout (DTP), the time domain, or pulse width or modulated light delivered according to the methodology of the present invention (29, light source not shown) is substantially shorter then the thermal dynamic of the packaging material or the oxygenation threshold of the flavoring, sugars, additives, in said packaging or bottle thus non invasively, the method of the present invention facilitate disinfection through packaging without damage to packaging composition or content compositions therein. Especially beneficial for bottled water production lines, or for agro-food industries, beneficial for biomedical domain and for the production of optoprobiotic drinks for enhancing the immune system of human, animal or plants. Especially beneficial for the bottling industry for disinfection through packaging without damage to flavors or preservatives or additives, or packaging, or freshness, eliminating the need to use thermal expensive aseptic filling, and processing, and reduce the high periodical maintenance and replacements associated with conventional non DTP technologies. The edge of the light (39) is illustrated after transgressing or penetrating the packaging (30-38) non-invasively. Especially beneficial for agro-food production, production and distribution lines for bottled water, flavored water, spring water, beverages, juices, and biomedical domain and preparation of optoprobiotic water based products.

Figure 7, illustrates Disinfection through packaging (DTP) without damaging said packaging or content therein, non invasively from (90 degrees to said packaging, illustrating the plurality of angular orientation possible with th methodology of the present invention) comprising; a packaging (50) having adequate refractive index profile throughout for the delivery, penetration or distribution of light (42) throughout for disinfection, or sterilization, or dissociation, or oxygenation through packaging (43) DTP (Disinfection Through Packaging), according to the present invention (1-80), a radiation unit (not shown) is illustrated having a sub-microsecond pulse duration or width and a wavelength (not Shown) at about 355nm, (46, 45, 44, 48, 47) illustrates flavorings, or additives, or sugars, or coloring, or carbonated, or $CO_2$, or Nitrogen, or bacteria, or toxic chemicals, or organic toxic compounds undamaged by the methodology of the present invention, (41) represent the cap having transparent, translucent or semi-opaque composition this light may be delivered for distribution throughout, especially beneficial for bottled water production sites, distribution lines, and for agro-food industries, beneficial for the production of opto-probiotic using DTP methodology according tot he present invention, especially beneficial for disinfection through packaging (DTP) of ½, 1, 2, and up to 20 liters (5 gallons) of bottled water products including flavored water, carbonated water, and for liquids and gases in need of disinfection in their packaging without damaging said packaging (43), (51) illustrates the air capsule or gap twisted with the inner volume or content of said packaging (Not shown) so a to create a diffuser for the delivered or distributed light for disinfection through packaging (DTP) the air remaining (51) after the bottle or jug, camber or conduit or container have being filled, thus according to the methodology of the present invention facilitate refractive index profile manipulation of the light delivered throughout (42)

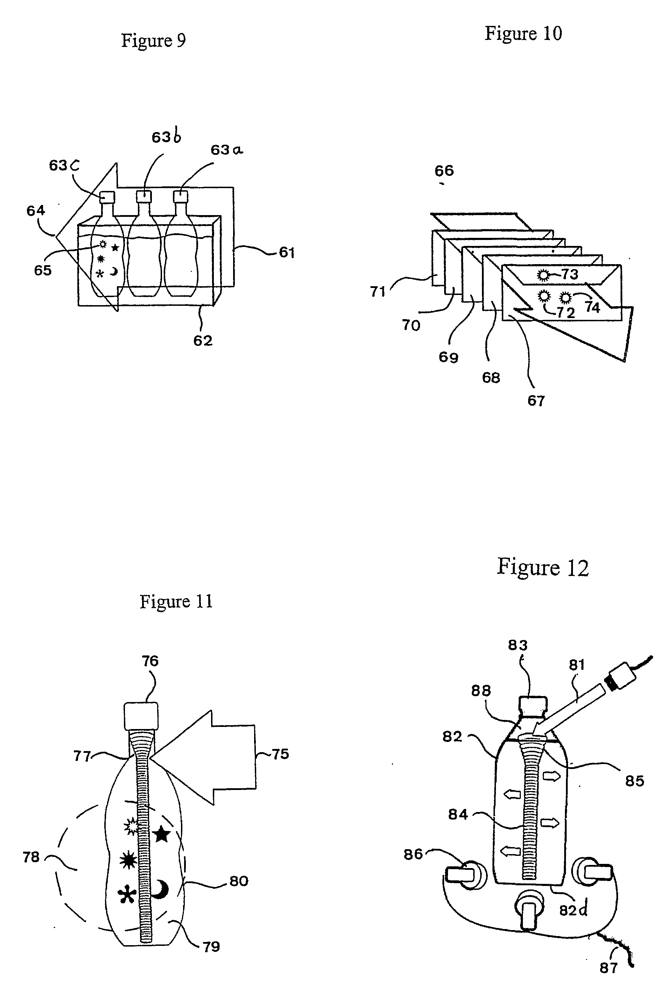

Figure 8, illustrates Disinfection through packaging (DTP) without damaging said packaging or content therein, thus disinfecting it non invasively illustrating preferred mode for coupling light to packaging using plurality of angular orientation possible [360 deg.] according to the methodology of the methodology of the present invention comprising; a packaging (53) having adequate refractive index throughout, a predetermined transparency and ability for the delivery, penetration or distribution of light (52) throughout for disinfection, or sterilization, or dissociation, or oxygenation through packaging (53) DTP (Disinfection Through Packaging), according to the present invention (1-80), a radiation unit (not shown) is illustrated having a sub-microsecond pulse duration or width and a wavelength (not Shown) at about 355nm, (57, 58, 59, 56, 55,) illustrates flavorings, or preservatives, or additives, or sugars, or coloring, or carbonated, or $CO_2$, or Nitrogen, or bacteria, or toxic chemicals, or organic toxic compounds undamaged by the methodology of the present invention, especially beneficial for bottled water production sites, distribution lines, and for agro-food industries, beneficial for the production of opto-probiotic using DTP methodology according tot he present invention, especially beneficial for disinfection through packaging (DTP) from about ½ liter to about 20 liters (5 gallons), or from about one micro-liter to about Two Thousand liters, or for bottled water products including flavored water, carbonated water, and for liquids and gases in need of disinfection in their packaging without damaging said packaging (60), (54) illustrates the air capsule or gap twisted with the inner volume or content of said packaging (Not shown) so a to create a diffuser for the delivered or distributed light for disinfection through packaging (DTP) the air remaining (54) after the bottle or jug, camber or conduit or container have being filled, thus according to the methodology of the present invention facilitate refractive index profile manipulation of the light delivered throughout (52). Preffered mode for devices utilizing the methodology of the present invention wherein DTP processes are being utilized for optoprobiotic production of bottled water aimed at strengthening the immune system of consumers while offering fresh, natural composition and flavored, or goodness or nutrient value, or shelf life or combinations.

Figure 9, illustrates Disinfection through packaging (DTP) without damaging said packaging or content wherein the packaging is immersed in water non invasively, comprising; a packaging (63,a,b,c) having adequate refractive index profile (62,a) throughout for the delivery, penetration or distribution of light (61) throughout for disinfection, or sterilization, or dissociation, or oxygenation through packaging (a,b,c) DTP (Disinfection Through Packaging), according to the present invention (1-80), a radiation unit (not shown) is illustrated having a sub-microsecond pulse duration or width and a wavelength (not Shown) at about 355nm, (57, 58, 59, 56, 55,) illustrates flavorings, or preservatives, or additives, or sugars, or coloring, or carbonated, or $CO_2$, or Nitrogen, or bacteria, or toxic chemicals, or organic toxic compounds undamaged by the methodology of the present invention, especially beneficial for bottled water production sites, distribution lines, and for agro-food industries, beneficial for the production of opto-probiotic using DTP methodology according to he present invention, especially beneficial for disinfection through packaging (DTP) from about ½ liter to about 20 liters (5 gallons), or from about one micro-liter to about Two Thousand liters, or for bottled water products (a,b,c,) including flavored water, carbonated water, and for liquids and gases in need of disinfection in their packaging without damaging said packaging (65, a,b,c, 63), Figure Nine illustrate a preferred mode for coupling the light (61) to the packaging (a,b,c, 65, 63) after the bottle or jug, camber or conduit or container (63 -65, a,b,c,) have being filled, thus according to the methodology of the present invention facilitate refractive index profile manipulation of the light delivered throughout (61), the benefit of coupling light (61) to the packaging through a predetermined volume of water (62,a) is a narrow refractive index profile (air is 1.00, water is 1.3, PET at 1.45, quartz wall of camber (62) at 1.45 thus reducing the refractive index profile range facilitate better coupling of the light to the packaging thus performing disinfection through packaging DTP. The shapes in the bottle (c) illustrated as (65) illustrate bacteria, viruses, molds, cysts, germs, toxic organic molecules or compounds in the bottle such that only noxious species are inactivated without damaging sensitive components therein (in the content or on the surfaces of the packaging).

Figure 10, illustrates a method for disinfection non-invasively through packaging comprising; An envelope array or stack (67, 68, 69, 70, 71), the optronic (or optoelectronic) energy dose is represented by (66), illustrating the high peak power, sub-microsecond, pulsed light beams generated by a radiation unit having a high intensity source of light (not shown), spores (74), or noxious biological or chemical species is represented by (72) being inactivated (such as inactivating DNA/RNA replication sequences), or photochemical oxidized or reduced such that the content (74, 72, 73) is being made innocuous by the methodology of the present invention for Disinfection Through Packaging (DTP). The methodology of the present invention does not cause molecular migration or does not cause heat as the time domain of the delivered light (Example: i.e. submicrosecond, 266nm or 355nm pulsed UV laser, or lamps or hybrid platform or combinations respectively) is considerably shorter then the thermal dynamic of the packaging, envelope (67, 68, 69, 70, 71), (paper for example, or polymer varieties). Especially beneficial for disinfection or sterilization of mail items from noxious species of biological or chemical origins (74, 72, 73). Especially beneficial for post offices, and mail delivery and sorting facilities and post office authorities.

Figure 11, illustrates a schematic view of the method of the present invention for enhancing biosecurity, and chemical safety in a mass production line of mineral water, flavored water, beverages, spring water, bottled water, or carbonated water, or enhanced water, or any combination thereof or in biotechnology production lines for medical preparation, or for blood processing; A schematic view of the method of the present invention for non invasive, non damaging disinfection through packaging DTP is herewith disclosed comprising: 5 gallons jug, or a container, or chamber, or bottle, or conduit is Shown (80), wherein a beam of light (75) is illustrated penetrating (75, 76, 77), the packaging (80) wherein (77) illustrate the air capsule or pocket left in any bottled water, or packaged beverage, juice, milk or water thus illustrating the potential diversity of refractive index profiles thus being generated by twisting the volume (and/or the packaging with the content) according tot he methodology of the present invention therein (in the packaging) or by delivering light throughout from adequate angular orientation, thus disinfecting and sterilizing, dissociating or oxidizing noxious species on the packaging surfaces, or throughout the packaging content, or combination, according to the present invention the time domain of the delivered light (pulsed sub-microsecond time domain) is shorter then the thermal dynamics of he packaging material (80), (for example , PET penetrated by a diod pumped Yag at 355nm, 75mHz repetition rate, and 3 ns pulse width), thus disinfection, or phototreatment (75, 76, 77, 78, 79,) (i.e. Oxidation, dissociation, reduction, elimination, inactivation, sterilization, photolysis, photo activation), optoprobiotic effects are achieved, and occurs according to the present invention non invasively without causing damage to said packaging (80), or content (78, 79). Especially beneficial for agro-food industries, for bottling (80) industry (75, 76, 77, 78, 79,) , and for the production of bottled water (75, 76, 77, 78, 79,), and biomedical preparation (75, 76, 77, 78, 79,) of optoprobiotic water based or bottled water products (75, 76, 77, 78, 79, 80,), the present invention by coupling light (75) to the vortex r tornado shaped diffuser having a predetermined refractive index profile in relation to the content or the volume of the packaging (80), therein (in the packaging) for distributing appropriate spectral distribution uniformly within a predetermined space (packaging receptive capabilities of inner volume) over predetermined period of time for disinfection sterilization oxidation and reduction or elimination through the packaging (DTP, Disinfection through Packaging) according tot he methodology of the present invention.

Figure 12 illustrate the methodology of the present invention for disinfection through packaging non invasively comprising:

A high intensity beam of light from about 1nm to About 999nm (81), penetrating through the packaging (82), wherein the packaging (82), (83), contains remaining air capsule or gas (88), illustrating the refractive index profile therein (88), (81), (84), (82), and when said air space or capsule is twisted or spun (85) it extend to reach (85) across any portion of the camber or conduit , or bottle (82, 83, 85) length, or depth, or width, or inner volume (84) , or content such that noxious species (not shown) of biological, or chemical origin which may be present in the content, and/or said packaging surfaces (inner surfaces, or outer surfaces)are being inactivated, or disinfected, or reduced, or equalized, or eliminated, or oxygenated, or dissociated (81, 85, 84) by light (81, 85, 84), or being sterilized through packaging (83, 82), (84) without causing damage to said packaging or content composition (82), (84), thus facilitating disinfection Through Packaging (DTP) according to the methodology of the present invention. Especially beneficial for agro-food production processes, and for production of opto-pro-biotic (immunifying) bottled water based products. The time domain or pulse width of the delivered light from about below the one microsecond (1 ms) to about above 1 Ato-second (As), and wherein pulse repetition rates, quasi-CW modulation or cross modulation frequency is from about 1 Hz, to about the 18 GHz, Substantially faster then the thermal dynamic of the polymer, or packaging materials, thus no temperature rise, or damage to packaging material (82) occurs, yet noxious species in said packaging (82), and threatening species of biological or chemical origin are disinfected through packaging, DTP such that they are innocuous forms, and may be beneficial for the immune system, or MHC type I, II, III, or according to the present invention made biocompatible for consumption or use throughout (i.e. both packaging and content or volume therein have being disinfected or sterilized through utilization of the methodology of the present invention for disinfection through packaging (DTP), (87), illustrates the electrical wiring for Three individual ultrasound drivers, or tapered extensions wherein once active, spin the content, or packaging such that a diffuser (88, 85, 84, 81) is formed (extending to reach across the entire length of the bottle, camber or conduit (82, d,), (a,b,c,) represent the transient, or static cavitation (ultra sound), or ultrasound probes, or drivers responsible for vibrationally (not shown) twisting or spinning the air remaining in the bottle, or the air capsule therein (in the packaging), thus creating from the air or gas therein having a refractive index therein (in the packaging) from about 1.00 for air, 1.3 for liquids (water), and 1.45 for the packaging material, thus facilitating uniform distribution of the delivered light throughout the packaging or the content or the volume therein simultaneously, or sequentially, or cyclically, or non recurrently, or recurrently for disinfection of said packaging or content through packaging (DTP). (81) and (81a) represent a radiation unit and its associated high intensity light, coupled to the vortex or tornado shaped diffuser illustrated by (84).

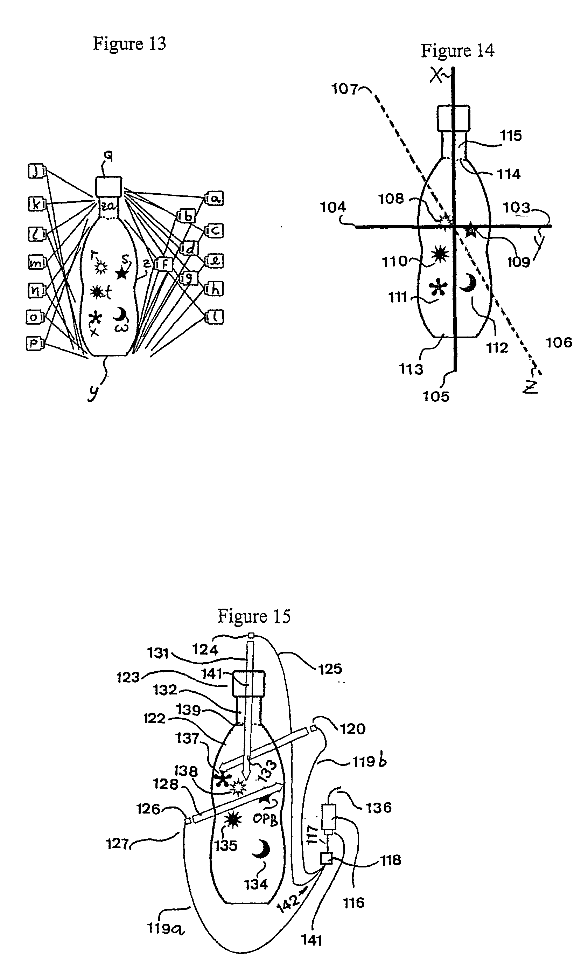

Figure 13 illustrate a methodology of the present invention for disinfection through packaging non invasively, comprising: A bottled water (z), illustrated with its bottom (y), an its top capped (q), wherein a radiation unit, or instruments (a-p) are illustrated operating singularly (a-p), or in unison (a, b, c, d, e, f, g, h, I, j, k, I, m, n, o, p, q, r, s, t, w, x, y, z) for the purposes of disinfection,or sterilization or dissociation or reduction or oxygenation, or elimination or equalization through packaging (disinfection through packaging i.e. DTP), and wherein each of said units having a high intensity source of light selected from lasers, or lamps CW, PW, quasi CW, hybrid, liquid or gas based lasers, (a-p)or the radiation unit or light sources combined or integrated include diamond based lasers, fiber lasers, TSA/SE/EAIFELs lasers, or electrical discharge lasers, or water lasers, or diode pumped or organic type lasers, or plasma lasers, or semi-conductor lasers, or amplified or harmonically generated lasers, or CW, PW lamps, or combination thereof (a-p) each capable of individually or collectively (a-p) to disinfect the packaging or the content or the volume (z/a) illustrated by the dotted line underneath the cap (q), without damaging said packaging or content or causing migration from packaging to content therein (in the packaging) thus according to the methodology of the invention facilitates disinfection through packaging DTP (a-p), (a-z). a/1 illustrates the light beam generated by each of the units (a-p) coupled to the bottle (z) from any angular orientation over 360 degrees to said packaging surface (z).

Figure 14 illustrate a methodology of the present invention for disinfection through packaging non invasively, comprising: A conduit or chamber or bottled water (100-115) illustrated on positioning coordinates (X = 102, 105), Y= 103, 104), wherein x represent the longitudinal length of the bottled water, and y represent its width, z represent its depth (106, 107), the lines (x,y,z) have been included for clarity in order to -portray to the reader that any angular orientation(360degrees) could be accommodated according the methodology of the present invention for disinfection and photo-treatment through packaging DTP. (110, 111, 112, 109) collectively illustrate the noxious, and health threatening compounds of biological, or chemical origin (110, 111, 112, 109), (115) illustrate the air capsule or remaining air, or gas at the top of the bottled water, underneath the cap (102), (100) represent the boundary of the packaging, and (114) represent the apex top water level line in the packaging (100), (108) represent an inactivated bacteria which can not replicate as a result of exposure to the methodology of the present invention for Disinfection through packaging (DTP), thus a bacteria which can not replicate can not infect, especially beneficial for the agro-food industries, and for production of bottled water products, having opto-pro-biotic competitive advantages for enhancing the immune system of human, animal or plants according to the methodology of the present ikbnvewnhtion for disinfection through packaging DTP.

Figure 15 illustrate the methodology of the present invention for disinfection through packaging non invasively using wave-guides and instrumental beam management, comprising: A radiation unit having a high intensity, or pulsed source of light (133) represent spectral distribution within a predetermined space or volume over a predetermined period of time wherein the delivered light is having at least one wavelength at 355nm, and pulsed submicrosecond pulse width extending from about 1 micro-second to about 1 Ato-second, and wherein cross sectional energy density, or interference (133), or energy density per cubic centimeter (Cm3) is reaching to extend from about 1nJ (1 Nano Joul) per Cm2, to about 18 Js Cm2, and wherein repetition rate or modulation frequencies reaching between about 1 Hz, to about 118 THz (131, 133, 129, 1170), and wherein said radiation unit, or instrument, or spectral distribution of the delivered light or applied bio-dosimetric, or chemical dosimetric energy curve or levels is adjusted, calibrated, or having a radiance, or fluence rate from About 250,000 photons per about 187 angstrom, or per adequate measure of contaminants (i.e. of biological or chemical origin to about 118 Trillion photons per measure (116), coupled to (117), a common end termination or optical distribution system (118) or detection circuits (141), wherein at least one optical wave guide (119a, 119b) is illustrated (119b) as being Bi- directionally able to deliver light throughout (i.e. to and from bottled water or target site or packaging or detection circuit) represent an additional wave guide (119b) extending to reach the target site, or packaging, or bottled water, or biomedical camber or conduit (122) for distribution of the light therein (134, 135, 128, 137,, 138, 129, 133,), into the packaging (122), for disinfection of said packaging surfaces and the content of the liquid or gas therein). An additional optical wave-guide is shown (125) extending to reach the proximity or appropriate angular orientation for delivering (131), (132), (141), (140), or distribution, (133), or diffusion, or projection, or inactivation (138), or disinfection (138, 133, 129, 137, 135, 128), or sterilization, or photo-reactivation, or proteolysis, or oxidation, or illumination, or exposure, or Combination, or for receiving light from said packaging or bottle for diagnostics, collecting calibration data or fluorescent events or spectroscopy observing packaging and/or content (OPB), or combination therein (through the packaging), for transmission from target site, or packaging, or bottled water (122) to said detection circuit for adjusting bio-dosimetric curves, energy levels, and spectral, and spatial properties of the delivered light for disinfection, or oxidation (129) 133) through packaging DTP. (126) illustrates a light projection point shown (129) to inactivate (129,, 137, 128) at least one DNA/RNA replication sequences thus a bacteria (135, 137) which cannot replicate, can not infect (129, 137, 135,), (134), represent a toxic organic or chemical component not yet oxidized, or reduced, or eliminated or dissociated (OPB), or processed (OPB), or photo-treated or combination (129,, 133, 128, 137) by the delivered light (131, 132, 133, 128, 129, 119a, 119b, 117). (130) represent the bottom of the bottled water , or packaging, (141) represent a transparent Cap (141, 131, 132, 133, i.e. light seeing as penetrating said cap)) wherein light from the radiation unit (116) is delivered through said cap, or lid, or top, or cover (141) for disinfection, or sterilization, or photo-treatment through packaging or DTP according to the methodology of the present invention. Especially beneficial for the agro-food industries, and bottled water industries, and for biomedical preparation of opto-pro-biotic compounds (OPB) through packaging such that once consumed or reach their respective destination, enhances the opulent, or positive, educated, or rehearsed human immune system, or acquired immune system of human, animal or plants. Especially beneficial for the production of opto-pro-biotic (optoprobiotic meaning that the dissociated or photo-treated components therein in the packaging are being dissociated without damaging their geometrical integrity, thus allowing traceable recognition by the acquired immune system of human, animal or plants) bottled water products (122), chemical vaccination and programming and preparation (OPB of immune system [ Computer is the MHC I, II, III, disk is the water , or the content of the packaging, and the data is the noxious species therein in the packaging), such that opto-pto-biotic compounding and photo-production is herewith illustrated according to the methodology of the present invention, especially beneficial for medical preparation requiring high level of bio-compatibility, and bio-security. Light (133, 129, 132, 131, 117) I seeing projected, or delivered, or combination distributed from optical wave guide distal tips (far ends) (126, 120) so as to penetrate the packaging (126, 129, 131, 132, 133, 129), via DTP (disinfection through packaging) sufficiently fast for disinfection or sterilization of noxious species in said packaging (122), but substantially faster then the thermal dynamics of the packaging material (122) such that no molecular migration occurs from packaging to its content or volume internally (i.e. in the packaging) especially beneficial for bottled water products, and for agro-food production process, and for biomedical procedures, providing bio-security by light through packaging DTP.

## Claims

1. Method for non invasive disinfection of packages (2, 5-8, 15, 20, 21, 24-27, 31, 43, 60, 63 a-c, 80, 82, 113, 122) using light radiation, said method is especially useful for disinfecting non-opaque package, and for disinfecting the content of packages (2, 5-8, 15, 20, 21, 24-27, 31, 43, 60, 63 a-c, 80, 82, 113, 122) having at least one non-opaque portion through which light radiation may enter inside the package (2, 5-8, 15, 20, 21, 24-27, 31, 43, 60, 63 a-c, 80, 82, 113, 122), comprising the steps of:

(a) positioning at least one package (2, 5-8, 15, 20, 21, 24-27, 31, 43, 60, 63 a-c, 80, 82, 113, 122) opposite at least one light radiation means (12, 12a, 18, 18a, 22, 120, 124, 126) capable of emitting submicrosecond light pulses;

(b) creating a light diffuser (84) in a mid portion inside said package (2, 5-8, 15, 20, 21, 24-27, 31, 43, 60, 63 a-c, 80, 82, 113, 122), made of a plurality of refractive index profiles between liquid and air content inside said at least one package (2, 5-8, 15, 20, 21, 24-27, 31, 43, 60, 63 a-c, 80, 82, 113, 122);

(c) directing at least one submicrosecond pulse of light emitted from said light radiation means (12, 12a, 18, 18a, 22, 120, 124, 126), to penetrate the package (2, 5-8, 15, 20, 21, 24-27, 31, 43, 60, 63 a-c, 80, 82, 113, 122) through at least one non-opaque portion of said at least one package (2, 5-8, 15, 20, 21, 24-27, 31, 43,

## EP 1 334 031 B1

60, 63 a-c, 80, 82, 113, 122) towards said light diffuser;

wherein said pulse of light contains at least one light component having a wavelength of between 1 nm and 999nm, and wherein said component has a peak power of energy sufficient to destroy the toxic or noxious nature of compounds of biological or chemical origin anticipated to be present on said package (2, 5-8, 15, 20, 21, 24-27, 31, 43, 60, 63 a-c, 80, 82, 113, 122) or in its content, and wherein the time extent of said pulse is short enough such that its energy is insufficient for damaging or releasing unwanted particles from said package (2, 5-8, 15, 20, 21, 24-27, 31, 43, 60, 63 a-c, 80, 82, 113, 122) or its content, wherein said pulse of light is being diffused from said light diffuser throughout the package (2, 5-8, 15, 20, 21, 24-27, 31, 43, 60, 63 a-c, 80, 82, 113, 122).

2. Method for non invasive disinfection of packages (2, 5-8, 15, 20, 21, 24-27, 31, 43, 60, 63 a-c, 80, 82, 113, 122) according to claim 1, wherein the package is sealed with its content inside prior to the disinfection.

3. Method for non invasive disinfection of packages (2, 5-8, 15, 20, 21, 24-27, 31, 43, 60, 63 a-c, 80, 82, 113, 122) according to claim 1, wherein the diffuser of the plurality of refractive index profiles is created by twisting the package content.

4. Method for non invasive disinfection of packages (2, 5-8, 15, 20, 21, 24-27, 31, 43, 60, 63 a-c, 80, 82, 113, 122) according to claim 1, wherein the diffuser of the plurality of refractive index profiles is created by forming a vortex inside the package (2, 5-8, 15, 20, 21, 24-27, 31, 43, 60, 63 a-c, 80, 82, 113, 122).

5. Method for non invasive disinfection of packages (2, 5-8, 15, 20, 21, 24-27, 31, 43, 60, 63 a-c, 80, 82, 113, 122) according to claim 1, wherein the liquid content of the package (2, 5-8, 15, 20, 21, 24-27, 31, 43, 60, 63 a-c, 80, 82, 113, 122) further includes frozen liquid, jell, powder, solid or gas, or a combination thereof.

6. Method for non invasive disinfection of packages (2, 5-8, 15, 20, 21, 24-27, 31, 43, 60, 63 a-c, 80, 82, 113, 122) according to claim 4, wherein the vortex is formed by applying acoustic energy at a predetermined angle, to stir the liquid inside the package (2, 5-8, 15, 20, 21, 24-27, 31, 43, 60, 63 a-c, 80, 82, 113, 122).

7. Method for non invasive disinfection of packages (2, 5-8, 15, 20, 21, 24-27, 31, 43, 60, 63 a-c, 80, 82, 113, 122) according to claim 4, wherein the volume of the liquid in the package (2, 5-8, 15, 20, 21, 24-27, 31, 43, 60, 63 a-c, 80, 82, 113, 122) is spun at speed of between 18-1800 RPM to create the vortex.

8. Method for non invasive disinfection of packages (2, 5-8, 15, 20, 21, 24-27, 31, 43, 60, 63 a-c, 80, 82, 113, 122) according to claim 1, wherein the at least one light radiation means (12, 12a, 18, 18a, 22, 120, 124, 126) is being used during a disinfection sequence together with a plurality of other light sources useful for disinfection.

9. Method for non invasive disinfection of packages (2, 5-8, 15, 20, 21, 24-27, 31, 43, 60, 63 a-c, 80, 82, 113, 122) according to claim 1, wherein the at least one light radiation means (12, 12a, 18, 18a, 22, 120, 124, 126) is being used during a disinfection sequence together with at least one ultrasonic transducer.

10. Method for non invasive disinfection of packages (2, 5-8, 15, 20, 21, 24-27, 31, 43, 60, 63 a-c, 80, 82, 113, 122) according to claim 1, wherein the wavelength of the at least one light radiation means (12, 12a, 18, 18a, 22, 120, 124, 126) is between about 347nm and 399nm.

11. System for non-invasive disinfection of packages (2, 5-8, 15, 20, 21, 24-27, 31, 43, 60, 63 a-c, 80, 82, 113, 122), said system is especially useful for disinfecting non-opaque package (2, 5-8, 15, 20, 21, 24-27, 31, 43, 60, 63 a-c, 80, 82, 113, 122), and for disinfecting the content of packages (2, 5-8, 15, 20, 21, 24-27, 31, 43, 60, 63 a-c, 80, 82, 113, 122) having at least one non-opaque portion through which light radiation may enter inside the package (2, 5-8, 15, 20, 21, 24-27, 31, 43, 60, 63 a-c, 80, 82, 113, 122), comprising;

(a) at least one light radiation instrument capable of emitting pulses of light that contain at least one light component having a wavelength of between 200nm and 1000nm;

(b) mechanical means for positioning at least one package (2, 5-8, 15, 20, 21, 24-27, 31, 43, 60, 63 a-c, 80, 82, 113, 122) intended to be disinfected, opposite said at least one light radiation instrument, for receiving light radiation;

47

(c) means for creating a light diffuser made of a plurality of refractive index profiles between a liquid and air content in a mid portion inside the package (2, 5-8, 15, 20, 21, 24-27, 31, 43, 60, 63 a-c, 80, 82, 113, 122);

(d) control means for controlling said light radiation instrument to transmit pulses of light toward the light diffuser created inside said packages (2, 5-8, 15, 20, 21, 24-27, 31, 43, 60, 63 a-c, 80, 82, 113, 122);

wherein said pulses of light contain at least one light component having a wavelength of between 1 nm and 999nm, and wherein said component has a peak power of energy sufficient to destroy the toxic or noxious nature of compounds of biological or chemical origin anticipated to be present on said packages (2, 5-8, 15, 20, 21, 24-27, 31, 43, 60, 63 a-c, 80, 82, 113, 122) or in their content, and
wherein the time extent of said pulse is short enough such that the accumulative energy of the pulses being transmitted into one package during a disinfection sequence is insufficient for damaging or releasing unwanted particles from said package (2, 5-8, 15, 20, 21, 24-27, 31, 43, 60, 63 a-c, 80, 82, 113, 122) or its content,
wherein said pulses of light are being diffused from said diffuser throughout the package (2, 5-8, 15, 20, 21, 24-27, 31, 43, 60, 63 a-c, 80, 82, 113, 122).

12. System for non-invasive disinfection of packages (2, 5-8, 15, 20, 21, 24-27, 31, 43, 60, 63 a-c, 80, 82, 113, 122) according to claim 11, wherein the means for creating the plurality refractive index diffuser is acoustic energy.

13. System for non-invasive disinfection of packages (2, 5-8, 15, 20, 21, 24-27, 31, 43, 60, 63 a-c, 80, 82, 113, 122) according to claim 12, wherein the acoustic energy means are adapted to form a vortex inside the package content.

14. System for non-invasive disinfection of packages (2, 5-8, 15, 20, 21, 24-27, 31, 43, 60, 63 a-c, 80, 82, 113, 122) according to claim 11, wherein the light radiation instrument includes a laser.

15. System for non-invasive disinfection of packages (2, 5-8, 15, 20, 21, 24-27, 31, 43, 60, 63 a-c, 80, 82, 113, 122) according to claim 11, further comprising a plurality of light sources in combination with the at least one light radiation means (12, 12a, 18, 18a, 22, 120, 124, 126).

16. System for non-invasive disinfection of packages (2, 5-8, 15, 20, 21, 24-27, 31, 43, 60, 63 a-c, 80, 82, 113, 122) according to claim 11, further comprising an ultra-sonic trasducer in combination with the at least one light radiation means (12, 12a, 18, 18a, 22, 120, 124, 126).

17. System for non-invasive disinfection of packages (2, 5-8, 15, 20, 21, 24-27, 31, 43, 60, 63 a-c, 80, 82, 113, 122) according to claim 14, wherein the laser is a solid state laser.

18. System for non-invasive disinfection of packages (2, 5-8, 15, 20, 21, 24-27, 31, 43, 60, 63 a-c, 80, 82, 113, 122) according to claim 14, wherein the laser is a q-switched lamp pumped laser with 2nd, 3rd, 4th, 5th, 6th harmonic generator or frequency doubler or converter.

19. System for non-invasive disinfection of packages (2, 5-8, 15, 20, 21, 24-27, 31, 43, 60, 63 a-c, 80, 82, 113, 122) according to claim 11, wherein the light radiation instrument includes a hybrid laser and lamp integrated on a single platform.

20. System for non-invasive disinfection of packages (2, 5-8, 15, 20, 21, 24-27, 31, 43, 60, 63 a-c, 80, 82, 113, 122) according to claim 11, wherein the light radiation instrument includes a LED.

21. System for non-invasive disinfection of packages (2, 5-8, 15, 20, 21, 24-27, 31, 43, 60, 63 a-c, 80, 82, 113, 122) according to claim 11, wherein the light radiation instrument includes a pulsed UV solid state laser operating at between 1 Hz and 75MHz, having a wavelength of about 355nm, and a sub-microsecond pulse duration, causing 2nd order interactions or multiphoton absorption processes on the surface of packaging and through out the volume or content of said packaging.

**Patentansprüche**

1. Verfahren zum nicht-invasiven Desinfizieren von Verpackungen (2, 5-8, 15, 20, 21, 24-27, 31, 43, 60, 63 a-c, 80, 82, 113, 122) unter Verwendung von Lichtstrahlung, wobei das Verfahren insbesondere für das Desinfizieren einer nicht-opaken Verpackung und für das Desinfizieren des Inhalts von Verpackungen (2, 5-8, 15, 20, 21, 24-27, 31, 43, 60, 63 a-c, 80, 82, 113, 122) mit wenigstens einem nicht-opaken Anteil, durch den Lichtstrahlung in das Innere der Verpackung (2, 5-8, 15, 20, 21, 24-27, 31, 43, 60, 63 a-c, 80, 82, 113, 122) eintreten kann, geeignet ist, umfassend die Schritte;

(a) Positionieren von wenigstens einer Verpackung (2, 5-8, 15, 20, 21, 24-27, 31, 43, 60, 63 a-c, 80, 82, 113, 122) gegenüber wenigstens einer Lichtstrahlungseinrichtung (12, 12a, 18, 18a, 22, 120, 124, 126), die geeignet ist, Lichtpulse im Sub-Mikrosekundenbereich zu emittieren;

(b) Bilden eines Lichtdiffusors (84) in einem mittleren Abschnitt innerhalb der genannten Verpackung (2, 5-8, 15, 20, 21, 24-27, 31, 43, 60, 63 a-c, 80, 82, 113, 122), der aus einer Vielzahl von Refraktionsindexprofilen zwischen dem flüssigen Inhalt und Luft innerhalb der genannten wenigstens einen Verpackung (2, 5-8, 15, 20, 21, 24-27, 31, 43, 60, 63 a-c, 80, 82, 113, 122) besteht;

(c) Lenken von wenigstens einem Lichtpuls im Sub-Mikrosekundenbereich von der genannten Lichtstrahlungseinrichtung (12, 12a, 18, 18a, 22, 120,124, 126), um die Verpackung (2, 5-8, 15, 20, 21, 24-27, 31, 43, 60, 63 a-c, 80, 82, 113, 122) durch wenigstens einen nicht-opaken Anteil der genannten wenigstens einen Verpackung (2, 5-8, 15, 20, 21, 24-27, 31, 43, 60, 63 a-c, 80, 82, 113, 122) in Richtung zu dem genannten Lichtdiffusor zu durchdringen;

wobei der genannte Lichtpuls wenigstens einen Lichtbestandteil mit einer Wellenlänge von zwischen 1 nm und 999 nm aufweist und wobei der genannte Bestandteil eine Spitzenleistung an Energie aufweist, die ausreichend ist, um die toxische oder schädliche Natur von Verbindungen mit biologischem oder chemischem Ursprung, von denen es bekannt ist, dass sie auf der genannten Verpackung (2, 5-8, 15, 20, 21, 24-27, 31, 43, 60, 63 a-c, 80, 82, 113, 122) oder in dem Inhalt der Verpackung vorhanden sind, zu zerstören und wobei der Zeitumfang des genannten Pulses kurz genug ist, dass dessen Energie nicht ausreichend ist, um Partikel von der genannten Verpackung (2, 5-8, 15, 20, 21, 24-27, 31, 43, 60, 63 a-c, 80, 82, 113, 122) oder von dessen Inhalt zu schädigen oder um unerwünschte Partikel freizugeben, wobei der genannte Lichtpuls von dem genannten Lichtdiffusor durch die Verpackung (2, 5-8, 15, 20, 21, 24-27, 31, 43, 60, 63 a-c, 80, 82, 113, 122) diffundiert wird.

2. Verfahren zum nicht-invasiven Desinfizieren von Verpackungen (2, 5-8, 15, 20, 21, 24-27, 31, 43, 60, 63 a-c, 80, 82, 113, 122) nach Anspruch 1, wobei die Verpackung mit ihren Inhalt darin vor dem Desinfizieren verschlossen wird.

3. Verfahren zum nicht-invasiven Desinfizieren von Verpackungen (2, 5-8, 15, 20, 21, 24-27, 31, 43, 60, 63 a-c, 80, 82, 113, 122) nach Anspruch 1, wobei der Diffusor der Vielzahl an Refraktionsindexprofilen gebildet wird, indem der Verpackungsinhalt gedreht wird.

4. Verfahren zum nicht-invasiven Desinfizieren von Verpackungen (2, 5-8, 15, 20, 21, 24-27, 31, 43, 60, 63 a-c, 80, 82, 113, 122) nach Anspruch 1, wobei der Diffusor der Vielzahl an Refraktionsindexprofilen gebildet wird, indem innerhalb der Verpackung (2, 5-8, 15, 20, 21, 24-27, 31, 43, 60, 63 a-c, 80, 82, 113, 122) ein starker Wirbel gebildet wird.

5. Verfahren zum nicht-invasiven Desinfizieren von Verpackungen (2, 5-8, 15, 20, 21, 24-27, 31, 43, 60, 63 a-c, 80, 82, 113, 122) nach Anspruch 1, wobei der flüssige Inhalt der Verpackung (2, 5-8, 15, 20, 21, 24-27, 31, 43, 60, 63 a-c, 80, 82, 113, 122) ferner gefrorene Flüssigkeit, Gel, Pulver, einen Feststoff oder ein Gas oder eine Kombination davon umfasst.

6. Verfahren zum nicht-invasiven Desinfizieren von Verpackungen (2, 5-8, 15, 20, 21, 24-27, 31, 43, 60, 63 a-c, 80, 82, 113, 122) nach Anspruch 4, wobei der starke Wirbel gebildet wird, indem akustische Energie unter einem vorbestimmten Winkel aufgebracht wird, um die Flüssigkeit innerhalb der Verpackung (2, 5-8, 15, 20, 21, 24-27, 31, 43, 60, 63 a-c, 80, 82, 113, 122) zu rühren.

7. Verfahren zum nicht-invasiven Desinfizieren von Verpackungen (2, 5-8, 15, 20, 21, 24-27, 31, 43, 60, 63 a-c, 80, 82, 113, 122) nach Anspruch 4, wobei das Volumen der Flüssigkeit in der Verpackung (2, 5-8, 15, 20, 21, 24-27, 31, 43, 60, 63 a-c, 80, 82, 113, 122) mit einer Geschwindigkeit von zwischen 18-1800 U/min geschleudert wird, um den starken Wirbel zu bilden.

**8.** Verfahren zum nicht-invasiven Desinfizieren von Verpackungen (2, 5-8, 15, 20, 21, 24-27, 31, 43, 60, 63 a-c, 80, 82, 113, 122) nach Anspruch 1, wobei wenigstens eine Lichtstrahlungseinrichtung (12, 12a, 18, 18a, 22, 120, 124, 126) während einer Desinfizierabfolge zusammen mit einer Vielzahl von anderen Lichtquellen, die für das Desinfizieren geeignet sind, verwendet wird.

**9.** Verfahren zum nicht-invasiven Desinfizieren von Verpackungen (2, 5-8, 15, 20, 21, 24-27, 31, 43, 60, 63 a-c, 80, 82, 113, 122) nach Anspruch 1, wobei die wenigstens eine Lichtstrahlungseinrichtung (12, 12a, 18, 18a, 22, 120, 124, 126) während einer Desinfizierabfolge zusammen mit wenigstens einem Ultraschallwandler verwendet wird.

**10.** Verfahren zum nicht-invasiven Desinfizieren von Verpackungen (2, 5-8, 15, 20, 21, 24-27, 31, 43, 60, 63 a-c, 80, 82, 113, 122) nach Anspruch 1, wobei die Wellenlänge der wenigstens einen Lichtstrahlungseinrichtung (12, 12a, 18, 18a, 22, 120, 124, 126) zwischen etwa 347 nm und 399 nm liegt.

**11.** System zum nicht-invasiven Desinfizieren von Verpackungen (2, 5-8, 15, 20, 21, 24-27, 31, 43, 60, 63 a-c, 80, 82, 113, 122), wobei das System insbesondere für das Desinfizieren einer nicht-opaken Verpackung (2, 5-8, 15, 20, 21, 24-27, 31, 43, 60, 63 a-c, 80, 82, 113, 122) und für das Desinfizieren des Inhalts der Verpackungen (2, 5-8, 15, 20, 21, 24-27, 31, 43, 60, 63 a-c, 80, 82, 113, 122) mit wenigstens einem nicht-opaken Anteil, durch den Lichtstrahlung in das Innere der Verpackung (2, 5-8, 15, 20, 21, 24-27, 31, 43, 60, 63 a-c, 80, 82, 113, 122) eintreten kann, geeignet ist, umfassend;

> (a) wenigstens ein Lichtstrahlungsinstrument, das geeignet ist, Lichtpulse, die wenigstens einen Lichtbestandteil mit einer Wellenlänge von zwischen 200 nm und 1000 nm enthalten, zu emittieren;

> (b) mechanische Einrichtungen zum Positionieren von wenigstens einer Verpackung (2, 5-8, 15, 20, 21, 24-27, 31, 43, 60, 63 a-c, 80, 82, 113, 122), die zum Desinfizieren vorgesehen ist, gegenüber wenigstens einem Lichtstrahlungsinstrument für die Aufnahme von Lichtstrahlung;

> (c) Einrichtungen zum Erzeugen eines Lichtdiffusors, der aus einer Vielzahl von Refraktionsindexprofilen zwischen einem flüssigen Inhalt und Luft innerhalb eines mittleren Abschnitts innerhalb der Verpackung (2, 5-8, 15, 20, 21, 24-27, 31, 43, 60, 63 a-c, 80, 82, 113, 122) besteht;

> (d) Steuereinrichtungen zum Steuern des genannten Lichtstrahlungsinstruments, um Lichtpulse zu dem Lichtdiffusor, der innerhalb der genannten Verpackungen (2, 5-8, 15, 20, 21, 24-27, 31, 43, 60, 63 a-c, 80, 82, 113, 122) gebildet wird, zu übertragen;

wobei die genannten Lichtpulse wenigstens einen Lichtbestandteil mit einer Wellenlänge von zwischen 1 nm und 999 nm aufweisen und wobei der Bestandteil eine Spitzenleistung an Energie aufweist, die ausreichend ist, um die toxische oder schädliche Natur von Verbindungen mit biologischem oder chemischem Ursprung, von denen es bekannt ist, dass sie auf den genannten Verpackungen (2, 5-8, 15, 20, 21, 24-27, 31, 43, 60, 63 a-c, 80, 82, 113, 122) oder in dem Inhalt der Verpackung vorhanden sind, zu zerstören und wobei der Zeitumfang des genannten Pulses kurz genug ist, dass die kumulative Energie des Pulses, der während einer Desinfizierabfolge in einer Verpackung übertragen wird, nicht ausreichend ist, um Partikel von der genannten Verpackung (2, 5-8, 15, 20, 21, 24-27, 31, 43, 60, 63 a-c, 80, 82, 113, 122) oder von dessen Inhalt zu schädigen oder um unerwünschte Partikel freizugeben, wobei die genannten Lichtpulse von dem genannten Lichtdiffusor durch die Verpackung (2, 5-8, 15, 20, 21, 24-27, 31, 43, 60, 63 a-c, 80, 82, 113, 122) diffundiert werden.

**12.** System zum nicht-invasiven Desinfizieren von Verpackungen (2, 5-8, 15, 20, 21, 24-27, 31, 43, 60, 63 a-c, 80, 82, 113, 122) nach Anspruch 11, wobei die Einrichtung zum Erzeugen eines Lichtdiffusors, der aus einer Vielzahl von Refraktionsindizes besteht, akustische Energie ist.

**13.** System zum nicht-invasiven Desinfizieren von Verpackungen (2, 5-8, 15, 20, 21, 24-27, 31, 43, 60, 63 a-c, 80, 82, 113, 122) nach Anspruch 12, wobei die akustischen Energieeinrichtungen eingestellt sind, um einen starken Wirbel innerhalb des Verpackungsinhalts zu bilden.

**14.** System zum nicht-invasiven Desinfizieren von Verpackungen (2, 5-8, 15, 20, 21, 24-27, 31, 43, 60, 63 a-c, 80, 82, 113, 122) nach Anspruch 11, wobei das Lichtstrahlungsinstrument einen Laser umfasst.

**15.** System zum nicht-invasiven Desinfizieren von Verpackungen (2, 5-8, 15, 20, 21, 24-27, 31, 43, 60, 63 a-c, 80, 82, 113, 122) nach Anspruch 11, das ferner eine Vielzahl von Lichtquellen zusammen mit der wenigstens einen Lichtstrahlungseinrichtung (12, 12a, 18, 18a, 22, 120, 124, 126) umfasst.

**16.** System zum nicht-invasiven Desinfizieren von Verpackungen (2, 5-8, 15, 20, 21, 24-27, 31, 43, 60, 63 a-c, 80, 82, 113, 122) nach Anspruch 11, das ferner einen Ultraschallwandler zusammen mit der wenigstens einen Lichtstrahlungseinrichtung (12, 12a, 18, 18a, 22, 120, 124, 126) umfasst.

**17.** System zum nicht-invasiven Desinfizieren von Verpackungen (2, 5-8, 15, 20, 21, 24-27, 31, 43, 60, 63 a-c, 80, 82, 113, 122) nach Anspruch 14, wobei der Laser ein Festkörperlaser ist.

**18.** System zum nicht-invasiven Desinfizieren von Verpackungen (2, 5-8, 15, 20, 21, 24-27, 31, 43, 60, 63 a-c, 80, 82, 113, 122) nach Anspruch 14, wobei der Laser ein q-geschalteter Lampenpumplaser mit einem 2., 3., 4., 5., 6. harmonischen Generator oder einem Frequenzverdoppler oder Konverter ist.

**19.** System zum nicht-invasiven Desinfizieren von Verpackungen (2, 5-8, 15, 20, 21, 24-27, 31, 43, 60, 63 a-c, 80, 82, 113, 122) nach Anspruch 11, wobei das Lichtstrahlungsinstrument einen Hybridlaser und eine Lampe umfasst, die auf einer einzigen Plattform integriert sind.

**20.** System zum nicht-invasiven Desinfizieren von Verpackungen (2, 5-8, 15, 20, 21, 24-27, 31, 43, 60, 63 a-c, 80, 82, 113, 122) nach Anspruch 11, wobei das Lichtstrahlungsinstrument eine LED umfasst.

**21.** System zum nicht-invasiven Desinfizieren von Verpackungen (2, 5-8, 15, 20, 21, 24-27, 31, 43, 60, 63 a-c, 80, 82, 113, 122) nach Anspruch 11, wobei das Lichtstrahlungsinstrument einen gepulsten UV-Festkörperlaser umfasst, der zwischen 1 Hz und 75 MHz mit einer Wellenlänge von etwa 355 nm in Betrieb ist und eine Pulsdauer im Sub-Mikrosekundenbereich aufweist, was Wechselwirkungen 2. Ordnung oder Multiphotonabsorptionsverfahren auf der Oberfläche der Verpackung und durch das Volumen oder den Inhalt der genannten Verpackung verursacht.

## Revendications

**1.** Procédé de désinfection non invasive d'emballages (2, 5-8, 15, 20, 21, 24-27, 31, 43, 60, 63 a-c, 80, 82, 113, 122) en utilisant un rayonnement lumineux, ledit procédé étant spécialement utile pour désinfecter l'emballage non opaque, et pour désinfecter le contenu d'emballages (2, 5-8, 15, 20, 21, 24-27, 31, 43, 60, 63 a-c, 80, 82, 113, 122) ayant au moins une partie non opaque à travers laquelle un rayonnement lumineux peut pénétrer à l'intérieur de l'emballage (2, 5-8, 15, 20, 21, 24-27, 31, 43, 60, 63 a-c, 80, 82, 113, 122), comprenant les étapes de :

(a) positionnement d'au moins un emballage (2, 5-8, 15, 20, 21, 24-27, 31, 43, 60, 63 a-c, 80, 82, 113, 122) opposé à au moins un moyen de rayonnement lumineux (12, 12a, 18, 18a, 22, 120, 124, 126) capable d'émettre des impulsions lumineuses inférieures à la microseconde ;

(b) création d'un diffuseur lumineux (84) dans une partie médiane à l'intérieur dudit emballage (2, 5-8, 15, 20, 21, 24-27, 31, 43, 60, 63 a-c, 80, 82, 113, 122), constitué d'une pluralité de profils d'indice de réfraction entre le contenu d'air et de liquide à l'intérieur dudit au moins un emballage (2, 5-8, 15, 20, 21, 24-27, 31, 43, 60, 63 a-c, 80, 82, 113, 122) ;

(c) orientation d'au moins une impulsion de lumière sous la microseconde émise par ledit moyen de rayonnement lumineux (12, 12a, 18, 18a, 22, 120, 124, 126), pour pénétrer dans l'emballage (2, 5-8, 15, 20, 21, 24-27, 31, 43, 60, 63 a-c, 80, 82, 113, 122) à travers au moins une partie non opaque dudit au moins un emballage (2, 5-8, 15, 20, 21, 24-27, 31, 43, 60, 63 a-c, 80, 82, 113, 122) vers ledit diffuseur lumineux ;

dans lequel ladite impulsion de lumière contient au moins un composant lumineux ayant une longueur d'onde comprise entre 1 nm et 999 nm, et dans lequel ledit composant a une puissance énergétique maximale suffisante pour détruire la nature nocive ou toxique de composés d'origine chimique ou biologique supposés être présents sur ledit emballage (2, 5-8, 15, 20, 21, 24-27, 31, 43, 60, 63 a-c, 80, 82, 113, 122) ou dans son contenu, et dans lequel l'étendue temporelle de ladite impulsion est assez courte pour que son énergie soit insuffisante pour endommager ledit emballage ou libérer des particules indésirables de celui-ci (2, 5-8, 15, 20, 21, 24-27, 31, 43, 60, 63 a-c, 80, 82, 113, 122) ou de son contenu,
dans lequel ladite impulsion de lumière est diffusée par ledit diffuseur lumineux à travers l'emballage (2, 5-8, 15, 20, 21, 24-27, 31, 43, 60, 63 a-c, 80, 82, 113, 122).

**2.** Procédé de désinfection non invasive d'emballages (2, 5-8, 15, 20, 21, 24-27, 31, 43, 60, 63 a-c, 80, 82, 113, 122) selon la revendication 1, dans lequel l'emballage est fermé hermétiquement avec son contenu à l'intérieur avant la désinfection.

**3.** Procédé de désinfection non invasive d'emballages (2, 5-8, 15, 20, 21, 24-27, 31, 43, 60, 63 a-c, 80, 82, 113, 122) selon la revendication 1, dans lequel le diffuseur de la pluralité de profils d'indice de réfraction est créé en tordant le contenu de l'emballage.

**4.** Procédé de désinfection non invasive d'emballages (2, 5-8, 15, 20, 21, 24-27, 31, 43, 60, 63 a-c, 80, 82, 113, 122) selon la revendication 1, dans lequel le diffuseur de la pluralité de profils d'indice de réfraction est créé en formant un vortex à l'intérieur de l'emballage (2, 5-8, 15, 20, 21, 24-27, 31, 43, 60, 63 a-c, 80, 82, 113, 122).

**5.** Procédé de désinfection non invasive d'emballages (2, 5-8, 15, 20, 21, 24-27, 31, 43, 60, 63 a-c, 80, 82, 113, 122) selon la revendication 1, dans lequel le contenu de liquide de l'emballage (2, 5-8, 15, 20, 21, 24-27, 31, 43, 60, 63 a-c, 80, 82, 113, 122) inclut en outre du liquide congelé, de la gelée, de la poudre, un solide ou un gaz, ou une combinaison de ceux-ci.

**6.** Procédé de désinfection non invasive d'emballages (2, 5-8, 15, 20, 21, 24-27, 31, 43, 60, 63 a-c, 80, 82, 113, 122) selon la revendication 4, dans lequel le vortex est formé en appliquant de l'énergie acoustique à un angle prédéterminé, pour agiter le liquide à l'intérieur de l'emballage (2, 5-8, 15, 20, 21, 24-27, 31, 43, 60, 63 a-c, 80, 82, 113, 122).

**7.** Procédé de désinfection non invasive d'emballages (2, 5-8, 15, 20, 21, 24-27, 31, 43, 60, 63 a-c, 80, 82, 113, 122) selon la revendication 4, dans lequel le volume du liquide dans l'emballage (2, 5-8, 15, 20, 21, 24-27, 31, 43, 60, 63 a-c, 80, 82, 113, 122) est centrifugé à une vitesse comprise entre 18 et 1800 TR/MIN pour créer le vortex.

**8.** Procédé de désinfection non invasive d'emballages (2, 5-8, 15, 20, 21, 24-27, 31, 43, 60, 63 a-c, 80, 82, 113, 122) selon la revendication 1, dans lequel le au moins un moyen de rayonnement lumineux (12, 12a, 18, 18a, 22, 120, 124, 126) est utilisé pendant une séquence de désinfection avec une pluralité d'autres sources lumineuses utiles pour la désinfection.

**9.** Procédé de désinfection non invasive d'emballages (2, 5-8, 15, 20, 21, 24-27, 31, 43, 60, 63 a-c, 80, 82, 113, 122) selon la revendication 1, dans lequel le au moins un moyen de rayonnement lumineux (12, 12a, 18, 18a, 22, 120, 124, 126) est utilisé pendant une séquence de désinfection avec au moins un capteur à ultrasons.

**10.** Procédé de désinfection non invasive d'emballages (2, 5-8, 15, 20, 21, 24-27, 31, 43, 60, 63 a-c, 80, 82, 113, 122) selon la revendication 1, dans lequel la longueur d'onde du au moins un moyen de rayonnement lumineux (12, 12a, 18, 18a, 22, 120, 124, 126) est comprise entre environ 347 nm et 399 nm.

**11.** Système de désinfection non invasive d'emballages (2, 5-8, 15, 20, 21, 24-27, 31, 43, 60, 63 a-c, 80, 82, 113, 122), ledit système étant spécialement utile pour désinfecter un emballage non opaque (2, 5-8, 15, 20, 21, 24-27, 31, 43, 60, 63 a-c, 80, 82, 113, 122), et pour désinfecter le contenu d'emballages (2, 5-8, 15, 20, 21, 24-27, 31, 43, 60, 63 a-c, 80, 82, 113, 122) ayant au moins une partie non opaque à travers laquelle un rayonnement lumineux peut entrer à l'intérieur de l'emballage (2, 5-8, 15, 20, 21, 24-27, 31, 43, 60, 63 a-c, 80, 82, 113, 122), comprenant :

(a) au moins un appareil à rayonnement lumineux capable d'émettre des impulsions de lumière qui contiennent au moins un composant lumineux ayant une longueur d'onde comprise entre 200 nm et 1000 nm ;

(b) un moyen mécanique pour positionner au moins un emballage (2, 5-8, 15, 20, 21, 24-27, 31, 43, 60, 63 a-c, 80, 82, 113, 122) destiné à être désinfecté, à l'opposé dudit au moins un appareil à rayonnement lumineux, pour recevoir un rayonnement lumineux ;

(c) un moyen pour créer un diffuseur lumineux constitué d'une pluralité de profils d'indice de réfraction entre un contenu d'air et de liquide dans une partie médiane à l'intérieur de l'emballage (2, 5-8, 15, 20, 21, 24-27, 31, 43, 60, 63 a-c, 80, 82, 113, 122) ;

(d) un moyen de commande pour commander ledit appareil à rayonnement lumineux pour transmettre des impulsions de lumière vers le diffuseur lumineux créé à l'intérieur desdits emballages (2, 5-8, 15, 20, 21, 24-27, 31, 43, 60, 63 a-c, 80, 82, 113, 122) ;

dans lequel lesdites impulsions de lumière contiennent au moins un composant lumineux ayant une longueur d'onde comprise entre 1 nm et 999 nm, et dans lequel ledit composant a une puissance énergétique maximale suffisante pour détruire la nature nocive ou toxique de composés d'origine chimique ou biologique supposés être présents sur lesdits emballages (2, 5-8, 15, 20, 21, 24-27, 31, 43, 60, 63 a-c, 80, 82, 113, 122) ou dans leur contenu, et dans lequel l'étendue temporelle de ladite impulsion est assez courte pour que l'énergie cumulée des impulsions transmises dans un emballage pendant une séquence de désinfection soit insuffisante pour endommager ledit emballage ou libérer des particules indésirables de celui-ci (2, 5-8, 15, 20, 21, 24-27, 31, 43, 60, 63 a-c, 80, 82, 113, 122) ou son/de son contenu,
dans lequel lesdites impulsions de lumière sont diffusées par ledit diffuseur lumineux à travers l'emballage (2, 5-8, 15, 20, 21, 24-27, 31, 43, 60, 63 a-c, 80, 82, 113, 122).

12. Système de désinfection non invasive d'emballages (2, 5-8, 15, 20, 21, 24-27, 31, 43, 60, 63 a-c, 80, 82, 113, 122) selon la revendication 11, dans lequel le moyen pour créer le diffuseur à pluralité d'indices de réfraction est l'énergie acoustique.

13. Système de désinfection non invasive d'emballages (2, 5-8, 15, 20, 21, 24-27, 31, 43, 60, 63 a-c, 80, 82, 113, 122) selon la revendication 12, dans lequel les moyens à énergie acoustique sont conçus pour former un vortex à l'intérieur du contenu de l'emballage.

14. Système de désinfection non invasive d'emballages (2, 5-8, 15, 20, 21, 24-27, 31, 43, 60, 63 a-c, 80, 82, 113, 122) selon la revendication 11, dans lequel l'appareil à rayonnement lumineux inclut un laser.

15. Système de désinfection non invasive d'emballages (2, 5-8, 15, 20, 21, 24-27, 31, 43, 60, 63 a-c, 80, 82, 113, 122) selon la revendication 11, comprenant, en outre, une pluralité de sources lumineuses en combinaison avec le au moins un moyen à rayonnement lumineux (12, 12a, 18, 18a, 22, 120, 124, 126).

16. Système de désinfection non invasive d'emballages (2, 5-8, 15, 20, 21, 24-27, 31, 43, 60, 63 a-c, 80, 82, 113, 122) selon la revendication 11, comprenant, en outre, un capteur à ultrasons en combinaison avec le au moins un moyen à rayonnement lumineux (12, 12a, 18, 18a, 22, 120, 124, 126).

17. Système de désinfection non invasive d'emballages (2, 5-8, 15, 20, 21, 24-27, 31, 43, 60, 63 a-c, 80, 82, 113, 122) selon la revendication 14, dans lequel le laser est un laser à solide.

18. Système de désinfection non invasive d'emballages (2, 5-8, 15, 20, 21, 24-27, 31, 43, 60, 63 a-c, 80, 82, 113, 122) selon la revendication 14, dans lequel le laser est un laser pompé à lampe déclenchée avec un ou convertisseur ou doubleur de fréquence ou générateur d'harmoniques de 2ème, 3ème, 4ème, 5ème, et 6ème rang.

19. Système de désinfection non invasive d'emballages (2, 5-8, 15, 20, 21, 24-27, 31, 43, 60, 63 a-c, 80, 82, 113, 122) selon la revendication 11, dans lequel l'appareil à rayonnement lumineux inclut un laser hybride et une lampe intégrés sur une plate-forme unique.

20. Système de désinfection non invasive d'emballages (2, 5-8, 15, 20, 21, 24-27, 31, 43, 60, 63 a-c, 80, 82, 113, 122) selon la revendication 11, dans lequel l'appareil à rayonnement lumineux inclut une DEL.

21. Système de désinfection non invasive d'emballages (2, 5-8, 15, 20, 21, 24-27, 31, 43, 60, 63 a-c, 80, 82, 113, 122) selon la revendication 11, dans lequel l'appareil à rayonnement lumineux inclut un laser à solide UV pulsé fonctionnant entre 1 Hz et 75 MHz, ayant une longueur d'onde d'environ 355 nm, et une durée d'impulsion inférieure à la microseconde, entraînant des interactions de 2ème ordre ou des processus d'absorption multiphotonique sur la surface d'emballage et dans le volume ou contenu dudit emballage.

Figure 1

Figure 2

Figure 3

Figure 4

Figure 5

Figure 6

Figure 7

Figure8

Figure 9

Figure 10

Figure 11

Figure 12

Figure 13

Figure 14

Figure 15